# EUROPÄISCHE PATENTSCHRIFT

(11) **EP 2 948 538 B1**
(45) Veröffentlichungstag und Bekanntmachung des Hinweises auf die Patenterteilung: **29.03.2017**
(21) Anmeldenummer: 14701699.2
(22) Anmeldetag: 21.01.2014
(51) Int. Cl.: C12M 1/00, C12M 1/26, C12M 1/34

(54) **ZELLKULTURANLAGE ZUR KULTIVIERUNG ADHÄRENTER ZELLEN SOWIE FLUID-VERSORGUNGSSCHNITTSTELLE MIT ZELLKULTURBEHÄLTER**
CELL CULTURING SYSTEM FOR CULTIVATING ADHERENT CELLS AND LIQUID SUPPLY INTERFACE COMPRISING A CELL CULTURE CONTAINER
SYSTÈME DE CULTURE DE CELLULES UTILISÉ POUR LA CULTURE DE CELLULES ADHÉRENTES, ET INTERFACE D'ALIMENTATION EN FLUIDE À RÉCIPIENT DE CULTURE DE CELLULES

(30) Priorität: 23.01.2013 DE 102013201069
(43) Veröffentlichungstag der Anmeldung: 02.12.2015
(73) Patentinhaber: Hamilton Bonaduz AG, 7402 Bonaduz (CH)
(72) Erfinder: JÄGER, Thomas, CH-7402 Bonaduz (CH); SCHLENKER, Dirk, 70563 Stuttgart (DE); OTHMAN, Nabih, 70771 Musberg (DE); KÜHNE, Oliver, CH-7208 Malans (CH)
(74) Vertreter: RLTG
(86) Internationale Anmeldenummer: PCT/EP2014/051072
(87) Internationale Veröffentlichungsnummer: WO 2014/114610

(56) Entgegenhaltungen:
- WO-A1-96/16161
- WO-A1-2011/090781
- WO-A2-03/076599
- WO-A2-2004/106484
- DE-A1- 3 508 151
- DE-A1- 4 207 346
- DE-A1-102008 035 644
- US-A- 5 994 129
- US-A1- 2011 223 076

## Beschreibung

Die vorliegende Erfindung betrifft Verbesserungen an Zellkulturanlagen mit wenigstens einem Zellkulturbehälter zur Aufnahme und Versorgung, also insbesondere zur Kultivierung mit dem Ziel der Vermehrung, von adhärenten Zellen darin, mit einem Nährmediumreservoir zur Versorgung der adhärenten Zellen im Zellkulturbehälter mit Nährstoffen, mit einem Reinigungsfluidreservoir zur Reinigung von Fluidströmungspfaden oder/und Strömungsräumen mit dem Reinigungsfluid, sowie mit einer Fluid-Versorgungsschnittstelle zur Ankopplung an den Zellkulturbehälter.

Die vorliegende Anmeldung betrifft weiter insbesondere die genannte Fluid-Versorgungsschnittstelle für eine Zellkulturanlage zur Versorgung von in unterschiedlichen Zellkulturbehältern vorhandenen Zellkulturen mit einem Nährmedium. Weiter betrifft die vorliegende Anmeldung Zellkulturbehälter, welche zur vorübergehenden strömungsmechanischen Kopplung mit der Fluid-Versorgungsschnittstelle ausgebildet sind, um dann, wenn die Fluid-Versorgungsschnittstelle mit einem Zellkulturbehälter strömungsmechanisch gekoppelt ist, frisches Nährmedium in den Zellkulturbehälter einzuleiten und, in der Regel verbrauchtes oder wenigstens betagtes, Nährmedium aus dem Zellkulturbehälter auszuleiten.

Bisher sind im Stand der Technik zur Kultivierung von Zellen einerseits technisch sehr aufwendige und für ihre jeweilige Aufgabe spezialisierte Bioreaktoren in Gebrauch, welche üblicherweise einen Reaktorraum aufweisen, der durch eine in den Bioreaktor integrierte Heizeinrichtung beheizbar ist und dessen Inhalt durch eine am Bioreaktor fest installierte Rühreinrichtung rührbar oder durchmischbar ist.

An derartigen Bioreaktoren sind üblicherweise ein Nährmediumreservoir vorgesehen, welches fest über Leitungen mit dem Reaktorraum als dem Herzstück der Zellkultivierung verbunden ist. Eine weitere Leitung kann von dem Reaktorraum zu einem Abfluss- oder einem Entsorgungssammelbehälter führen. Auch diese Leitung ist üblicherweise fest mit dem Reaktorraum verbunden. Es besteht also eine 1:1-Beziehung zwischen der Anzahl an Reaktorräumen und der Anzahl an Nährmediumreservoirs.

Bei der Kultivierung adhärenter Zellen sind im Stand der Technik andererseits häufig Wegwerf- oder Einweg-Zellkulturbehälter im Einsatz, welche üblicherweise zur Beobachtung der kultivierten Zellen aus durchsichtigem Kunststoff hergestellt und völlig passiv sind, also ohne den Behälterraum thermisch (Heizen/Kühlen) oder mechanisch (Rühren) beeinflussende Funktionsaggregate ausgebildet sind. Sofern ein derartiger Einweg-Zellkulturbehälter einer vorbestimmten Temperierung bedarf, muss diese in einem hierzu ausgebildeten Brutschrank oder einer ähnlichen Vorrichtung erfolgen. Auch eine Durchmischung der im Behälterraum eines derartigen Einweg-Zellkulturbehälters aufgenommenen Flüssigkeit ist mit dem Einweg-Zellkulturbehälter mangels eines daran vorgesehenen Rührwerks nicht oder bestenfalls durch Schütteln des Behälters erreichbar.

Die Einweg-Zellkulturbehälter des Standes der Technik weisen üblicherweise als einzige Zugangsöffnung einen mit Außengewinde versehenen Halsstutzen auf, welcher mit einem Schraubdeckel in herkömmlicher Weise verschließbar ist. Durch den Halsstutzen ist der Einweg-Zellkulturbehälter befüllbar, entleerbar und gewünschtenfalls auch belüftbar.

Nachteilig an den oben genannten Bioreaktoren in einer Zellkulturanlage ist deren hochgradige Spezialisierung auf vorbestimmte Anwendungsfälle, so dass es schon bei der Kultivierung lediglich geringfügig unterschiedlicher Zellkulturen notwendig sein kann, unterschiedliche Bioreaktoren vorzuhalten.

Bei einem Bioreaktor ist nach einer Anwendung zur Vermeidung von Kontaminationen von später bearbeiteten Kulturen entweder der notwendige Reinigungsaufwand für eine folgende Anwendung sehr groß oder der Bioreaktor wird trotz seiner verhältnismäßig hohen Anschaffungskosten schon nach einer einzigen Anwendung außer Betrieb gestellt und entsorgt. Beides erhöht die mit einer Zellkultivierung in einem derartigen Bioreaktor verbunden Kosten erheblich.

Zwar sind bei Einsatz der geschilderten Einweg-Zellkulturbehälter die mit diesen verbundenen Anschaffungs- und Betriebskosten, verglichen mit den zuvor diskutierten Bioreaktoren, erheblich niedriger. Jedoch weisen derartige Einweg-Zellkulturbehälter häufig ein Nutzvolumen von weniger als einem Liter auf und sind somit nur auf einen manuellen Betrieb in einem Labormaßstab ausgerichtet, was die mit diesen Einweg-Zellkulturbehältern erzielbare Ausbeute unerwünschterweise gering hält. Die bekannten Einweg-Zellkulturbehälter sind aufgrund der einzigen verfügbaren und mit Schraubverschluss verschließbaren Zugangsöffnung für eine Automatisierung und damit für eine Verwendung zur Zellkultivierung in einem Industriemaßstab nicht geeignet.

Aufgabe der vorliegenden Erfindung ist es daher, die oben genannten Nachteile des Standes der Technik in der Kultivierung adhärenter Zellen zu überwinden und eine technische Lehre anzugeben, welche es gestattet, adhärente Zellen bei vergleichsweise niedrigen Kosten (Bezugsgröße sollen hier Kosten pro Gewichtseinheit gewonnenes Zellmaterial sein) und verhältnismäßig hoher Ausbeute zu kultivieren.

Diese Aufgabe löst die vorliegende Erfindung anhand dreier Gesichtspunkte, welche über einen gemeinsamen Erfindungsgedanken verbunden sind und welche miteinander wechselwirken und unterschiedliche Aspekte ein- und derselben Zellkulturanlage betreffen.

Gemäß einem ersten Gesichtspunkt der vorliegenden Erfindung wird die oben genannte Aufgabe gelöst durch eine Fluid-Versorgungsschnittstelle für eine Zellkulturanlage zur Versorgung von in unterschiedlichen Zellkulturbehältern vorhandenen Zellkulturen mit einem Nährmedium, wobei die erfindungsgemäße Fluid-Versorgungsschnittstelle umfasst:
ein einen Strömungsraum definierendes Gehäuse,
eine erste Anschlussformation zur fluidübertragenden Verbindung einer ersten Fluidleitung mit dem Gehäuse,
eine von der ersten gesondert ausgebildete zweite Anschlussformation zur fluidübertragenden Verbindung einer zweiten Fluidleitung mit dem Gehäuse, eine von den ersten beiden gesondert ausgebildete dritte Anschlussformation zur fluidübertragenden Verbindung des Gehäuses mit einer dritten Fluidleitung,
eine von den Anschlussformationen gesondert ausgebildete Kopplungsformation, welche zum betriebsmäßig herstellbaren und lösbaren fluidübertragenden Kopplungseingriff mit einer entsprechenden Gegenkopplungsformation eines Zellkulturbehälters ausgebildet ist,
einen ersten Fluidströmungspfad, welcher zwischen dem Strömungsraum und der ersten Anschlussformation zur Einleitung eines ersten Fluids von außen in den Strömungsraum verläuft,
einen zweiten Fluidströmungspfad, welcher zwischen dem Strömungsraum und der zweiten Anschlussformation zur Einleitung eines vom ersten verschiedenen zweiten Fluids von außen in den Strömungsraum verläuft,
einen dritten Fluidströmungspfad, welcher zur Ausleitung eines Fluids aus dem Strömungsraum zwischen dem Strömungsraum und der dritten Anschlussformation verläuft, und
einen Kopplungsströmungspfad, welcher zwischen dem Strömungsraum und der Kopplungsformation verläuft, um über die Kopplungsformation ein Fluid aus dem Strömungsraum auszuleiten oder/und in diesen einzuleiten,
wobei der erste, der zweite und der dritte Fluidströmungspfad jeweils eine Ventilanordnung aufweisen, welche ohne eine von der Ventilanordnung bis zur Außenseite des Gehäuses kontinuierlich durchgehende signal- oder/und energieübertragende körperliche Verbindung vollständig - mit Ausnahme des jeweiligen Fluidströmungspfads - von dem Gehäuse umgeben in diesem aufgenommen ist,
wobei jeder Ventilanordnung eine Steueranordnung mit einem ein elektrisches oder/und magnetisches oder/und elektromagnetisches Feld erzeugenden Signalmittel zugeordnet ist, dessen Feld auf ein entsprechend feldsensitives Gegensignalmittel der Ventilanordnung berührungslos einwirkt, wobei jede Ventilanordnung mittels des auf ihr Gegensignalmittel einwirkenden Feldes zwischen einer Sperrstellung, in der die Ventilanordnung eine Fluidströmung in dem Fluidströmungspfad, in dem sie angeordnet ist, unterbricht, und einer Durchlassstellung, in der die Ventilanordnung eine Fluidströmung gestattet, schaltbar ist.

Durch die genannten Anschlussformationen ist es möglich, Fluide in das Gehäuse der Fluid-Versorgungsschnittstelle und damit in den Strömungsraum einzuleiten und aus diesem auszuleiten. Diese Fluide können beispielsweise ein Nährmedium und ein Reinigungsfluid sein. Weiter kann über eine der Anschlussformationen Fluid aus dem Gehäuse, also aus dem im Gehäuse definierten Strömungsraum, ausgeleitet werden.

Mit der wenigstens einen Kopplungsformation kann die Fluid-Versorgungsschnittstelle mit einem Zellkulturbehälter fluidübertragend gekoppelt werden. Somit ist es möglich, ein- und dieselbe Fluid-Versorgungsschnittstelle nacheinander mit jeweils einem aus einer Mehrzahl von Zellkulturbehältern in fluidübertragenden Kopplungseingriff zu bringen und dadurch die Möglichkeit zu erhalten, Fluid aus dem Strömungsraum der Fluid-Versorgungsschnittstelle in den jeweils angekoppelten Zellkulturbehälter einzuleiten oder aus diesem in den Strömungsraum der Fluid-Versorgungsschnittstelle auszuleiten. Beispielsweise kann aus einem fluidübertragend an die Kopplungsformation angekoppelten Zellkulturbehälter verbrauchtes oder nicht mehr frisches Nährmedium über den Kopplungsströmungspfad vom Zellkulturbehälter in den Strömungsraum ausgeleitet und von diesem durch den oben genannten dritten Fluidströmungspfad aus dem Strömungsraum durch die dritte Anschlussformation hindurch zu einem Abfluss oder einem Sammelbehälter geleitet werden.

Durch die drei gesondert voneinander ausgebildeten Anschlussformationen ist, wie nachfolgend im Detail erläutert werden wird, die Voraussetzung geschaffen, die Fluid-Versorgungsschnittstelle mit unterschiedlichen Zellkulturbehältern in Kontakt zu bringen, ohne im Falle eines Zellkulturbehälters mit verunreinigtem Inhalt eine Kreuzkontamination nachfolgend angekoppelter Zellkulturbehälter befürchten zu müssen. Denn durch die erste Anschlussformation hindurch kann über den ersten Fluidströmungspfad beispielsweise Nährmedium von einem Nährmediumreservoir in den Strömungsraum eingeleitet und von diesem weiter über den Kopplungsströmungspfad in einen fluidübertragend angekoppelten Zellkulturbehälter weitergeleitet werden. Die entsprechenden Ventilanordnungen können durch die Steueranordnung berührungslos über die felderzeugenden Signalmittel zwischen Sperrstellung und Durchlassstellung geschaltet werden, ohne dass hierfür ein mechanischer Zugriff auf einen Venilkörper oder/und Ventilsitz der Ventilanordnung notwendig wäre.

Ebenso kann, wie oben bereits geschildert, durch die Kopplungsformation hindurch längs des Kopplungsströmungspfades im Zellkulturbehälter nicht mehr erwünschtes Nährmedium aus diesem in den Strömungsraum ausgeleitet und über den dritten Fluidströmungspfad aus dem Strömungsraum zu einem Abfluss- oder Sammelbehälter abgeleitet werden.

Durch Vorsehen der zweiten Anschlussformation mit dem dort ausgebildeten zweiten Fluidströmungspfad kann beispielsweise ein Reinigungsfluid aus einem an die zweite Anschlussformation fluidübertragend angeschlossenen Reinigungsfluidreservoir in den Strömungsraum eingeleitet und über den dritten Fluidströmungspfad aus diesem ausgeleitet werden. Somit kann der Strömungsraum durch das Reinigungsfluid reinigend gespült werden, indem das Reinigungsfluid von der zweiten Anschlussformation zur dritten Anschlussformation durch den Strömungsraum hindurch strömt.

Durch geeignete Lage der ersten, der zweiten und der dritten Anschlussformation kann sichergestellt werden, dass das reinigende Spülen alle Fluidströmungspfade erfasst, vor allem jene, die für die Einleitung frischen Nährmediums in den Zellkulturbehälter notwendig sind.

Für eine unerwünschte Kreuzkontamination bei der Versorgung unterschiedlicher Zellkulturbehälter durch ein- und dieselbe Fluid-Versorgungsschnittstelle ist gerade das Einleiten frischen Nährmediums in einen an die Kopplungsformation angekoppelten Zellkulturbehälter kritisch, da nur über den Kopplungsströmungspfad verunreinigtes Material in einen bisher einwandfreien Zellkulturbehälter gelangen kann. Das Ausleiten von Nährmedium aus Zellkulturbehältern ist dagegen so lange unkritisch, so lange das aus einem Zellkulturbehälter ausgeleitete Nährmedium lediglich entsorgt oder gesondert aufbewahrt werden soll.

Die Ventilanordnungen können - worauf oben ebenfalls bereits hingewiesen wurde - vorteilhaft berührungslos durch ein felderzeugendes Signalmittel, welches mit einem entsprechend feldsensitiven Gegensignalmittel der Ventilanordnung zusammenwirkt, zwischen Sperrstellung und Durchlassstellung geschaltet werden. Somit kann die Ventilanordnung hermetisch von der Außenwelt abgeschlossen sein. Die Ventilanordnungen bzw. Bauteile derselben kommen somit lediglich mit Fluiden in Kontakt, welche längs ihrer jeweiligen Fluidströmungspfade strömen können. Eine Verschmutzung von Ventilanordnungen von außen ist durch die Schaltung mittels felderzeugender Signalmittel und entsprechend feldsensitiver Gegensignalmittel bei fehlender Außenverbindung dagegen ausgeschlossen.

Wenn in dieser Anmeldung ausgesagt ist, dass eine Ventilanordnung ohne eine von der Ventilanordnung bis zur Außenseite des Gehäuses kontinuierlich durchgehende signal- oder/und energieübertragende körperliche Verbindung in dem Gehäuse aufgenommen ist, so schließt dies Fälle mechanischer Signal- oder/und Energieübertragung durch Gestänge oder/und Getriebe, Schrauben- oder Spindeltriebe und dergleichen ein, mit welchen von außerhalb des Gehäuses ein Ventilkörper der Ventilanordnung von seinem Ventilsitz abgehoben und wieder auf diesen aufgesetzt werden könnte. Ebenfalls vom Begriff der kontinuierlich durchgehenden signal- oder/und energieübertragenden körperlichen Verbindung umfasst sind von der Außenseite des Gehäuses zur Ventilanordnung oder einem Ventilantrieb durchgehend laufende Drähte, mit welchen elektrische Energie zu einem elektrischen Antrieb geleitet werden kann, mit dem wiederum ein Ventilkörper der Ventilanordnung zur Bewegung zwischen Sperrstellung und Durchlassstellung angetrieben werden kann.

Zur Vermeidung jeglichen Einflusses von außen auf die Ventilanordnungen der Anschlussformationen soll daher jede Ventilanordnung mit Ventilsitz und Ventilkörper vollständig von dem Gehäuse umgeben sein, ohne dass diese Umgebung durch eine körperliche signal- oder/und energieübertragende Verbindung durchbrochen wäre. Einzige Ausnahme des vollständig Umgebenseins der Ventilanordnung durch das Gehäuse sind die den Ventilanordnungen zugeordneten Fluidströmungspfade, welche von Material des Gehäuses frei bleiben müssen, um dort eine Fluidströmung längs des jeweiligen Fluidströmungspfades zu ermöglichen.

Durch eine entsprechende Ansteuerung der Steueranordnung können die Ventilanordnungen der Fluid-Versorgungsschnittstelle beispielsweise derart geschaltet werden, dass zunächst nach Herstellung eines fluidübertragenden Kopplungseingriffs der Kopplungsformation mit einer entsprechenden Gegenkopplungsformation eines Zellkulturbehälters der Strömungsraum über den zweiten und den dritten Fluidströmungspfad mit Reinigungsfluid gespült wird. Dadurch können auch etwaige Ventilanordnungen an der entsprechenden Gegenkopplungsformation des Zellkulturbehälters gereinigt werden.

Im Anschluss kann über den Kopplungsströmungspfad und den dritten Fluidströmungspfad Nährmedium aus dem Zellkulturbehälter ausgeleitet werden. Im Anschluss daran kann über den zweiten und den dritten Fluidströmungspfad erneut ein Spülen des Strömungsraums der Fluid-Versorgungsschnittstelle mit Reinigungsfluid und dadurch ein erneutes Reinigen des Strömungsraums bewirkt werden.

Im Anschluss daran kann über den ersten und den dritten Fluidströmungspfad der Strömungsraum der Fluid-Versorgungsschnittstelle, eventuell nach einer weiteren Reinigungsspülung mit frischem Nährmedium gespült werden, um etwaige Reste von Reinigungsfluid aus der Fluid-Versorgungsschnittstelle zu entfernen.

Wiederum im Anschluss daran kann über den ersten Fluidströmungspfad und den Kopplungsströmungspfad frisches Nährmedium in den jeweils angekoppelten Zellkulturbehälter eingeleitet werden.

Im Anschluss daran kann der nun mit frischem Nährmedium versorgte Zellkulturbehälter von der Fluid-Versorgungsschnittstelle, eventuell nach einer weiteren Reinigungsspülung mit Reinigungsfluid über den zweiten und den dritten Fluidströmungsraum, abgekoppelt und ein weiterer Zellkulturbehälter an diese angekoppelt werden. Die obigen Spül- und Ein- bzw. Ausleitungsmaßnahmen können dann erneut ablaufen. Dies kann für beliebig viele Zellkulturbehälter wiederholt werden, so dass eine n:1-Anzahlbeziehung zwischen einer Mehrzahl von Zellkulturbehältern und einem Nährmediumreservoir realisiert werden kann.

Grundsätzlich kann jedoch auch daran gedacht werden, nur frisches Nährmedium in einen Zellkulturbehälter einzuleiten oder nur bereits darin vorhandenes Nährmedium aus dem Zellkulturbehälter auszuleiten. Entscheidend ist, dass durch entsprechende Spülprozeduren die Fluid-Versorgungsschnittstelle zwischen einem fluidübertragenden Kopplungseingriff mit unterschiedlichen Zellkulturbehältern in ausreichendem Maße mit Reinigungsfluid gespült und gereinigt werden kann, um hierdurch die sonst bei Ankopplung ein- und derselben Fluid-Versorgungsschnittstelle an unterschiedlichen Zellkulturbehältern zu befürchtenden Kreuzkontaminationen ausschließen zu können.

Zur Erleichterung einer möglichst wirksamen Reinigung des Strömungsraums der Fluid-Versorgungsschnittstelle kann gemäß einer vorteilhaften Weiterbildung der vorliegenden Erfindung vorgesehen sein, dass der Strömungsraum wenigstens zwischen der Anschlussformation, welche zur Verbindung der Fluid-Versorgungsschnittstelle mit einem Reinigungsfluidreservoir ausgebildet ist - das ist bevorzugt die zweite Anschlussformation - und der Anschlussformation, welche zur Verbindung der Fluid-Versorgungsschnittstelle mit einem Abfluss oder einem Entsorgungsbehälter oder dergleichen ausgebildet ist - das ist bevorzugt dritte Anschlussformation - im wesentlichen geradlinig verläuft. Zur Vermeidung von Zwickelbereichen ist der Strömungsraum bevorzugt im Wesentlichen kreiszylindrisch ausgebildet. Die kreiszylindrische Ausbildung kann jedoch in jenen Bereichen, in welchen der erste, der zweite oder der dritte Fluidströmungspfad oder der Kopplungsströmungspfad in den Strömungsraum mündet, von einer idealen Kreiszylinderform abweichen.

Um zusätzlich zu den oben genannten Funktionen den Funktionsumfang der hier diskutierten Fluid-Versorgungsschnittstelle zu erweitern, etwa um die Möglichkeit der Entnahme von Proben von Medien aus einem Zellkulturbehälter, kann gemäß einer Weiterbildung der vorliegenden Erfindung vorgesehen sein, dass die Fluid-Versorgungsschnittstelle weiter umfasst:
eine von den übrigen dreien gesondert ausgebildete vierte Anschlussformation zur fluidübertragenden Verbindung des Gehäuses mit einer vierten Fluidleitung und
einen vierten Fluidströmungspfad, welcher zur Ausleitung eines Fluids aus dem Strömungsraum zwischen dem Strömungsraum und der vierten Anschlussformation verläuft.

Der vierte Fluidströmungspfad kann beispielsweise zu einem Probeentnahmebehälter oder einem Probeentnahmeauslass führen, an welchem die aus dem Zellkulturbehälter entnommene Medienprobe zur Weiterbearbeitung aufgefangen wird. Durch eine derartige Probenentnahme kann beispielsweise anhand chemischer Analysen des aus einem Zellkulturbehälter entnommenen Nährmediums bestimmt werden, ob diese ausreichend rein ist, die darin kultivierten Zellkulturen erwartungsgemäße Lebenszyklen aufweisen und dergleichen mehr.

Auch der vierte Fluidströmungspfad weist vorzugsweise eine Ventilanordnung auf, wie sie auch der erste, der zweite und der dritte Fluidströmungspfad aufweisen. Zur Vermeidung unnötiger Wiederholungen wird zur Ausgestaltung der Ventilanordnung des vierten Fluidströmungspfads und der damit verbundenen technischen Vorteile auf die obige sowie auf die nachfolgende Beschreibung der Ventilanordnungen des ersten, des zweiten und des dritten Fluidströmungspfads verwiesen, welche ebenso für die Ventilanordnung des vierten Fluidströmungspfads gelten.

Eine oder mehrere der ersten bis vierten Anschlussformation können als lösbare Anschlussformation ausgebildet sein, an welchen eine Fluidleitung lösbar mit dem Gehäuse der Fluid-Versorgungsschnittstelle verbindbar ist, etwa durch lösbare Steckverbindungen, wie sie im Stand der Technik bekannt sind. Dies kann dann hilfreich sein, wenn mit einer oder mehreren Anschlussformationen zu unterschiedlichen Zeiten unterschiedliche Fluidleitungen fluidübertragend verbunden sein sollen. Dies ist bei der bevorzugten Verwendung der hier diskutierten Fluid-Versorgungsschnittstelle für eine Zellkulturanlage zur Versorgung von unterschiedlichen Zellkulturbehältern mit Nährmedium in der Regel jedoch nicht der Fall. Aus Gründen erhöhter Betriebssicherheit und vor allem zur Verbesserung der Hygiene ist daher bevorzugt jede der ersten bis vierten Anschlussformation als eine Daueranschlussformation ausgebildet, an welchen erste bis vierte Fluidleitungen betriebsmäßig dauerhaft mit dem Gehäuse der Fluid-Versorgungsschnittstelle fluidübertragend verbunden sind. Beispielsweise können derartige Anschlussformationen durch Verschrauben, ggf. unter Zwischenanordnung von Dichtungsmitteln, von Anschlussformation und Fluidleitung oder durch Verkleben, Verschweißen, Verlöten derselben und dergleichen realisiert sein. Mit "betriebsmäßig dauerhaft fluidübertragend verbunden" ist dabei gemeint, dass außer im Schadens- oder Wartungsfall nicht vorgesehen ist, eine einmal mit einer Anschlussformation verbundene Fluidleitung während der herkömmlichen Betriebslebenszeit einer Fluid-Versorgungsschnittstelle wieder von dieser zu lösen. Dies steht im Gegensatz zur Kopplungsformation, welche betriebsmäßig geradezu dazu ausgebildet ist, häufig mit einer Gegenkopplungsformation eines Zellkulturbehälters fluidübertragend gekoppelt und von diesem wieder gelöst zu werden.

Eine weitere Erhöhung der in der Fluid-Versorgungsschnittstelle der vorliegenden Anmeldung erzielbaren Hygiene kann durch Vorsehen mehrerer Kopplungsformationen und durch Trennung von deren Funktion erfolgen. So kann beispielsweise die zuvor diskutierte Kopplungsformation eine erste Kopplungsformation sein, durch die beispielsweise im Betrieb der Fluid-Versorgungsschnittstelle ausschließlich frisches Nährmedium in einen daran angekoppelten Zellkulturbehälter eingeleitet wird. Weiter kann die Fluid-Versorgungsschnittstelle eine von dieser ersten Kopplungsformation gesondert ausgebildete zweite Kopplungsformation aufweisen, durch welche beispielsweise bei hergestelltem Kopplungseingriff ausschließlich Nährmedium aus einem angekoppelten Zellkulturbehälter ausgeleitet wird.

Allgemein gilt also zur Verbesserung der Hygiene gemäß einer vorteilhaften Weiterbildung der vorliegenden Erfindung, dass die oben genannte Kopplungsformation eine erste Kopplungsformation sein kann, dass die Fluid-Versorgungsschnittstelle eine von der ersten gesondert ausgebildete zweite Kopplungsformation aufweisen kann, welche zum betriebsmäßig herstellbaren und lösbaren fluidübertragenden Kopplungseingriff mit einer entsprechenden zweiten Gegenkopplungsformation des Zellkulturbehälters ausgebildet ist, und dass die Fluid-Versorgungsschnittstelle dann einen zweiten Kopplungsströmungspfad aufweisen kann, welcher zwischen dem Strömungsraum und der zweiten Kopplungsformation verläuft, um über die zweite Kopplungsformation Fluid aus dem Strömungsraum auszuleiten oder/und in diesen einzuleiten.

Die oben genannte vorteilhafte Funktionstrennung der beiden Kopplungsformationen einer wie oben beschrieben vorteilhaft weitergebildeten Fluid-Versorgungsschnittstelle kann dadurch noch weiter hinsichtlich des erzielbaren Hygienestandards verbessert werden, dass die Fluid-Versorgungsschnittstelle einen zwischen der ersten und der zweiten Kopplungsformation verlaufenden Verbindungsströmungspfad aufweist und die Fluid-Versorgungsschnittstelle in diesem eine Trenn-Ventilanordnung aufweist, welche ohne eine von der Trenn-Ventilanordnung bis zur Außenseite des Gehäuses kontinuierlich durchgehende signal- oder/und energieübertragende körperliche Verbindung vollständig - mit Ausnahme des Verbindungsströmungspfads - von dem Gehäuse umgeben in diesem aufgenommen ist,
wobei der Trenn-Ventilanordnung eine Steueranordnung mit einem ein elektrisches oder/und magnetisches oder/und elektromagnetisches Feld erzeugenden Signalmittel zugeordnet ist, dessen Feld auf ein entsprechend feldsensitives Gegensignalmittel der Trenn-Ventilanordnung berührungslos einwirkt, wobei die Trenn-Ventilanordnung mittels des auf ihr Gegensignalmittel einwirkenden Feldes zwischen einer Sperrstellung, in der die Trenn-Ventilanordnung eine Fluidströmung in dem Verbindungsströmungspfad unterbricht, und einer Durchlassstellung, in der die Trenn-Ventilanordnung eine Fluidströmung gestattet, schaltbar ist. Durch die Trenn-Ventilanordnung kann das Einleiten von Nährmedium in einen angekoppelten Zellkulturbehälter und das Ausleiten von Nährmedium aus diesem strömungsmechanisch vollständig voneinander funktionell und räumlich entkoppelt werden. Bei entsprechender Schaltung der Trenn-Verntilanordnung kann somit der Strömungsraum der Fluid-Versorgungsschnittstelle in zwei Teil-Strömungsräume unterteilt werden, in welchen bei entsprechender Betätigung der Trenn-Ventilanordnung durch den einen hindurch ausschließlich Reinigungsfluid und frisches Nährmedium strömen kann und durch den anderen Teil-Strömungsraum hindurch gebrauchtes Nährmedium aus dem Zellkulturbehälter, Reinigungsfluid, und beim Ausspülen von Reinigungsfluid auch frisches Nährmedium strömen kann. Somit kann eine Störung der Versorgung von Zellkulturbehältern mit frischem Nährmedium durch Verunreinigung mit zuvor abgeleitetem gebrauchtem Nährmedium eines anderen, zuvor angekoppelten Zellkulturbehälters, vermieden werden.

Zur Reinigung des Strömungsraums kann die Trenn-Ventilanordnung in ihre Durchlassstellung geschaltet werden, so dass für die Reinigung und auch für das anschließende Ausspülen von Reinigungsfluid durch frisches Nährmedium der gesamte Strömungsraum von Reinigungsfluid oder/und frischem Nährmedium durchströmbar sein kann.

Zur Vermeidung von unerwünschten Bypass-Strömungen ist es vorteilhaft, wenn der Verbindungsströmungspfad der einzige zwischen der ersten und der zweiten Kopplungsformation verlaufende Fluidströmungspfad ist. Auch dies dient der Vermeidung unerwünschter Kreuzkontamination zeitlich nacheinander angekoppelter Zellkulturbehälter.

Eine sinnvolle Funktionstrennung der beiden Kopplungsformationen und der ihnen zugeordneten Kopplungsströmungspfade kann weiter dadurch unterstützt werden, dass die Trenn-Ventilanordnung derart angeordnet ist, dass durch sie der erste und der zweite Fluidströmungspfad vom zweiten Kopplungsströmungspfad, aber nicht vom ersten Kopplungsströmungspfad trennbar ist, und dass durch sie der dritte Fluidströmungspfad vom ersten Kopplungsströmungspfad, aber nicht vom zweiten Kopplungsströmungspfad trennbar ist. In diesem Fall weist jeder Teil-Strömungsraum einen Kopplungsströmungspfad und wenigstens einen Fluidströmungspfad auf, denn an jeden Teil-Strömungsraum ist jeweils eine Kopplungsformation und wenigstens eine Anschlussformation fluidübertragend angeschlossen.

Genauer münden bevorzugt der erste und der zweite Fluidströmungspfad in den einen Teil-Strömungsraum und mündet der dritte Fluidströmungspfad in den jeweils anderen Teil-Strömungsraum.

Dann, wenn die obige Zuordnung der Fluidströmungspfade bzw. der ihnen zugeordneten Anschlussformationen eingehalten wird, also etwa Einleitung eines frischen Nährmediums in den Strömungsraum über den ersten Kopplungsströmungspfad, Einleitung eines Reinigungsfluids in den Strömungsraum über den zweiten Fluidströmungspfad und Ausleitung von Fluiden aus dem Strömungsraum über dem dritten Fluidströmungspfad, kann der eine Teil-Strömungsraum zur Einleitung von frischem Nährmedium in einen angekoppelten Zellkulturbehälter und kann der jeweils andere Teil-Strömungsraum zur Ausleitung von benutztem Nährmedium aus dem angekoppelten Zellkulturbehälter verwendet werden, wobei diese beiden Funktionen hygienisch vorteilhaft durch die Trenn-Ventilanordnung voneinander getrennt sind.

Dann, wenn die zuvor genannte vierte Anschlussformation mit einem vierten Fluidströmungspfad an der Fluid-Versorgungsschnittstelle ausgebildet ist, mündet der vierte Fluidströmungspfad vorteilhafterweise in den selben Teil-Strömungsraum wie der ebenfalls zur Ausleitung von Fluid aus dem Strömungsraum vorgesehene dritte Fluidströmungspfad. Hierdurch wird überdies eine symmetrische Verteilung von Fluidströmungspfaden und Kopplungsströmungspfaden in den beiden durch die Trenn-Ventilanordnung gebildeten Teil-Strömungsräumen erreicht.

Da bei der Ausleitung von Fluid aus einem an die Fluid-Versorgungsschnittstelle angekoppelten Zellkulturbehälter die Möglichkeit einer Verschmutzung des ausleitenden Fluidströmungspfads, etwa durch Stoffwechselprodukte der Zellkulturen im Zellkulturbehälter, größer ist als bei den von einem Reservoir mit frischem Fluid (etwa Nährmedium oder/und Reinigungsfluid) ausgehenden Fluidströmungspfaden, kann es dann, wenn eine gesonderte Möglichkeit zur Entnahme von Medienproben aus einem angekoppelten Zellkulturbehälter gewünscht ist, vorteilhaft sein, gesonderte Kopplungsströmungspfade für die Entnahme von Medienproben aus einem zellkulturbehälter einerseits und für das bloße Entsorgen von gebrauchtem Nährmedium andererseits vorzusehen.

Dementsprechend kann gemäß einer Weiterbildung der vorliegenden Erfindung zur Verwirklichung dieser hygienesteigernden Maßnahme konstruktiv vorgesehen sein, dass die Fluid-Versorgungsschnittstelle eine von der ersten und der zweiten gesondert ausgebildete dritte Kopplungsformation und einen dritten Kopplungsströmungspfad aufweist, welcher zwischen dem Strömungsraum und der dritten Kopplungsformation verläuft, um über die dritte Kopplungsformation Fluid aus dem Strömungsraum auszuleiten oder/und in diesen einzuleiten, wobei die dritte Kopplungsformation zum betriebsmäßig herstellbaren und lösbaren fluidübertragenden Kopplungeingriff mit einer entsprechenden dritten Gegenkopplungsformation des Zellkulturbehälters ausgebildet ist.

Wiederum kann es aus den oben bereits angeregten Erwägungen zur Verbesserung der mit der Fluid-Versorgungsschnittstelle erzielbaren Hygiene vorteilhaft sein, die einzelnen Kopplungsformationen durch Trenn-Ventilanordnungen voneinander strömungsmechanisch trennbar auszugestalten. Hierzu kann gemäß einer Weiterbildung der vorliegenden Erfindung vorgesehen sein, dass der oben genannte Verbindungsströmungspfad ein erster Verbindungsströmungspfad und dass die oben genannte Trenn-Ventilanordnung eine erste Trenn-Ventilanordnung ist und dass die Fluid-Versorgungsschnittstelle einen zwischen der zweiten und der dritten Kopplungsformation verlaufenden zweiten Verbindungsströmungspfad aufweist und die Fluid-Versorgungsschnittstelle in diesem eine von der ersten gesonderte zweite Trenn-Ventilanordnung aufweist, welche ohne eine von der zweiten Trenn-Ventilanordnung bis zur Außenseite des Gehäuses kontinuierlich durchgehende signal- oder/und energieübertragende körperliche Verbindung vollständig - mit Ausnahme des zweiten Verbindungsströmungspfads - von dem Gehäuse umgeben in diesem aufgenommen ist,
wobei der zweiten Trenn-Ventilanordnung eine Steueranordnung mit einem ein elektrisches oder/und magnetisches oder/und elektromagnetisches Feld erzeugenden Signalmittel zugeordnet ist, dessen Feld auf ein entsprechend feldsensitives Gegensignalmittel der zweiten Trenn-Ventilanordnung berührungslos einwirkt, wobei die zweite Trenn-Ventilanordnung mittels des auf ihr Gegensignalmittel einwirkenden Feldes zwischen einer Sperrstellung, in der die zweite Trenn-Ventilanordnung eine Fluidströmung in dem zweiten

Verbindungsströmungspfad unterbricht, und einer Durchlassstellung, in der die zweite Trenn-Ventilanordnung eine Fluidströmung gestattet, schaltbar ist.

Die Trenn-Ventilanordnungen sind vorzugsweise mit den oben genannten Ventilanordnungen der Anschlussformationen identisch ausgebildet. Dies erleichtert Fertigung und Montage der Fluid-Versorgungsschnittstelle, da nur eine Art von Ventilanordnung hergestellt und in der Fluid-Versorgungsschnittstelle verbaut und betrieben werden muss. Insofern gilt das oben und unten zu den Ventilanordnungen Gesagte für die Trenn-Ventilanordnungen entsprechend.

Wiederum ist zur Vermeidung unerwünschter Bypass-Strömungen vorzugsweise vorgesehen, dass der zweite Verbindungsströmungspfad der einzige zwischen der zweiten und der dritten Kopplungsformation verlaufende Fluidströmungspfad ist. Dadurch kann gewährleistet werden, dass durch eine einzige Trenn-Ventilanordnung die zweite und die dritte Kopplungsformation strömungsmechanisch vollständig voneinander trennbar sein können. Ebenso kann durch oben erwähnte Maßnahme erreicht werden, dass mit der ersten Trenn-Ventilanordnung die erste und die zweite Kopplungsformation strömungsmechanisch vollständig voneinander trennbar sind. Vorzugsweise sind zur Gewährleistung einer sicheren Reinigung des Strömungsraums die mit einem Reinigungsfluidreservoir verbundene Anschlussformation und die mit einem Abfluss oder/und einem Entsorgungsbehälter verbundene Anschlussformation derart vorgesehen, dass auf dem Weg von der ersteren zur letzteren die erste und die zweite Trenn-Ventilanordnung sich in Durchlassstellung befinden müssen und somit von Reinigungsfluid durchströmt werden. Dies gewährleistet bei Spülung des Strömungsraums auch eine Reinigung der beiden Trenn-Ventilanordnungen.

Zur Erzielung obiger Funktionen: Einleitung von Reinigungsfluid, Einleitung von frischem Nährmedium, Entnahme von Fluidproben aus einem angekoppelten Zellkulturbehälter und Entsorgung von gebrauchtem Nährmedium bei geringsmöglicher Gefahr einer späteren Kreuzkontamination kann gemäß einer weiteren vorteilhaften Weiterbildung der vorliegenden Erfindung vorgesehen sein, dass die zweite Trenn-Ventilanordnung derart angeordnet ist, dass entweder durch sie der dritte Fluidströmungspfad vom zweiten Kopplungsströmungspfad, aber nicht vom dritten Kopplungsströmungspfad trennbar ist, und dass durch sie der vierte Fluidströmungspfad vom dritten Kopplungsströmungspfad, aber nicht vom zweiten Kopplungsströmungspfad trennbar ist, oder dass durch sie der vierte Fluidströmungspfad vom zweiten Kopplungsströmungspfad, aber nicht vom dritten Kopplungsströmungspfad trennbar ist, und dass durch sie der dritte Fluidströmungspfad vom dritten Kopplungsströmungspfad, aber nicht vom zweiten Kopplungsströmungspfad trennbar ist.

Die berührungslos signal- oder/und energieübertragenden Signalmittel und Gegensignalmittel können beispielsweise ein elektrisches Feld zwischen sich zur Signal- oder/und Energieübertragung nutzen. Hierzu können das Signalmittel und das Gegensignalmittel jeweils eine Elektrode zum Aufbau des elektrischen Feldes zwischen sich umfassen, wobei das Gegensignalmittel mit einem piezoelektrischen Aktuator der Ventilanordnung derart zusammenwirken kann, dass das zwischen Signalmittel und Gegensignalmittel aufgebaute elektrische Feld eine Gestaltänderung des piezoelektrischen Aktuators bewirkt. Bei geeignetem Einbau des piezoelektrischen Aktuators in die Ventilanordnung kann wiederum die Gestaltänderung des piezoelektrischen Aktuators eine Verstellung der Ventilanordnung zwischen der Sperrstellung und der Durchlassstellung bewirken. Material des Gehäuses zwischen den Elektroden kann als Dielektrikum wirken.

Zwar sind voraussichtlich mit dieser Anordnung aufgrund der lediglich geringen Gestaltänderung von piezoelektrischen Aktuatoren nur geringe Strömungsspalte zwischen Ventilkörper und zugeordneten Ventilsitz herstellbar. Gleichwohl können diese ausreichen, um die Ventilanordnung zwischen Sperrstellung und Durchlassstellung zu verstellen, also einen Zustand hervorzurufen, bei welchem eine Fluidströmung durch die Ventilanordnung nicht möglich ist (Sperrstellung) und einen davon verschiedenen Zustand, bei welchem eine derartige Fluidströmung möglich ist (Durchlassstellung).

Alternativ können das Signalmittel und das Gegensignalmittel einen Magneten und ein auf dessen Magnetfeld ansprechendes ferromagnetisches oder/und magnetisiertes Bauteil aufweisen. Dann kann das zwischen Signalmittel und Gegensignalmittel wirkende Magnetfeld eine Verlagerung des Gegensignalmittels bewirken. Diese Verlagerung des Gegensignalmittels kann wiederum eine Verstellung der Ventilanordnung zwischen Sperrstellung und Durchlasstellung bewirken. Dies kann beispielsweise konstruktiv dadurch realisiert sein, dass das Gegensignalmittel zur gemeinsamen Bewegung mit einem Ventilkörper einer Ventilanordnung gekoppelt ist, so dass eine Verlagerung des Gegensignalmittels den Ventilkörper von seinem Ventilsitz abhebt oder ihn wieder zur Anlage an diesen bringt. Ebenso kann das Gegensignalmittel selbst in einer besonders bevorzugten Ausführungsform der Ventilkörper sein.

Gemäß einer weiteren Alternative soll nicht ausgeschlossen sein, dass das Signalmittel einen Magneten umfasst und dass das Gegensignalmittel ein auf das Magnetfeld des Magneten des Signalmittels induktiv ansprechendes elektrisch leitendes Bauteil umfasst. In diesem Falle kann das zwischen Signalmittel und Gegensignalmittel wirkende Magnetfeld eine Induktion im Gegensignalmittel bewirken und diese Induktion kann wiederum eine Verstellung der Ventilanordnung zwischen Sperrstellung und Durchlassstellung bewirken. Beispielsweise kann so induktiv ein in dem Gehäuse vorgesehener Aktuator, etwa ein Elektromotor oder ein Elektromagnet mit verlagerbarem Anker, durch Induktion mit ausreichend elektrischer Energie versorgt werden, um den Aktuator zur Bewegung anzutreiben. Ein mit dem Aktuator gekoppelter Ventilkörper kann somit von seinem Ventilsitz abgehoben und wieder in Anlage an diesen gebracht werden.

Schließlich kann in einer technisch aufwendigeren Alternative das Signalmittel auch einen Sender elektromagnetischer Wellen umfassen. Dies können optische Signale oder Funksignale sein. Das Gegensignalmittel umfasst dann einen entsprechenden Empfänger. Die Ventilanordnung kann einen von dem Gehäuse umgebenen Energiespeicher und einen Aktuator aufweisen, welche derart untereinander mit dem Gegensignalmittel gekoppelt sind, dass das Gegensignalmittel abhängig von den empfangenen elektromagnetischen Wellen den aus dem Energiespeicher gespeisten Aktuator zur Schaltung der Ventilanordnung zwischen der Sperrstellung und der Durchlassstellung steuert. In diesem Falle werden Signalmittel und Gegensignalmittel wie eine Fernsteuerung verwendet, etwa vergleichbar mit der bekannten Fernsteuerung eines Fernsehers oder eines ferngesteuerten Spielzeugs. Der vom Gehäuse der Fluid-Versorgungsschnittstelle umgebene Energiespeicher, welcher definitionsgemäß von außen nicht durch eine körperliche signal- oder energieübertragende Verbindung zugänglich sein soll, kann als elektrischer Energiespeicher induktiv aufgeladen werden. Der Aktuator kann wiederum ein Elektromotor oder ein Elektromagnet mit beweglichem Anker sein, wobei in letztgenanntem Fall der Anker abhängig vom Bestromungszustand des Elektromagneten unterschiedliche Positionen einnimmt. Ist der Ventilkörper einer Ventilanordnung zur gemeinsamen Bewegung mit einem beweglichen Ausgabeteil des Aktuators gekoppelt, kann somit in nachvollziehbarer Weise die Ventilanordnung zwischen Sperrstellung und Durchlassstellung geschaltet werden.

Aus Gründen der einfachen und robusten Bauart bei gleichzeitig besonders sicheren Betrieb und leichter Reinigbarkeit durch das durch den Signalraum hindurch strömbare Reinigungsfluid ist von obigen Alternativen jene Weiterbildung bevorzugt, gemäß welcher das Signalmittel und das Gegensignalmittel einen Magneten einerseits und ein auf dessen Magnetfeld ansprechendes ferromagnetisches oder/und magnetisiertes Bauteil andererseits umfassen, wobei das Gegensignalmittel aus Gründen einer möglichst geringen Bauteileanzahl bevorzugt ein Ventilkörper der Ventilanordnung ist, der unter Einwirkung des zwischen Signalmittel und Gegensignalmittel wirkenden Magnetfelds von seinem Ventilsitz weg oder/und zur dichtenden Anlage an diesen verlagerbar ist.

Mit anderen Worten: die Steueranordnung ist gemäß einer bevorzugten Weiterbildung der vorliegenden Erfindung zur zeitlichen oder/und örtlichen Veränderung eines von seinem wenigstens einen Signalmittel ausgehenden Magnetfeld ausgebildet.

Diese bevorzugte Ausbildung gestattet überdies, dass die Ventilanordnung magnetisch in die Sperrstellung vorspannbar ist und durch das vom Signalmittel ausgehende Magnetfeld in die Durchlassstellung verstellbar ist. Konstruktiv kann diese Vorspannung dadurch realisiert sein, dass ein Ventilsitz der Ventilanordnung ein permanentmagnetisches oder ein ferromagnetisches Spannbauteil aufweist, so dass zwischen dem Spannbauteil und dem Ventilkörper eine magnetische Spannkraft, insbesondere Anziehungskraft, wirkt, welche den Ventilkörper zur dichtenden Anlage an den Ventilsitz spannt.

Zur Sicherstellung einer möglichst vollständigen Durchströmungsverhinderung der Ventilanordnung in der Sperrstellung kann der Ventilsitz ein elastomeres Anlagebauteil aufweisen, an welchem der Ventilkörper in der Sperrstellung der Ventilanordnung unmittelbar anliegt. Durch Verwendung eines solchen elastomeren Anlagebauteils kann sich der Ventilkörper unter Verformung des Anlagebauteils aufgrund der magnetischen Spannkraft in dieses eindrücken und so für eine flächige Anlage des Ventilkörpers am Anlagebauteil sorgen. Das elastomete Anlagebauteil ist bevorzugt ein ringförmiges Bauteil mit einer Durchströmungsöffnung, welche in der Sperrstellung der Ventilanordnung von dem Ventilkörper verschlossen ist und welche in der Durchlassstellung der Ventilanordnung von einem Fluid durchströmbar ist. Konstruktiv einfach kann die magnetische Spannkraft zur verformenden Anlage des Ventilkörpers an dem elastomeren Anlagebauteil dadurch realisiert werden, dass als magnetische Spannkraft eine Anziehungskraft zwischen dem Ventilkörper und dem Spannbauteil genutzt wird, wobei es dann ausreicht, das elastomere Anlagebauteil zwischen Ventilkörper und Spannbauteil anzuordnen. Das elastomere Anlagebauteil kann aus Gummi, geschlossenzelligem Schaumstoff, Silikon und dergleichen gebildet sein. Das Spannbauteil kann aus mehreren Teilbauteilen bestehen, was jedoch seine Montage erschwert. Bevorzugt ist das Spannbauteil ein permanentmagnetisches Ringbauteil, das, wie die bevorzugte Ausführungsform des elastomeren Anlagebauteils, eine Öffnung umgibt, die in der Durchlassstellung der Ventilanordnung von Fluid durchströmbar ist.

Für den oben geschilderten bevorzugten Fall, bei welchem Signalmittel und Gegensignalmittel einen Magneten einerseits und ein auf dessen Magnetfeld ansprechendes ferromagnetisches oder/und magnetisiertes Bauteil andererseits umfassen, kann das Signalmittel einen örtlich verlagerbaren Permanentmagneten umfassen. Durch Annäherung des örtlich verlagerbaren Permanentmagneten an das Gegensignalmittel, welches bevorzugt der Ventilkörper selbst ist, kann dieser von seiner Vorspannstellung, welche bevorzugt der Sperrstellung der Ventilanordnung zugeordnet ist, entfernt werden, was gleichbedeutend mit einer Schaltung der Ventilanordnung in die Durchlassstellung ist. Einzige Voraussetzung hierfür ist, dass das von dem Permanentmagneten des Signalmittels ausgehende Magnetfeld auf den Ventilkörper (Gegensignalmittel) stärker einwirkt als das vom Spannbauteil ausgehende Magnetfeld, so dass ab einer gewissen Annäherung des Permanentmagneten des Signalmittels die von ihm ausgehende und auf das Gegensignalmittel einwirkende magnetische Kraft, vorzugsweise Anziehungskraft, größer ist als die vom Spannbauteil ausgehende magnetische Spannkraft, so dass die vom Permanentmagneten ausgehende magnetische Kraft überwiegt und eine Verstellung des Gegensignalmittels, insbesondere des Ventilkörpers, bewirkt.

Zusätzlich oder alternativ zu einem örtlich verlagerbaren Permanentmagneten kann das Signalmittel einen Elektromagneten umfassen, welcher ein abhängig von seiner Bestromung zeitlich unterschiedlich starkes Magnetfeld erzeugt. Auch mit einem derartigen Elektromagneten kann durch ausreichend starke Bestromung ein Magnetfeld erzeugt werden, dessen magnetische Kraft die magnetische Spannkraft des Spannbauteils überwiegt und somit für eine Verstellung des Gegensignalmittels, insbesondere des Ventilkörpers sorgt. Damit wird wiederum eine Schaltung der Ventilanordnung zwischen Sperrstellung und Durchlassstellung bewirkt.

Eine besonders einfache, aber effektive Steuerung der Ventilanordnung der Fluid-Versorgungsschnittstelle kann dadurch erfolgen, dass die Steueranordnung eine Mehrzahl von Sätzen von Signalmitteln aufweist, wobei die Signalmittel eines Satzes jeweils eine Ventilstellungskonfiguration von Ventilanordnungen der Fluid-Versorgungsschnittstelle definieren. Somit kann durch einen Satz von Signalmitteln genau eine Ventilstellungskonfiguration definiert sein. Je nachdem, welcher Satz von Signalmitteln den Gegensignalmitteln in der Fluid-Versorgungsschnittstelle angenähert wird, können somit unterschiedliche Ventilstellungskonfigurationen schnell und eindeutig bei gleichzeitig geringer Fehleranfälligkeit geschaltet werden. Der oben geschilderte Betrieb zur Ausleitung von Fluid aus einem angekoppelten Zellkulturbehälter, zur Reinigung und zur Spülung der Fluid-Versorgungsschnittstelle und zum Einleiten von frischem Nährmedium in einen angekoppelten Zellkulturbehälter zeigt, dass selbst in der komplexesten Ausbauform der Fluid-Versorgungsschnittstelle mit drei Kopplungsformationen und vier Anschlussformationen im Wesentlichen sechs Ventilstellungskonfigurationen ausreichen, nämlich je eine für eine Grundstellung (beispielsweise alle Ventilanordnungen in Sperrstellung) während einer Verlagerung der Fluid-Versorgungsschnittstelle zwischen zwei Kopplungseingriffen mit unterschiedlichen Zellkulturbehältern, für das Ausleiten von Medium aus einem Zellkulturbehälter, für das Reinigen der Fluid-Versorgungsschnittstelle bzw. ihres Strömungsraums, für das Spülen desselben mit frischem Nährmedium, für die Entnahme einer Medienprobe aus dem Zellkulturbehälter und für das Einleiten von frischem Nährmedium in einen angekoppelten Zellkulturbehälter. Durch Bereitstellung von sechs Sätzen von Signalmitteln, oder einem Satz von Signalmitteln, mit sechs unterschiedlichen Schaltzuständen im Falle von Elektromagneten als Signalmitteln, kann somit die Fluid-Versorgungsschnittstelle vollständig betrieben werden.

Eine bauraumsparende Unterbringung dieser Sätze von Signalmitteln kann konstruktiv dadurch gelöst werden, dass die Steueranordnung eine um eine Walzenachse drehbare Walze aufweist, wobei die Mehrzahl von Signalmittelsätzen in Umfangsrichtung um die Walzenachse derart verteilt angeordnet sind, dass durch Verdrehung der Walze unterschiedliche Ventilstellungskonfigurationen der Fluid-Versorgungsschnittstelle einstellbar sind.

Zwar kann das Annähern der Signalmittelsätze durch beispielsweise Verdrehung der oben genannten Walze ausreichen, die einzelnen Ventilanordnungen an einer Fluid-Versorgungsschnittstelle eindeutig zu schalten. Jedoch kann es unter Umständen zu unerwünschten zeitlichem Versatz zwischen dem Schalten unterschiedlicher Ventilanordnungen der Fluid-Versorgungsschnittstelle kommen.

Eine möglichst präzise Schaltung der Ventilanordnungen der Fluid-Versorgungsschnittstelle kann in vorteilhafter Weise dadurch erreicht werden, dass die Steueranordnung eine Vermittlungsanordnung aufweist, welche zwischen einem Signalmittel und einer durch das Signalmittel verstellbaren Ventilanordnung angeordnet ist, wobei die Vermittlungsanordnung wenigstens einen zwischen einer der Ventilanordnung näher gelegenen Aktivstellung und einer dem Signalmittel näher gelegenen Inaktivstellung verlagerbaren Magneten, insbesondere Permanentmagneten aufweist.

Beispielsweise kann die Vermittlungsanordnung für jedes Signalmittel eines Satzes von Signalmitteln einen verlagerbaren Magneten aufweisen.

Der wenigstens eine verlagerbare Magnet ist vorzugsweise in eine seiner Stellungen vorgespannt. Hierzu können eigens Spannmittel vorgesehen sein. Diese können gemäß einer bevorzugten Ausführungsform jedoch entfallen, wenn die Vorspannung des wenigstens einen verlagerbaren Magnets durch Ausnutzung der Schwerkraft erfolgt. Vorzugsweise ist der wenigstens eine verlagerbare Magnet in seine Inaktivstellung vorgespannt, so dass er ohne weitere Maßnahme durch die Steueranordnung eine Schaltung der ihm zugeordneten Ventilanordnung verhindert. Gemäß obiger bevorzugter Weiterbildung der vorliegenden Erfindung sind die Ventilanordnungen in ihre Sperrstellung vorgespannt, so dass ohne weitere Maßnahme eine Durchströmung einer Ventilanordnung der Fluid-Versorgungsschnittstelle nicht möglich ist. Somit wird an der Fluid-Versorgungsschnittstelle durch die hier vorgestellte Weiterbildung eine Failsafe-Maßnahme verwirklicht.

Grundsätzlich kann daran gedacht sein, die Ventilanordnungen derart auszubilden, dass sie ausschließlich durch die Steueranordnung zwischen Sperrstellung und Durchlassstellung schaltbar sind. Es kann jedoch in bestimmten Betriebsstellungen vorteilhaft sein, wenn diese unabhängig von ihrer Steueranordnung durch einen ausreichend hohen Fluiddruck im zugeordneten Fluidströmungspfad in einer Durchlassströmungsrichtung durchlässig werden. Somit kann beispielsweise unabhängig von der Schaltung einer Ventilanordnung eine Befüllung eines Zellkulturbehälters mit frischem Nährmedium unterstützt werden. Ebenso kann das Einleiten von Reinigungsfluid in dem Strömungsraum unterstützt werden.

Gemäß einer vorteilhaften Weiterbildung der vorliegenden Erfindung ist daher vorgesehen, dass wenigstens ein Teil der Ventilanordnungen, vorzugsweise alle Ventilanordnungen, in einer Durchlassströmungsrichtung längs des Fluidströmungspfads, in dem sie angeordnet sind, durch einen vorbestimmten Fluiddruckunterschied von der Sperrstellung in die Durchlassstellung verstellbar sind, in der entgegengesetzten Strömungsrichtung dagegen nicht, wobei bevorzugt die Durchlassströmungsrichtung des ersten und des zweiten Fluidströmungspfads in den Strömungsraum hinein gerichtet ist und die Durchlassströmungsrichtung des dritten Fluidströmungspfads aus dem Strömungsraum hinaus gerichtet ist.

Vorteilhafterweise sind die Zellkulturbehälter an den Gegenkopplungsformationen ebenfalls mit Ventilanordnungen versehen, wie sie oben beschrieben wurden. Dadurch wird die Anordnung einer Ventilanordnung an den Kopplungsströmungspfaden bei gelöstem Kopplungseingriff entbehrlich. Das heißt, die Kopplungsformation der Fluid-Versorgungsschnittstelle weist bevorzugt zur Verringerung der zur Bildung der Fluid-Versorgungsschnittstelle notwendigen Bauteile keine derartige Ventilanordnung auf.

Mit einem Vorsehen von Ventilanordnungen in den Gegenkopplungsformationen des Zellkulturbehälters wird überdies sichergestellt, dass sich die Befüllung des Zellkulturbehälters nach Lösen des Kopplungseingriffs zwischen Fluidversorgungsschnittstelle und Zellkulturbehälter nicht ändert, sondern die Kopplungsströmungspfade zellkulturbehälterseitig von dort vorgesehenen Ventilanordnungen gesperrt bleiben. Vorzugsweise ist die Steueranordnung zur gemeinsamen Bewegung mit der Fluid-Versorgungsschnittstelle verbunden. Dadurch ist aufgrund ausreichender Annäherung von Steuer-und Ventilanordnung vorteilhaft nur die Ventilanordnung in der Gegenkopplungsformation des jeweils gerade angekoppelten Zellkulturbehälters überhaupt schaltbar.

Die eingangs genannte Aufgabe wird gemäß einem weiteren Gesichtspunkt der vorliegenden Erfindung ebenfalls gelöst durch eine Zellkulturanlage mit wenigstens einem Zellkulturbehälter zur Aufnahme und Versorgung adhärenter Zellen darin, mit einem Nährmediumreservoir, mit einem Reinigungsfluidreservoir und mit einer Fluid-Versorgungsstelle, wie sie vorstehend beschrieben und vorteilhaft weitergebildet wurde. Die Einbindung der oben stehend beschriebenen Fluid-Versorgungsschnittslle in die Zellkulturanlage mit ihren übrigen genannten Komponenten erfolgt mit der Maßgabe, dass:
- die erste Anschlussformation das Gehäuse mit dem Nährmediumreservoir fluidübertragend verbindet und somit der erste Fluidströmungspfad zwischen dem Strömungsraum und dem Nährmediumreservoir verläuft,
- die zweite Anschlussformation das Gehäuse mit dem Reinigungsfluidreservoir fluidübertragend verbindet und somit der zweite Fluidströmungspfad zwischen dem Strömungsraum und dem Reinigungsfluidreservoir verläuft,
- die dritte Anschlussformation das Gehäuse mit einem Abfluss fluidübertragend verbindet und somit der dritte Fluidströmungspfad zwischen dem Strömungsraum und dem Abfluss verläuft,
- die Kopplungsformation zum betriebsmäßig herstellbaren und lösbaren fluidübertragenden Kopplungseingriff mit einer Gegenkopplungsformation des Zellkulturbehälters ausgebildet ist,
- das erste Fluid das Nährmedium ist,
- das zweite Fluid das Reinigungsfluid ist,
- der Kopplungsströmungspfad dazu ausgebildet ist, über die Kopplungsformation in einem mit der Gegenkopplungsformation gekoppelten Zustand Nährmedium aus dem Strömungsraum in den Zellkulturbehälter einzuleiten oder/und aus diesem in den Strömungsraum auszuleiten.

Dies entspricht dem im Zusammenhang mit dem Betrieb der Fluid-Versorgungsschnittstelle bereits beschriebenen bevorzugten Anschlussschema. Sofern für die Fluid-Versorgungsschnittstelle oben bereits Vorteile angegeben sind, gelten diese selbstverständlich auch für die Zellkulturanlage, in welcher eine entsprechend an das Nährmediumreservoir, an das Reinigungsfluidreservoir und an den Abschluss angeschlossene Fluid-Versorgungsschnittstelle vorgesehen ist. In der nachfolgenden Beschreibung der Zellkulturanlage aufscheinende vorteilhafte Ausgestaltungen der Fluid-Versorgungsschnittstelle und des Zellkulturbehälters sind selbstverständlich auch an der Fluid-Versorgungsschnittstelle bzw. am Zellkulturbehälter alleine realisierbar.

Die Zellkulturanlage kann weiterhin die erforderliche Anzahl von Förderpumpen aufweisen, um die einzelnen Fluide in den Fluidleitungen gesondert voneinander zu fördern.

Der fluidübertragende Kopplungseingriff zwischen der Kopplungsformation der Fluid-Versorgungsschnittstelle und der Gegenkopplungsformation des Zellkulturbehälters kann in an sich aus dem Stand der Technik bekannter Art und Weise erfolgen, beispielsweise durch strömungsmechanische Buchse-Stecker-Verbindungen, bei welchen ein zapfenartiger, also "männlicher" Stecker oder Stutzen in eine "weibliche" Buchse eingesteckt wird, so dass bei hergestelltem fluidübertragenden Kopplungseingriff ein Längsabschnitt einer Formation aus Kopplungsformation und Gegenkopplungsformation einen Längsabschnitt der jeweils anderen Formation radial außen umgibt. Der Kopplungseingriff kann durch magnetische Haltemittel an Kopplungsformation und Gegenkopplungsformation unterstützt sein. Zusätzlich oder alternativ zu den magnetischen Haltemitteln können auch mechanische Haltemittel vorgesehen sein, beispielsweise in Form einer überwindbaren Verrastung, die bei hergestelltem fluidübertragenden Kopplungseingrif zwischen Kopplungsformation und Gegenkopplungsformation bestehen kann. Die Formationen aus Kopplungsformation und Gegenkopplungsformation können jedoch auch ohne an den Formationen selbst vorgesehene Haltemittel auskommen, beispielsweise wenn eine Bewegungsvorrichtung, welche die Fluid-Versorgungsschnittstelle zwischen den anzukoppelnden Zellkulturbehältern bewegt, bei hergestelltem Kopplungseingriff zwischen Kopplungsformation und Gegenkopplungsformation eine Kraft auf diese Formationen ausübt, welche einem Lösen des Kopplungseingriffs entgegenwirkt.

Die Anmelderin behält sich überdies vor, um gesonderten Schutz für eine Verwendung einer Fluid-Versorgungsschnittstelle, wie sie oben in ihrer Basiskonfiguration und ihren bevorzugten Weiterbildungen beschrieben wurde, in einer Zellkulturanlage zur Kultivierung adhärenter Zellen nachzusuchen.

Wie bereits oben angedeutet wurde, kann der Abfluss am von der Fluidversorgungsschnittstelle fern liegenden Längsende des dritten Fluidströmungspfads eine Entsorgungsdrainage oder ein Entsorgungssammelbehälter sein, in welchem zunächst Fluid gesammelt wird, bis eine ausreichende Menge in dem Entsorgungssammelbehälter vorhanden ist, und diese dann einer Entsorgung zugeführt wird.

Grundsätzlich kann das Reinigungsfluid flüssig oder gasförmig sein. Zur Erzielung einer möglichst großen Reinigungswirkung ist das Reinigungsfluid beforzugt eine Reinigungsflüssigkeit. Auch das Nährmedium kann grundsätzlich flüssig oder gasförmig vorliegen. In der Regel wird das Nährmedium zur Erzielung einer größtmöglichen Nährstoffdichte jedoch als Nährflüssigkeit vorliegen.

Wie oben bereits dargelegt wurde, kann die Zellkulturanlage zum Abgreifen von Proben von Inhalten eines Zellkulturbehälters, so genannten "Samples", eine Sample-Aufnahmevorrichtung aufweisen, in welchen aus einem Zellkulturbehälter entnommene Samples bis zu deren Weiterverarbeitung, etwa durch physikalische oder/und chemische Analyse und Untersuchung, aufgenommen werden. Zum Zwecke einer Vermeidung einer unerwünschten Verunreinigung der entnommenen Samples kann die Fluid-Versorgungsschnittstelle die oben bezeichnete vierte Anschlussformation aufweisen. Für diesen Fall kann an der Zellkulturanlage vorgesehen sein, dass
- die vierte Anschlussformation das Gehäuse mit einer Sample-Aufnahmevorrichtung fluidübertragend verbindet und somit der vierte Fluidströmungspfad zwischen dem Strömungsraum und der Sample-Aufnahmevorrichtung verläuft.

Da bei der hier diskutierten Zellkulturanlage eine Reinigung des Strömungsraums durch Einleiten des Reinigungsfluids in den Strömungsraum über die zweite Anschlussformation und durch Ausleiten des Reinigungsfluids aus dem Strömungsraum durch die dritte Anschlussformation erfolgt, kann eine räumlich möglichst umfangreiche Reinigung des Strömungsraums durch Spülen mit Reinigungsfluid dadurch erfolgen, dass im Strömungsraum ein Strömungsweg von der Ventilanordnung der zweiten Anschlussformation zur Ventilanordnung der dritten Anschlussformation der längste Strömungsweg zwischen zwei Ventilanordnungen oder zwischen einer Ventilanordnung und einer Kopplungsformation ist. In diesem Fall liegt das Reinigungsfluid zwischen Einleiten in den Strömungsraum und Ausleiten aus diesem den längsten Strömungsweg im Strömungsraum zurück, so dass das Reinigungsfluid auf diesen Strömungsweg den größtmöglichen Teil des Strömungsraums durchfließt und Wandabschnitte desselben benetzt und abspült.

Dabei können bei der geschilderten Spülung des Strömungsraums mit Reinigungsfluid zumindest die zu weiteren Anschlussformationen führenden Leitungswege des Strömungsraums dann mitgereinigt werden, wenn im Strömungsraum ein Strömungsweg von der Ventilanordnung der zweiten Anschlussformation zur Ventilanordnung der dritten Anschlussformation an der Ventilanordnung der ersten Anschlussformation und gegebenenfalls an der Ventilanordnung der vierten Anschlussformation vorbei verläuft.

Die Reinigung des Strömungsraums der Fluid-Versorgungsschnittstelle setzt somit auf Strategien, wie sie im Stand der Technik als "Sterilize-In-Place" (kurz "SIP") oder auch als "Clean-In-Place" (kurz: "CIP") bezeichnet sind. Für eine möglichst leistungsfähige SIP- oder CIP-Reinigung, die auch die Ventilkörper beteiligter Ventilanordnungen spülend reinigt, ist es vorteilhaft, wenn der Ventilkörper der Ventilanordnung der ersten Anschlussformation und gegebenenfalls der Ventilkörper der Ventilanordnung der vierten Anschlussformation wenigstens zum Teil, vorzugsweise wenigstens mehr als zur Hälfte in den Strömungsweg von der Ventilanordnung der zweiten Anschlussformation zur Ventilanordnung der dritten Anschlussformation hineinragen. Diese Weiterbildung ist ausdrücklich auch als Weiterbildung der Fluid-Versorgungsschnittstelle für sich alleine genommen zu verstehen.

Aus den oben bereits genannten Gründen ist es vorteilhaft, wenn der oder die Zellkulturbehälter jeweils mehr als nur eine Gegenkopplungsformation aufweist bzw. aufweisen, um beispielsweise durch Trennung von Nährmediumzuleitung und Nährmediumableitung einen höheren Hygienestandard zu erreichen. Hierfür kann gemäß einer vorteilhaften Weiterbildung der Zellkulturanlage in concreto vorgesehen sein, dass die oben genannte Gegenkopplungsformation des Zellkulturbehälters eine erste Gegenkopplungsformation ist, dass der Zellkulturbehälter eine von der ersten Gegenkopplungsformation gesondert ausgebildete zweite Gegenkopplungsformation aufweist, und dass die Zellkulturanlage weiter eine Fluid-Versorgungsschnittstelle gemäß der obigen einschlägigen Beschreibung aufweist, mit der Maßgabe, dass die erste Kopplungsformation zum betriebsmäßig herstellbaren und lösbaren fluidübertragenden Kopplungseingriff mit der ersten Gegenkopplungsformation und dass die zweite Kopplungsformation zum betriebsmäßig herstellbaren und lösbaren fluidübertragenden Kopplungseingriff mit der zweiten Gegenkopplungsformation des Zellkulturbehälters ausgebildet ist sowie dass der zweite Kopplungsströmungspfad zwischen dem Strömungsraum und der zweiten Kopplungsformation verläuft, um über die zweite Kopplungsformation bei mit der zweiten Gegenkopplungsformation hergestelltem Kopplungseingriff Nährmedium aus dem Strömungsraum in den Zellkulturbehälter einzuleiten oder/und aus diesem in den Strömungsraum auszuleiten. Für jede aus der ersten und der zweiten Gegenkopplungsformation bzw. aus der ersten und der zweiten Kopplungsformation gilt das oben allgemein zur Gegenkopplungsformation bzw. Kopplungsformation Gesagte entsprechend.

Dann, wenn an eine hygienisch einwandfreie Sample-Entnahme eines im Zellkulturbehälter vorhandenen Mediums gedacht ist, kann dies an einer weiter vorteilhaft ausgebildeten Zellkulturanlage dadurch realisiert sein, dass der Zellkulturbehälter eine von der ersten und der zweiten gesondert ausgebildete dritte Gegenkopplungsformation aufweist, und dass die Zellkulturanlage eine Fluid-Versorgungsschnittstelle der obigen einschlägigen Beschreibung aufweist, mit der Maßgabe, dass die dritte Kopplungsformation zum betriebsmäßig herstellbaren und lösbaren fluidübertragenden Kopplungseingriff mit der dritten Gegenkopplungsformation des Zellkulturbehälters ausgebildet ist sowie dass der dritte Kopplungsströmungspfad zwischen dem Strömungsraum und der dritten Kopplungsformation verläuft, um über die dritte Kopplungsformation bei mit der dritten Gegenkopplungsformation hergestelltem Kopplungseingriff Nährmedium aus dem Strömungsraum in den Zellkulturbehälter einzuleiten oder/und aus diesem in den Strömungsraum auszuleiten.

Zur Verhinderung eines Medienaustritts aus dem Zellkulturbehälter nach Lösen des fluidübertragenden Kopplungseingriffs zwischen der wenigstens einen Kopplungsformation der Fluid-Versorgungsschnittstelle und der wenigstens einen Gegenkopplungsformation des Zellkulturbehälters kann bei einer weitergebildeten Zellkulturanlage daran gedacht sein, dass wenigstens eine Gegenkopplungsformation, vorzugsweise jede Gegenkopplungsformation, des Zellkulturbehälters je eine Behälter-Ventilanordnung aufweist.

Dementsprechend kann an der Fluid-Versorgungsschnittstelle die Kopplungsformation frei von einer Ventilanordnung sein. Die Behälter-Ventilanordnung ist vorzugsweise ebenso aufgebaut wie die oben ausführlich diskutierten Ventilanordnungen der Anschlussformationen, auf deren Beschreibung zur Diskussion der Behälter-Ventilanordnungen ausdrücklich verwiesen wird.

Folglich ist es bevorzugt, wenn die Gegenkopplungsformation einen männlichen Stecker und die zur Verringerung der zu ihrer Bildung benötigten Bauteileanzahl bevorzugt von einer Ventilanordnung freie Kopplungsformation der Fluid-Versorgungsschnittstelle eine weibliche Buchse bildet.

Bevorzugt ist zur Erzielung hoher Hygienestandards die Behälter-Ventilanordnung bei hergestelltem Kopplungseingriff zwischen Gegenkopplungsformation und Kopplungsformation ohne eine von der Behälter-Ventilanordnung bis zur Außenseite der Gegenkopplungsformation und des Gehäuses der Fluid-Versorgungsschnittstelle kontinuierlich durchgehende signal- oder/und energieübertragende körperliche Verbindung vollständig-mit Ausnahme des die Kopplungsformation und die Gegenkopplungsformation durchsetzenden Fluidströmungspfads - von der Gegenkopplungsformation und dem Gehäuse umgeben.

Bei nicht hergestellten Kopplungseingriff kann die Behälter-Ventilanordnung wenigstens teilweise an der Gegenkopplungsformation frei liegen. Sie kann dann zwischen Zeiten eines Kopplungseingriffs mit einer Kopplungsformation durch eine Kappe abgedeckt sein, die von der Gegenkopplungsformation abziehbar und auf diese aufsteckbar sein kann. Hierzu kann eine geeignete Aufsteck- und Abziehvorrichtung vorgesehen sein, die entweder selbständig in der Zellkulturanlage vorgesehen ist oder zur gemeinsamen Bewegung mit der Fluid-Versorgungsschnittstelle ausgebildet ist. Diese Abzieh- und Aufsteckvorrichtung kann beispielsweise durch einen Mehrachsenroboter oder - bei Anbringung an der Fluid-Versorgungsschnittstelle zur gemeinsamen Bewegung mit dieser - durch einen in Abzieh- und Aufsteckrichtung beweglichen Greifer gebildet sein. Dieser kann beispielsweise durch einen Spindeltrieb oder eine doppelt wirkende Kolben-Zylinder-Anordnung zur Bewegung in Abzieh- und Aufsteckrichtung antreibbar sein.

Entsprechend der bevorzugten gleichartigen Ausbildung der Ventilanordnungen der Anschlussformationen und der wenigstens einen Behälter-Ventilanordnung kann auch letztere durch die Steueranordnung der Fluid-Versorgungsschnittstelle zwischen einer Sperrstellung und einer Durchlassstellung schaltbar sein. Somit reicht es aus, für die Ventilanordnungen der Zellkulturbehälter und für jene der Fluid-Versorgungsschnittstelle nur eine gemeinsame Steueranordnung vorzusehen. Hierzu ist Voraussetzung, dass die Behälter-Ventilanordnung in die Sperrstellung vorgespannt ist und nur dann überhaupt geschaltet werden muss, wenn ein fluidübertragender Kopplungseingriff zwischen der die Behälter-Ventilanordnung tragenden Gegenkopplungsformation und einer Kopplungsformation der Fluid-Versorgungsschnittstelle hergestellt ist. Dann können sich die wenigstens eine Behälter-Ventilanordnung und die Ventilanordnungen der Fluid-Versorgungsschnittstelle jedoch räumlich so dicht beieinander befinden, dass die Schaltung durch eine einzige gemeinsame Steueranordnung ohne weiteres realisierbar ist.

Die Möglichkeit, beim Spülen des Strömungsraums durch Reinigungsfluid von der zweiten zur dritten Anschlussformation auch die wenigstens eine Behälter-Ventilanordnung reinigen zu können, kann dadurch geschaffen werden, dass im Strömungsraum ein Strömungsweg von der Ventilanordnung der zweiten Anschlussformation zur Ventilanordnung der dritten Anschlussformation - bei hergestelltem Kopplungseingriff zwischen der wenigstens einen Kopplungsformation der Fluid-Versorgungsschnittstelle und der wenigstens einen Gegenkopplungsformation eines Zellkulturbehälters - an der wenigstens einen Behälter-Ventilanordnung, vorzugsweise an allen Behälter-Ventilanordnungen, vorbei verläuft. Hierdurch kann außerdem wenigstens ein Teil der in den Strömungsweg von der zweiten zur dritten Anschlussformation mündenden Kopplungsströmungspfade gereinigt werden.

Eine Reinigung insbesondere der Behälter-Ventilanordnungen durch Spülung des Strömungsraums kann konstruktiv dadurch erreicht oder sogar verbessert werden, dass - bei hergestelltem Kopplungseingriff zwischen der wenigstens einen Kopplungsformation der Fluid-Versorgungsschnittstelle und der wenigstens einen Gegenkopplungsformation eines Zellkulturbehälters - der Ventilkörper der wenigstens einen Behälter-Ventilanordnung, bevorzugt jeder Ventilkörper aller Behälter-Ventilanordnungen, wenigstens zum Teil, vorzugsweise wenigstens mehr als zur Hälfte, in den Strömungsweg von der Ventilanordnung der zweiten Anschlussformation zur Ventilanordnung der dritten Anschlussformation hineinragt. Die Behälter-Ventilanordnungen mit ihren Ventilkörpern ragen somit bevorzugt genauso weit in den Strömungsweg von der Ventilanordnung der zweiten Anschlussformation zur Ventilanordnung der dritten Anschlussformation wie die Ventilkörper der Ventilanordnungen der Anschlussformationen. Es gelten die im Zusammenhang mit letztgenannten Ventilanordnungen, insbesondere mit deren Reinigung, erwähnten Vorteile auch für die Behälter-Ventilanordnungen.

Zur Vereinfachung von Montage, Betrieb und Wartung kann die Zellkulturanlage derart weitergebildet sein, dass alle in der Fluid-Versorgungsschnittstelle vorgesehenen Ventilanordnungen, vorzugsweise auch alle in dem wenigstens einen Zellkulturbehälter vorgesehenen Ventilanordnungen, im Wesentlichen identisch aufgebaut sind.

Vorteilhaft für eine Reinigung des Strömungsraums verläuft der Strömungsweg von der Ventilanordnung der zweiten Anschlussformation zur Ventilanordnung der dritten Anschlussformation geradlinig, besonders bevorzugt zur Vermeidung von Zwickelstellen längs eines im Wesentlichen kreiszylindrischen Kanals. Die Relativbewegungsrichtung der Ventilkörper der Ventilanordnungen der zweiten und der dritten Anschlussformation bei deren Schaltung zwischen Sperrstellung und Durchlassstellung verläuft vorzugsweise längs des Strömungswegs von der Ventilanordnung der zweiten Anschlussformation zu jener der dritten Anschlussformation. Dadurch können die Ventilkörper dieser Ventilanordnungen beim Spülen des Strömungsraums möglichst großflächig von Reinigungsfluid umströmt und somit besonders gut und sicher gereinigt werden.

Das zuvor genannte elastomere Anlagebauteil, an welchem der Ventilkörper einer Ventilanordnung in deren Sperrstellung anliegt, kann nicht nur zur Abdichtung des Durchgangs durch die Ventilanordnung verwendet werden, sondern kann überdies auch zur Abdichtung einer Anschlussformation nach radial außen verwendet werden. Hierzu kann konstruktiv vorgesehen sein, dass in wenigstens einer Anschlussformation, vorzugsweise in einer Mehrzahl von Anschlussformationen, eine ringförmige axiale Endfläche des elastomeren Anlagebauteils als Dichtfläche den der Anschlussformation zugeordneten Fluidströmungspfad radial außen umgebend verformt an einer Gegendichtfläche anliegt. Die Gegendichtfläche kann dabei entweder am Gehäuse der Fluid-Versorgungsschnittstelle ausgebildet sein oder kann Teil der mit dem Gehäuse der Fluid-Versorgungsschnittstelle an der jeweiligen Anschlussformation fluidübertragend verbundenen Fluidleitung sein.

In analoger Weise kann auch die Behälter-Ventilanordnung an einer Gegenkopplungsformation eines Zellkulturbehälters vorgesehen sein und mit einer ringförmigen axialen Endfläche zur Abdichtung des hergestellten Kopplungseingriffs zwischen Gegenkopplungsformation und zugeordneter Kopplungsformation nach radial außen dienen. Hierzu kann vorgesehen sein, dass - bei hergestelltem Kopplungseingriff zwischen der wenigstens einen Kopplungsformation der Fluid-Versorgungsschnittstelle und der wenigstens einen Gegenkopplungsformation eines Zellkulturbehälters - eine ringförmige axiale Endfläche des elastomeren Anlagebauteils der Behälter-Ventilanordnung als Dichtfläche, den der Kopplungsformation zugeordneten Fluidströmungspfad radial außen umgebend, verformt an einer Gegendichtfläche der Fluid-Versorgungsschnittstelle anliegt.

Vorzugsweise kann das elastomere Anlagebauteil einen von einem elastomeren Material umgebenen Kanaldurchgang aufweisen, wobei bevorzugt das elastomere Anlagebauteil eine konische Ausnehmung aufweist, in der der Ventilkörper aufgenommen ist und an deren negativ-konischer Wandung der Ventilkörper in der Sperrstellung der Ventilanordnung dichtend in Anlage ist. Der Ventilkörper kann dabei selbst ein Konus oder - wegen der Symmetrie und der damit verbundenen Orientierungsinvarianz bevorzugt - eine Kugel sein.

Im Falle der am elastomeren Anlagebauteil vorgesehenen konischen Ausnehmung - welche sich zu einem freien, vom Spannbauteil wegweisenden Längsende hin aufweitet - kann die am Längsende der konischen Ausnehmung verbleibende ringförmige axiale Endfläche, wie oben dargelegt, besonders einfach zur Abdichtung an der Gegendichtfläche verwendet werden.

Ein wesentlicher Vorteil der erfindungsgemäßen Zellkulturanlage liegt darin, dass sie mehr Zellkulturbehälter als Fluid-Versorgungsschnittstellen und Medien- und Fluidreservoirs aufweisen kann, dass also aus ein- und demselben Nährmediumreservoir eine Mehrzahl von gesondert ausgebildeten Zellkulturbehältern gespeist werden kann. Dabei können beispielsweise fünf Fluid-Versorgungsschnittstellen für die gesamte Zellkulturanlage ausreichen. Die Kollisionsgefahr beim Verfahren der Fluid-Versorgungsschnittstellen zwischen den einzelnen Zellkulturbehältern kann durch Verringerung ihrer Anzahl weiter reduziert werden. Bevorzugt weist daher die Zellkulturanlage nicht mehr als drei Fluid-Versorgungsschnittstellen auf. Tatsächlich kann sogar genau eine Fluid-Versorgungsschnittstelle ausreichen, um eine Vielzahl von Zellkulturbehältern mit frischem Nährmedium zu versorgen und gebrauchtes Nährmedium aus den Zellkulturbehältern auszuleiten.

Zur Bewegung der wenigstens einen Fluid-Versorgungsschnittstelle zwischen dem an sie anzukoppelnden Zellkulturbehältern kann die Zellkulturanlage eine Bewegungsvorrichtung aufweisen. Diese ist dazu ausgebildet, die wenigstens eine Fluid-Versorgungsschnittstelle nacheinander in fluidübertragenden Kopplungseingriff mit unterschiedlichen Zellkulturbehältern zu bringen. Eine mögliche Ausgestaltung einer solchen Bewegungsvorrichtung kann eine Kreuztischanordnung mit daran vorgesehenen Bewegungsschlitten sein, welche eine zur Kreuztischebene orthogonale Bewegungsebene bietet, so dass eine an einer derartigen Bewegungsvorrichtung angeordnete Fluid-Versorgungsschnittstelle wenigstens in drei linear unabhängigen Raumrichtungen translatorisch bewegbar ist. Alternativ - und dies ist bevorzugt - kann die Bewegungsvorrichtung ein Mehrachsenroboter sein, welcher abhängig von seiner Achsanzahl eine Vielzahl von translatorischen und rotatorischen Bewegungsmöglichkeiten bietet. Weitere Bewegungsvorrichtungen, welche ein Anfahren unterschiedlicher Orte im Raum ermöglichen, sind dem Durchschnittsfachmann aus dem Stand der Technik bekannt.

Die eingangs genannte Aufgabe wird überdies auch durch einen Zellkulturbehälter für eine wie oben beschrieben ausgestaltete Zellkulturanlage gelöst. Dieser Zellkulturbehälter ist für ein Zusammenwirken mit der zuvor beschriebenen Fluid-Versorgungsschnittstelle zur Herstellung und Lösung eines Kopplungseingriffs mit einer Kopplungsformation der letztgenannten ausgebildet.

Dieser Zellkulturbehälter weist einen ein Kulturvolumen einschließenden Behälterkörper mit einer Einfüllöffnung auf, durch welche hindurch Gas, Flüssigkeit, Paste oder/und Festkörper in den Behälterkörper einfüllbar und aus diesem entnehmbar sind. Mithin kann die genannte Einfüllöffnung zusätzlich oder alternativ auch als Belüftungsöffnung dienen.

Erfindungsgemäß ist ein derartiger Zellkulturbehälter dadurch zur Herstellung und Lösung eines Kopplungseingriffs mit einer Fluid-Versorgungsschnittstelle ausgebildet, das der Zellkulturbehälter zusätzlich wenigstens eine von der Einfüll- oder/und Belüftungsöffnung gesondert ausgebildete Gegenkopplungsformation aufweist, welche zur Herstellung und Lösung eines Kopplungseingriffs mit einer entsprechenden Kopplungsformation der Fluid-Versorgungsschnittstelle ausgebildet ist, wobei ein Liefer-Fluidströmungspfad zwischen der wenigstens einen Gegenkopplungsformation und dem Kulturvolumen verläuft, um über den Liefer-Fluidströmungspfad ein Fluid in das Kulturvolumen einzuleiten oder/und aus diesem auszuleiten, wobei die wenigstens eine Gegenkopplungsformation eine Ventilanordnung aufweist. Die Ventilanordnung ist vorzugsweise durch eine Steueranordnung mit einem ein elektrisches oder/und magnetisches oder/und elektromagnetisches Feld erzeugenden Signalmittel, dessen Feld auf ein entsprechend feldsensitives Gegensignalmittel der Ventilanordnung berührungslos einwirkt, zwischen einer Sperrstellung, in der die Ventilanordnung eine Fluidströmung in dem Liefer-Fluidströmungspfad unterbricht, und einer Durchlassstellung, in der die Ventilanordnung eine Fluidströmung gestattet, schaltbar.

Wie bereits oben dargelegt wurde, kann durch die Behälter-Ventilanordnung in der wenigstens einen Gegenkopplungsformation diese zu jenen Zeiten, zu welchen kein Kopplungseingriff zwischen der Gegenkopplungsformation des Zellkulturbehälters und der Kopplungsformation der Fluid-Versorgungsschnittstelle besteht, durch Vorspannung der Behälter-Ventilanordnung in die Sperrstellung dicht verschlossen gehalten werden, so dass ein Eintreten von Fluid durch die Gegenkopplungsformation in das Zellkulturvolumen und ein Austreten von Fluid aus diesem sicher verhindert werden kann. Durch die Schaltbarkeit der Behälter-Ventilanordnung kann eine Unterbrechung des Liefer-Fluidströmungspfads gezielt aufgehoben und wieder herbeigeführt werden, etwa dann, wenn der oben genannte Kopplungseingriff mit der Fluid-Versorgungsschnittstelle hergestellt ist.

Zur Weiterbildung der Behälter-Ventilanordnung wird ausdrücklich auf die obigen Ausführungen zu der in dieser Anmeldung bevorzugten Ventilanordnung verwiesen, die auch auf Behälter-Ventilanordnung anwendbar sind.

Zur Vermeidung von Kollisionen oder auch um zu ermöglichen, dass an der Einfüll- oder/und Belüftungsöffnung selbst dann gearbeitet werden kann, wenn die wenigstens eine Gegenkopplungsformation in Kopplungseingriff mit der Fluid-Versorgungsschnittstelle ist, kann vorteilhafterweise vorgesehen sein, dass die Einfüll- oder/und Belüftungsöffnung und die wenigstens eine gesondert ausgebildete Gegenkopplungsformation an entgegengesetzten Enden des Zellkulturbehälters vorgesehen sind. Somit wird also die Möglichkeit geschaffen, an der Einfüll- oder/und Belüftungsöffnung einerseits und an der wenigstens einen Gegenkopplungsformation andererseits gleichzeitig auf das Kulturvolumen ein- und desselben Zellkulturbehälters zuzugreifen, ohne dass der eine Zugriff den jeweils anderen übergebühr behindern würde.

Der erhebliche Vorteil der vorliegenden Erfindung liegt darin, dass besonders vorteilhaft kostengünstige Einweg- oder Wegwerf-Zellkulturbehälter verwendet werden können. Bevorzugt ist daher der Zellkulturbehälter ein passiver Behälter, welcher abgesehen von der wenigstens einen Behälter-Ventilanordnung der wenigstens einen Gegenkopplungsformation ohne im Behälter eingebaute, also fest mit dem Behälter verbundene, durch Energiezufuhr betreibbare Funktionseinheiten ausgeführt ist. Mögliche derartige Funktionseinheiten sind etwa eine Heizeinrichtung oder eine Rühreinrichtung. Sofern für die Pflege von Zellkulturen im Zellkulturbehälter eine Temperierung des Kulturvolumens notwendig ist, kann dies kostengünstig in Brutschränken oder Heizschränken erfolgen, in die der Zellkulturbehälter, vorzugsweise zusammen mit weiteren Zellkulturbehältern, eingebracht werden kann.

Bevorzugt ist der Zellkulturbehälter stapelbar, weshalb er gemäß einer bevorzugten Weiterbildung der vorliegenden Erfindung zwei im Wesentlichen parallele Stirnwände und diese verbindende, um einen Stirnwandrand umlaufende Mantelwandabschnitte aufweist. Stirnwände und Mantelwandabschnitte begrenzen dabei gemeinsam das Kulturvolumen. Weiter bevorzugt sind die Stirnwände die flächengrößten Wandabschnitte des Zellkulturbehälters, wobei zur Erhöhung der Stapelbarkeit die Mantelwandabschnitte mit den Stirnwänden vorteilhafterweise einen rechten Winkel einschließen können.

Damit eine Mehrzahl von Zellkulturbehältern stabil durch Anlage der vorzugsweise flächengrößten Stirnwände unmittelbar benachbarter Zellkulturbehälter aneinander gestapelt werden kann, ist bevorzugt die wenigstens eine Gegenkopplungsformation, besonders bevorzugt auch die Einfüll- oder/und Belüftungsöffnung in einem Mantelwandabschnitt vorgesehen. So können die Stirnwände frei von Funktionselementen sein und eine möglichst große und stabile Stapelfläche bieten.

Die oben im Zusammenhang mit der Fluid-Versorgungsschnittstelle oder/und mit der Zellkulturanlage für Zellkulturbehälter angesprochenen Weiterbildungen, etwa das Vorsehen von zwei oder sogar drei Gegenkopplungsformationen, von welchen bevorzugt jede mit einer Behälter-Ventilanordnung versehen ist, gilt auch für den Zellkulturbehälter als Anmeldungsgegenstand für sich alleine genommen.

Vorzugsweise ist die wenigstens eine Gegenkopplungsformation als männlicher Kopplungsstutzen ausgebildet, welcher im Kopplungseingriff von einer Kopplungsbuchse der Kopplungsformation der Fluid-Versorgungsschnittstelle radial außen gegeben ist.

Bevorzugt ist das Kulturvolumen (Gesamtvolumen) des Zellkulturbehälters nicht größer als 2 Liter, besonders bevorzugt nicht größer als 1,3 Liter. Somit kann im Falle einzelner verunreinigter Zellkulturen der Gesamtschaden erheblich beschränkt werden.

Die vorliegende Erfindung wird nachfolgend anhand der beiliegenden Zeichnungen näher erläutert. Es stellt dar:
- Fig. 1: eine Längsschnittansicht durch eine erfindungsgemäße Ausführungsform eines Zellkulturbehälters, an welchem beispielhaft zwei Gegenkopplungsformationen vorgesehen sind.
- Fig. 2: eine Längsschnittansicht durch eine beispielhafte erfindungsgemäße Ausführungsform einer Fluid-Versorgungsschnittstelle, welche sich in einem fluidübertragenden Kopplungseingriff mit dem Zellkulturbehälter von Fig. 1 befindet.
- Fig. 3: eine Längsschnittansicht der Fluid-Versorgungsschnittstelle und des Zellkulturbehälters in der Schnittebene III-III von Fig. 2 mit einer Steueranordnung, die eine zwischen Signalmitteln und der Fluid-Versorgungsschnittstelle vorgesehene Vermittlungsanordnung aufweist.
- Fig. 4: eine Detailansicht einer Kopplungsformation der Fluid-Versorgungsschnittstelle von Fig. 2 und einer Gegenkopplungsformation des Zellkulturbehälters der Fig. 1 bis 3 in einem Zustand, in welchem kein Kopplungseingriff zwischen diesen hergestellt ist.
- Fig. 5: die Bauteile von Fig. 4 bei zwischen ihnen hergestelltem fluidübertragenden Kopplungseingriff.
- Fig. 6-14: Darstellungen unterschiedlicher Ventilschaltzustände (Ventilstellungskonfigurationen) einer erfindungsgemäßen Zellkulturanlage mit einer Fluid-Versorgungsschnittstelle mit einer ersten bis vierten Anschlussformation und mit einem Zellkulturbehälter gemäß den Fig. 1 bis 3.
- Fig. 15-18: unterschiedliche Ventilschaltzustände (Ventilstellungskonfigurationen) einer zweiten Ausführungsform einer erfindungsgemäßen Zellkulturanlage, bei welcher Zellkulturbehälter mit jeweils drei Gegenkopplungsformationen verwendet werden, und
- Fig. 19-21: Darstellungen unterschiedlicher Ventilschaltzustände (Ventilstellungskonfigurationen) einer dritten Ausführungsform einer erfindungsgemäßen Zellkulturanlage, welche Zellkulturbehälter mit jeweils nur einer einzigen Gegenkopplungsformation verwendet.

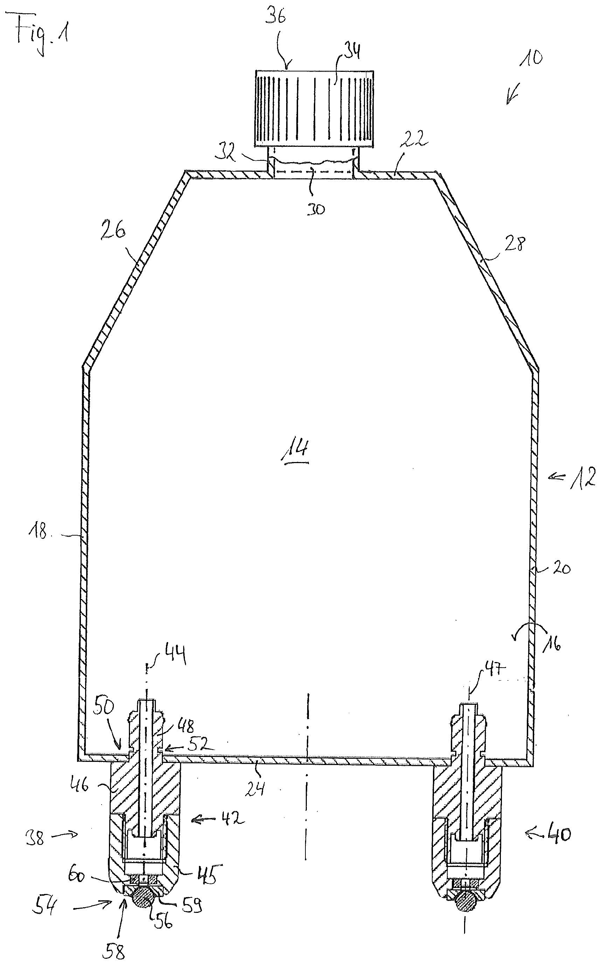

In Fig. 1 ist eine beispielhafte erfindungsgemäße Ausführungsform eines Zellkulturbehälters allgemein mit 10 bezeichnet. Ein solcher Zellkulturbehälter kann in einer erfindungsgemäßen Zellkulturanlage zur Anwendung kommen.

Der Zellkulturbehälter 10 weist einen Behälterkörper 12 auf, welcher ein Kulturvolumen 14 umschließt.

Bevorzugt weist der Behälterkörper 12 des Zellkulturbehälters 10 zwei im Wesentlichen parallele Stirnwände auf, von welchen in Fig. 1 aufgrund der zu den Strinwänden parallelen Schnittebene dieser Figur nur die hinter der Schnittebene gelegene Stirnwand 16 dargestellt ist. Zwischen den beiden Stirnwänden 16 sind Mantelwandabschnitte vorgesehen, die von der Schnittebene der Fig. 1 geschnitten sind.

Von den Mantelwandabschnitten sind vorzugsweise die beiden seitlichen Mantelwandabschnitte 18 und 20 zueinander parallel und sind die vorderen und hinteren Mantelwandabschnitte 22 und 24 zueinander parallel. Zur erleichterten Stapelung von erfindungsgemäßen Zellkulturbehältern 10 schließen vorzugsweise benachbarte der genannten Mantelwandabschnitte 20, 22 und 24 zwischen sich einen rechten Winkel ein. Weiterhin ist es bevorzugt, dass jeder der Mantelwandabschnitte 18 bis 24 mit den parallelen Stirnwänden einen rechten Winkel einschließt.

Es soll jedoch nicht ausgeschlossen sein, dass die Mantelwandabschnitte auch Mantelwandabschnitte 26 und 28 aufweisen, welche zwar bevorzugt mit den Stirnwänden jeweils einen rechten Winkel einschließen, jedoch nicht mit ihren benachbarten Mantelwandabschnitten 18 und 22 bzw. 20 und 22. Beispielsweise können diese Mantelwandabschnitte 26 und 28 zum vorderen Mantelwandabschnitt 22 hin verlaufend aufeinander zu geneigt sein, so dass der Behälterkörper 10 sich zum vorderen Wandabschnitt 22 hin verjüngt.

Der Behälterkörper 10 weist eine Zugangsöffnung 30 auf, welche als Einfüll- oder/und Belüftungsöffnung zum Befüllen oder Entleeren des Kulturvolumens 14 oder zu dessen Belüftung verwendet werden kann. Bevorzugt ist die Zugangsöffnung 30 an einem Zugangshals 32 ausgebildet, welcher durch eine Verschlusskappe 34 wahlweise verschlossen oder geöffnet werden kann. Die Verschlusskappe 34 kann zum besseren Halt am Zugangshals 32 als Schraubkappe mit einem Innengewinde ausgebildet sein, welches mit einem bevorzugt an der Außenseite des Zugangshalses 32 vorgesehenen Außengewinde in an sich bekannter Weise verschraubbar sein kann. Falls eine Belüftung des Kulturvolumens 14, also ein Gasaustausch zwischen dem Kulturvolumen 14 und der Außenumgebung des Zellkulturbehälters 10 auch bei ansonsten verschlossener Zugangsöffnung 30 erwünscht ist, kann die Verschlusskappe 34 in ihrer im Beispiel orthogonal zur Zeichenebene der Fig. 1 orientierten Stirnfläche 36 eine oder mehrere Durchbrechungsöffnungen aufweisen, durch welche zwar Gas in das Kulturvolumen 14 hinein und aus diesem hinaus strömen kann, welche jedoch in ihren Abmessungen so gewählt sind, dass übliche Laborgeräte nicht durch sie hindurchpassen.

Bevorzugt ist der Zellkulturbehälter 10 ein kostengünstiger Einweg-Wegwerf-Zellkulturbehälter ohne daran ausgebildete elektrisch betreibbare Heizvorrichtung und ohne daran vorgesehene elektrisch betreibbare Rührvorrichtung. Zur besseren Überwachung und Beobachtung des Zellkulturvorgangs ist bevorzugt der Behälterkörper 12 wenigstens abschnittsweise, vorzugsweise vollständig, aus optisch durchlässigem Kunststoff gebildet, etwa aus Polymetylmetacrylat (PMMA) oder einem anderen geeigneten transparenten Kunststoff, je nach gewünschter chemischer Beständigkeit abhängig von den erwarteten chemischen oder biochemischen Materialien, die sich betriebsgemäß im Inneren des Kulturvolumens 14 befinden werden.

Der Zellkulturbehälter 10 weist weiter eine oder mehr Gegenkopplungsformationen 38 auf, in dem in Fig. 1 dargestellten Beispiel genau zwei Gegenkopplungsformationen 38 und 40.

Der Zellkulturbehälter 10 kann an und für sich beliebig viele Gegenkopplungsformationen aufweisen, wobei die Anzahl von drei Gegenkopplungsformationen bereits separate Liefer-Fluidströmungspfade zur Befüllung, zur Entleerung und zur Stichprobenentnahme ("sampling") des Kuluturvolumens 14 bzw. aus dem Kulturvolumen 14 bereitstellt. Sofern an der in Fig. 1 gezeigten Ausführungsform eines Zellkulturbehälters 10 eine dritte Gegenkopplungsformation gewünscht sein sollte, kann diese mittig zwischen den dargestellten Gegenkopplungsformationen 38 und 40 angeordnet sein, wie durch die strichpunktierte Andeutung des Liefer-Fluidströmungspfads dieser dritten Gegenkopplungsformation gezeigt ist.

Falls nur eine einzige Gegenkopplungsformation gewünscht ist, kann sie an jeder der drei in Fig. 1 gezeigten Stellen, aber auch an einer beliebigen anderen Stelle in einem anderen Mantelwandabschnitt oder sogar in einer Stirnwand vorgesehen sein.

Die Stapelbarkeit der Zellkulturbehälter 10 durch Übereinanderlegen derselben an ihren Stirnwänden wird jedoch dadurch in vorteilhafter Weise erleichtert, dass sowohl die Zugangsöffnung 30, wie auch alle Gegenkopplungsformationen 38 und 40 an Mantelwandabschnitten vorgesehen sind. Die Zulänglichkeit der Gegenkopplungsformationen 38 und 40 einerseits sowie der Zugangsöffnung 30 andererseits unabhängig voneinander kann noch dadurch verbessert werden, dass, wie in Fig. 1 gezeigt ist, Gegenkopplungsformationen 38 und 40 und die Zugangsöffnung 30 an unterschiedlichen, besonders bevorzugt an einander entgegengesetzten Mantelwandabschnitten 22 und 24 ausgebildet sind. In diesem Falle können selbst an übereinander und nebeneinander gestapelten Zellkulturbehältern 10 auf der einen Seite der Stapelwand Zugangsöffnungen 30 durch einen Bediener erreichbar sein und können auf der gegenüberliegenden Seite der Stapelwand die Gegenkopplungsformationen 38 und 40 durch eine weiter unten beschriebene Fluid-Versorgungsschnittstelle zugänglich sein.

Die Gegenkopplungsformationen 38 und 40 sind zur Erleichterung von Fertigung und Montage vorzugsweise identisch ausgebildet, so dass nachfolgend lediglich eine Gegenkopplungsformation stellvertretend für alle am Zellkulturbehälter 10 vorgesehenen Gegenkopplungsformationen beschrieben werden wird.

Die Gegenkopplungsformation 38 weist ein Gegenkopplungsformationsgehäuse 42 auf, welches einstückig oder - wie in Fig. 1 dargestellt - mehrteilig ausgebildet sein kann. Das Gehäuse 42 definiert einen ersten Liefer-Fluidströmungspfad 44, welcher sich zwischen der Außenumgebung des Zellkulturbehälters 10 und dessen Kulturvolumen durch die erste Gegenkopplungsformation 38 hindurch erstreckt.

Das Gegenkopplungsformationsgehäuse 42 kann ein dem Zellkulturbehälter 10 ferner liegendes Gehäuseeingriffsbauteil 45 aufweisen, welches fest mit einem dem Zellkulturbehälter 10 näher liegenden Gehäuseträgerbauteil 46 verbunden sein kann. Diese Verbindung kann beispielsweise eine Schraubverbindung sein, insbesondere indem das Gehäuseeingriffsbauteil 45 auf einen Gewindeschaft des Gehäuseträgerbauteils 46 aufgeschraubt ist.

Vorzugsweise dient das Gehäuseträgerbauteil 46 zur Befestigung der ersten Gegenkopplungsformation 38 am Zellkulturbehälter 10, etwa indem ein Befestigungsschaft 48 des Gehäuseträgerbauteils 46 eine zugeordnete Öffnung 50 in einer Wandung des Zellkulturbehälters 10 durchsetzt. Der Befestigungsschaft 48 kann mit an sich bekannten Befestigungsmitteln in der betreffenden Wandung des Zellkulturbehälters 10 festgelegt sein, etwa durch in eine umlaufende Nut 52 eingesetzte mechanische Befestigungs- und Dichtungsmittel, welche in Fig. 1 nicht näher dargestellt sind, welche jedoch dem Durchschnittsfachmann ohne weiteres geläufig sind. Zusätzlich oder alternativ kann das Gegenkopplungsformationsgehäuse 42 mit dem Behälterkörper 12 verklebt oder/und verschweißt sein.

Bevorzugt sind die Gegenkopplungsformationen 38 und 40 an einem Mantelwandungsabschnitt, insbesondere an einem die Zugangsöffnung 30 aufweisenden Mantelabschnitt 22 diametral gegenüberliegenden Mantelwandabschnitt 24 vorgesehen.

Die erste Gegenkopplungsformation 38 kann weiter eine Behälter-Ventilanordnung 54 aufweisen, welche zwischen einer Sperrstellung und einer Durchlassstellung schaltbar ist. Die Behälter-Ventilanordnung 54 der ersten Gegenkopplungsformation 38 ist in Fig. 1 in ihrer Sperrstellung dargestellt, in welcher sie den Liefer-Fluidströmungspfad 44 unterbricht. In ihrer in Fig. 1 nicht gezeigten Durchlassstellung gestattet die Behälter-Ventilanordnung 54 eine Fluidströmung längs des Liefer-Fluidströmungspfads.

Wenngleich die Behälter-Ventilanordnung 54 mit Ausnahme des Liefer-Fluidströmungspfads 44 vollständig von dem Gehäuse 42 der ersten Gegenkopplungsformation umgeben sein kann, ist sie bevorzugt an dem eingriffsseitigen Längsendbereich der ersten Gegenkopplungsformation 38 vorgesehen. Dies erleichtert ihre Reinigung bei angekoppelter Fluid-Versorgungsschnittstelle, die weiter unten beschrieben werden wird.

Die Behälter-Ventilanordnung 54 weist einen Ventilkörper 56 auf, welcher in der in Fig. 1 gezeigten Sperrstellung auf einem Ventilsitz 58 mit negativ-konischer Anlagefläche anliegt. Der Ventilsitz 58 weist aus Gründen verbesserter Dichtigkeit bevorzugt ein Anlagebauteil aus einem Material auf, welches sich bereits bei der in der Behälter-Ventilanordnung 54 in die Sperrstellung wirkenden Vorspannkraft geringfügig verformt. Als Material kommt beispielsweise ein elastomeres Kunststoffmaterial, wie beispielsweise Silikon, Gummi oder ein fluiddichter geschlossenzelliger Kunststoff, etwa PU-Schaum oder dergleichen, in Frage.

Der Ventilkörper 56 ist aus Symmetriegründen bevorzugt als Kugel ausgebildet, so dass es für seine Funktion auf seine Orientierung relativ zum Ventilsitz 58 nicht ankommt.

Auf der vom Ventilkörper 56 abgewandten Seite des Anlagebauteils 59 weist der Ventilsitz vorzugsweise ein Spannbauteil 60 auf, welches im vorliegenden Fall als Permanentmagnet ausgebildet ist. Bevorzugt ist das permanentmagnetische Spannbauteil 60 ringförmig ausgebildet und ist wie das Anlagebauteil 59 vom zugeordneten Liefer-Fluidströmungspfad 44 durchsetzt.

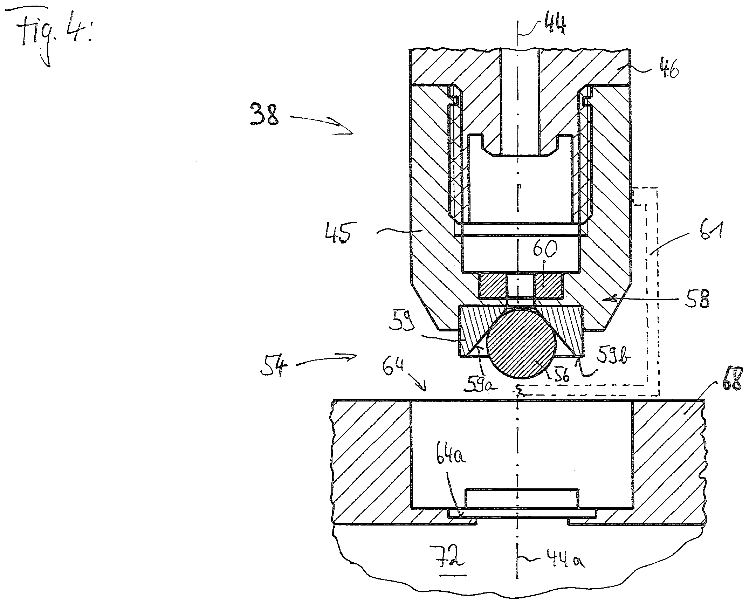

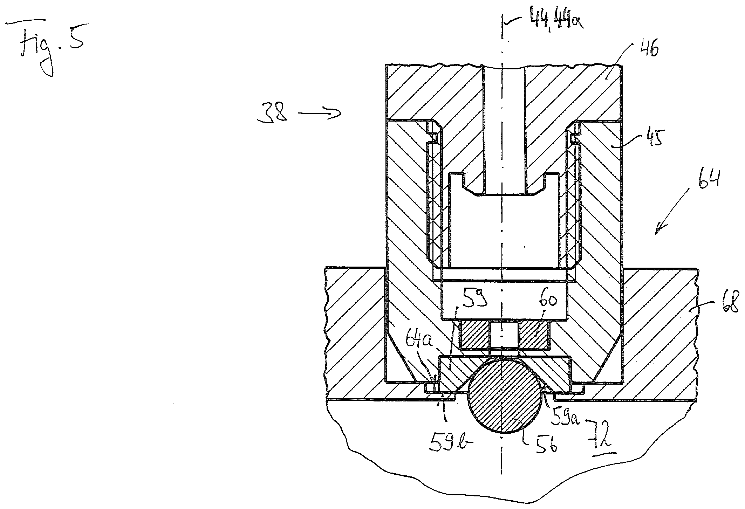

Das Anlagebauteil 59 dient, wie weiter unten im Zusammenhang mit den Fig. 4 und 5 beschrieben werden wird, nicht nur zur dichtenden Anlage des Ventilkörpers 56 daran, sondern dient auch zur dichtenden Anlage an eine Gegendichtfläche der Fluid-Versorgungsschnittstelle, welche zur Herstellung eines fluidübertragenden Kopplungseingriffs mit den am Zellkulturbehälter 10 vorgesehenen Gegenkopplungsformationen 38 und 40 ausgebildet ist.

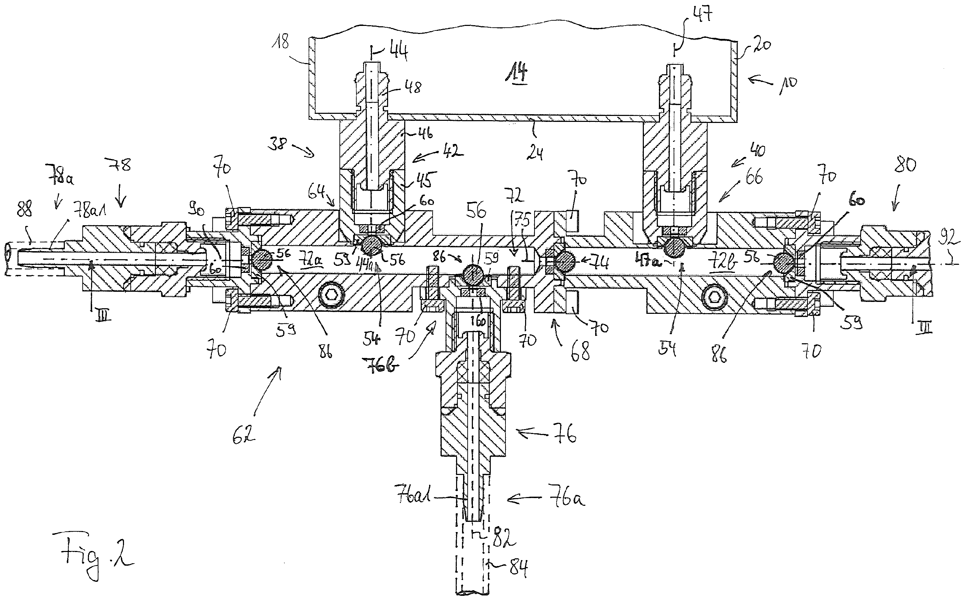

In Fig. 2 ist das gegenkopplungsformationsseitige Längsende des in Fig. 1 gezeigten Zellkulturbehälters 10 über seine Gegenkopplungsformationen 38 und 40 in fluidübertragendem Kopplungseingriff mit einer Fluid-Versorgungsschnittstelle 62 dargestellt.

Die Fluid-Versorgungsschnittstelle 62 hat in dem in Fig. 2 gezeigten Beispiel eine erste Kopplungsformation 64, welche mit der ersten Gegenkopplungsformation 38 in Kopplungseingriff ist und hat eine zweite Kopplungsformation 66, welche mit der zweiten Gegenkopplungsformation 40 in fluidübertragendem Kopplungseingriff ist. Die erste und die zweite Kopplungsformation 64 bzw. 66 sind bevorzugt als Buchsen in dem Gehäuse 68 der Fluid-Versorgungsschnittstelle 62 ausgebildet.

Dann, wenn an der Kopplungsstelle von Kopplungsformation und Gegenkopplungsformation nur eine zellkulturbehälterseitige Behälter-Ventilanordnung 54 vorgesehen ist, weist das Gehäuse 68 der Fluidversorgungsschnittstelle 62 genauso viele Kopplungsformationen auf, wie die damit zu koppelnden Zellkulturbehälter 10 Gegenkopplungsformationen aufweisen. Dementsprechend kann auch die Fluidversorgungsschnittstelle 62 nur eine oder auch drei oder mehr Kopplungsformationen aufweisen.

Das Gehäuse 68 der Fluid-Versorgungsschnittstelle 62 ist vorteilhafterweise mehrteilig aufgebaut. Dies muss jedoch nicht so sein. Im Falle eines mehrteiligen Gehäuses ist es bevorzugt, wenn Trennflächen zwischen den einzelnen Gehäuseteilen orthogonal zu einem am jeweiligen Gehäuseteil ausgebildeten Fluidströmungspfad orientiert und von dem Fluidströmungspfad durchsetzt sind. Die einzelnen Gehäuseteile sind im dargestellten Beispiel durch Schrauben 70 miteinander montiert. Alternativ oder zusätzlich zur Verschraubung können einzelne oder alle miteinander verbundenen Gehäuseteile miteinander verschweißt oder/und verklebt sein.

Das Gehäuse 68 definiert zunächst einen Strömungsraum 72, welcher im dargestellten Beispiel durch eine Trenn-Ventilanordnung 74 in zwei zu beiden Seiten der Trenn-Ventilanordnung 74 gelegenen Teil-Strömungsräumen 72a und 72b geteilt ist. Ein zwischen den beiden Kopplungsformationen 64 und 66 verlaufender Verbindungsströmungspfad 75 erstreckt sich im dargestellten Beispiel durch die Trenn-Ventilanordnung 74 hindurch.

Vorteilhafterweise sind beide Teil-Strömungsräume 72a und 72b bis auf Anfangs-, End- und Zwischenmündungen kreiszylindrisch ausgestaltet, was ihre weiter unten erläuterte Reinigung im CIP-Verfahren oder im SIP-Verfahren erheblich erleichtert.

Die Kopplungsformationen 64 und 66 sind in dem in Fig. 2 gezeigten Beispiel mit unterschiedlichen Durchmessern ausgebildet dargestellt, um darzulegen, dass zwar bei hergestelltem Kopplungseingriff zwischen Kopplungsformation und Gegenkopplungsformation vorteilhafterweise ein Abschnitt einer Formation aus Kopplungsformation und Gegenkopplungsformation einen Axialabschnitt der jeweils anderen Formation radial außen umgibt, die beiden Formationen im Bereich dieses Umgebenseins jedoch nicht notwendigerweise aneinander anliegen müssen. Die im Vergleich zum Außendurchmesser der zweiten Gegenkopplungsformation 40 größere lichte Weite der zweiten Kopplungsformation 66 erleichtert das Herstellen des Kopplungseingriffs zwischen der Kopplungsformation 66 und der Gegenkopplungsformation 40 erheblich, ohne dass eine Dichtigkeitseinbuße zu befürchten ist. Wichtig ist dann, dass die Fluid-Versorgungsschnittstelle 62 an einer entsprechenden Bewegungsvorrichtung ausreichend exakt zur Herstellung eines Kopplungseingriffs geführt ist.

Die in Fig. 2 beispielhaft gezeigte Ausführungsform der Fluid-Versorgungsschnittstelle 62 weist eine erste Anschlussformation 76, eine zweite Anschlussformation 78 und eine dritte Anschlussformation 80 auf. Die erste Anschlussformation 76 definiert einen ersten Fluidströmungspfad 82, welcher zwischen dem Strömungsraum 72, im vorliegenden Beispiel genauer dem Teilströmungsraum 72a, und der ersten Anschlussformation 76 und darüber hinaus in der ersten Anschlussformation 76 verläuft.

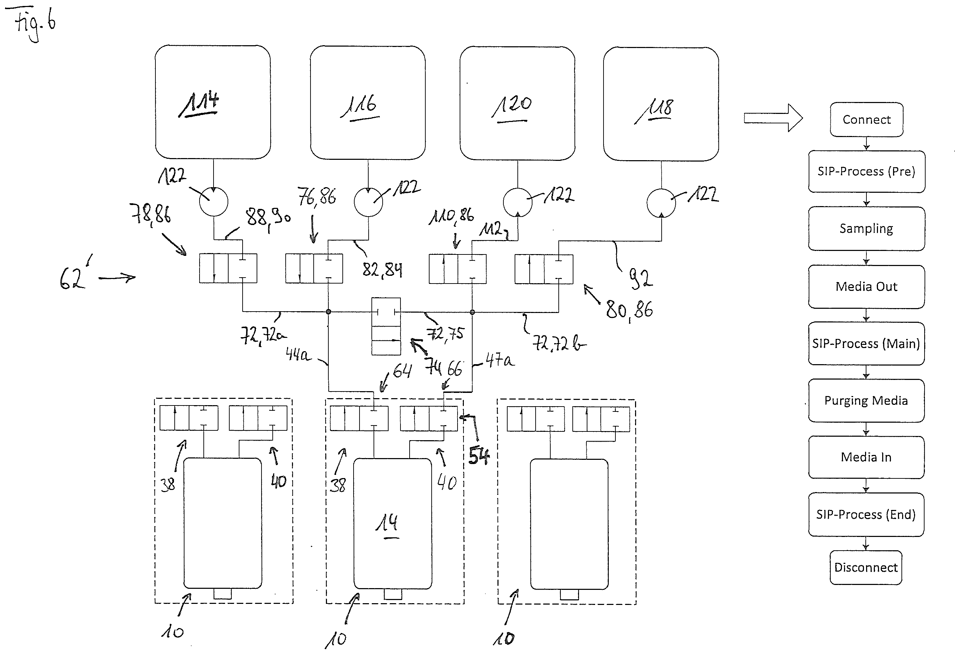

An seinem vom Strömungsraum 72 fern liegenden Längsende 76a ist die Anschlussformation 76, etwa durch einen Aufsteckstutzen 76a1, zur Verbindung mit einer Fluidleitung 84 ausgebildet, welche in dem in Fig. 2 gezeigten Beispiel bevorzugt zu einem in Fig. 2 nicht dargestellten Nährmediumreservoir führt (siehe Fig. 6 ff.). Die Fluidleitung 84 ist bevorzugt eine flexible Fluidleitung, etwa aus einem Elastomerschlauch, welche auf den Aufsteckstutzen 76a1 aufgesteckt und dort in an sich bekannter Weise mit Sicherungsmitteln, etwa einer Schlauchschelle, vor einem unerwünschten Abziehen sein kann.

Durch die Fluidleitung 84 kann also längs des ersten Fluidströmungspfads 82 Nährmedium in den Strömungsraum 72 eingeleitet und von diesem ausgehend weiter verteilt werden. Beispielsweise kann über die erste Kopplungsformation 64 und die erste Gegenkopplungsformation 38 frisches Nährmedium längs des Liefer-Strömungspfads 44 in das Kulturvolumen 14 des Zellkulturbehälters 10 eingeleitet werden.

Zwar kann die Fluidleitung 84 auch als starre Rohrleitung ausgebildet sein, jedoch ist dies weniger bevorzugt, da dann mit der Fluid-Versorgungsschnittstelle 62 auch das Nährmediumreservoir mitbewegt werden müsste.

In dem in Fig. 2 dargestellten angekoppelten Zustand, ist derjenige Teil des Liefer-Fluidströmungspfads 44, welcher im Bereich der ersten Kopplungsformation 64 bis in den Strömungsraum 72 hinein verläuft, mit einem Kopplungsströmungspfad 44a identisch, welcher zwischen dem Strömungsraum 72 und der ersten Kopplungsformation 64 verläuft. Beim herstellten fluidübertragenden Kopplungseingriff zwischen der ersten Gegenkopplungsformation 38 und der Kopplungsformation 64 geht also vom Strömungsraum 72 ausgehend der erste Kopplungsströmungspfad 44a in den Liefer-Strömungspfad 44 über. Diese Strömungspfade sind dann kollinear. Gleiches gilt für den zweiten Liefer-Fluidströmungspfad 47 der zweiten Gegenkopplungsformation 40 und dem zweiten Kopplungsströmungspfad 47a der zweiten Kopplungsformation 66.

Die erste Anschlussformation 76 kann, wie in Fig. 2 dargestellt ist, mehrteilig ausgebildet sein. Eine mehrteilige Ausbildung unterstützt dabei die Montage und spätere Wartung der Fluid-Versorgungsschnittstelle 62.

An dem dem Strömungsraum 72 näher liegenden Längsende 76b der ersten Anschlussformation 76 weist die erste Anschlussformation 76 eine Ventilanordnung 86 auf. Die Ventilanordnung 86 ist identisch zur Behälter-Ventilanordnung 54 der ersten und der zweiten Gegenkopplungsformation 38 bzw. 40 aufgebaut, so dass gleiche und funktionsgleiche Bauteile und Bauteilabschnitte wie an der Behälter-Ventilanordnung 54 an der Ventilanordnung 86 mit gleichen Bezugszeichen versehen sind. Ansonsten wird zur Beschreibung der Ventilanordnung 86 ausdrücklich auf die in der vorliegenden Anmeldung gegebene Beschreibung der Behälter-Ventilanordnung 54 verwiesen, die auch für die Ventilanordnung 86 nicht nur der ersten Anschlussformation 76, sondern auch der weiteren Anschlussformationen 78 und 80 gilt.

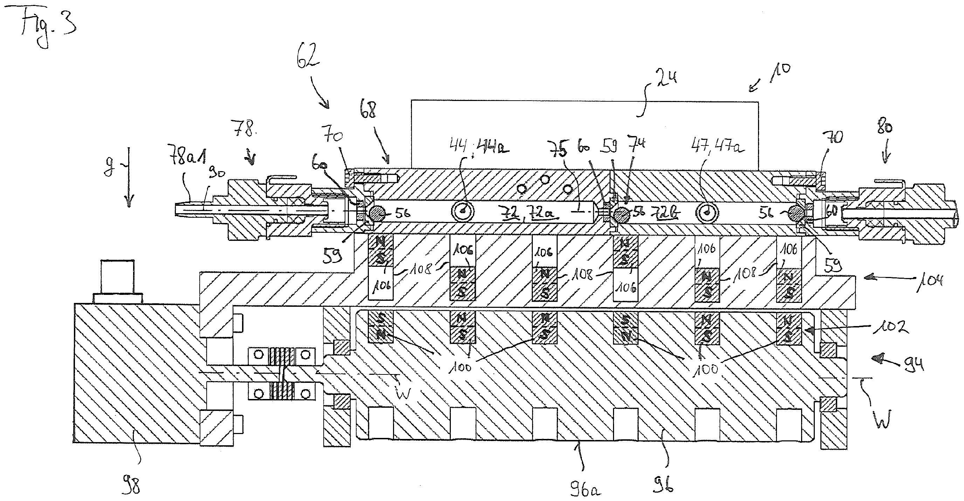

Durch die in Fig. 2 dargestellte bevorzugte dauerhafte Befestigung der Anschlussformation 76 an den den Strömungsraum 72 unmittelbar definierenden Gehäuseteil der Fluid-Versorgungsschnittstelle 62 mittels der Schrauben 70 - die im Übrigen entgegen der Darstellung von Fig. 2 nicht in den Strömungsraum 72 hineinragen, sondern vor der Zeichenebene von Fig. 2 liegen, wie der Fig. 3 zu entnehmen ist - ist die Ventilanordnung 86 der ersten Anschlussformation 76 mit Ausnahme des ersten Fluidströmungspfads 82 vollständig von dem Gehäuse 68 umgeben. Der Ventilkörper 56 ist bevorzugt eine Kugel aus ferromagnetischem Material, die durch das permanentmagnetische Spannbauteil 60 in ihre Sperrstellung gegen das elastisch verformbare Anlagebauteil 59 des Ventilsitzes 58 vorgespannt ist.

Die zweite und die dritte Anschlussformation 78 bzw. 80 sind jeweils identisch zur ersten Anschlussformation 76 ausgebildet, so dass zu deren Beschreibung ausdrücklich auf die Beschreibung der ersten Anschlussformation verwiesen wird.

Bevorzugt ist ein am strömungsraumfernen Längsende 78a vorgesehener Aufsteckstutzen 78a1 mit einer zweiten Fluidleitung 88 verbunden, welche andernends mit einem in Fig. 2 nicht dargestellten Reinigungsfluidreservoir verbunden ist. Ein zwischen dem Strömungsraum 72, in Fig. 2 genauer zwischen dem Teilströmungsraum 72a, und der zweiten Anschlussformation 78 und darüber hinaus verlaufender zweiter Fluidströmungspfad 90 gestattet in dem in Fig. 2 dargestellten Ausführungsbeispiel also bevorzugt ein Einleiten von Reinigungsfluid aus dem nicht dargestellten Reinigungsfluidreservoir in den Strömungsraum 72, genauer in den Teil-Strömungsraum 72a. Damit eine möglichst große Länge des Strömungsraums 72 von dem Reinigungsfluid erreichbar ist, ist vorzugsweise die zweite Anschlussformation 78 an einem axialen Längsende des Strömungsraums 72 vorgesehen.

Die dritte Anschlussformation 80 ist bevorzugt mit einem in Fig. 2 nicht dargestellten Abfluss verbunden. Dies kann in der mit Bezug auf die erste und die zweite Anschlussformation 76 bzw. 78 bereits beschriebenen Weise durch eine entsprechend an die dritte Anschlussformation 80 anmontierte Fluidleitung (in Fig. 2 nicht dargestellt) geschehen.

Zwischen dem Fluidströmungsraum 72, genauer in Fig. 2 zwischen dem Teil-Strömungsraum 72b, und der dritten Anschlussformation 80 sowie darüber hinaus verläuft ein dritter Fluidströmungspfad 92, durch welchen Fluid im Fluidströmungsraum 72 aus diesem zu einem Abfluss ausleitbar ist.

Um ein durch den zweiten Fluidströmungspfad 90 in den Strömungsraum 72 eingeleitetes Reinigungsfluid über eine möglichst lange Strecke des Fluidströmungsraums 72 zu leiten und damit einen möglichst großen Abschnitt des Fluidströmungsraums 72 reinigen zu können, ist bevorzugt die dritte Anschlussformation 80 an dem der Montagestelle der zweiten Anschlussformation 78 gegenüberliegenden Längsende des Strömungsraums 72 angeordnet. Damit kann ein durch die zweite Anschlussformation 78 in den Strömungsraum 72 eingeleitetes Reinigungsfluid erst nach Durchgang durch den im Wesentlichen gesamten Strömungsraum 72 aus diesem über die dritte Anschlussformation 80 ausgeleitet werden. Somit kann durch die zweite und die dritte Anschlussformation 78 bzw. 80 der Strömungsraum 72 im Wesentlichen über seine gesamte Länge mit Reinigungsfluid gespült werden.

Damit bei einem derartigen Reinigungsvorgang die gesamte Fluid-Versorgungsschnittstelle 62 samt der mit dieser im Kopplungseingriff befindlichen Gegenkopplungsformationen 38 und 40 möglichst effizient gereinigt werden kann, bevor dem Kulturvolumen 14 des jeweils angekoppelten Zellkulturbehälters 10 frisches Nährmedium zugeführt oder darin vorhandenes Nährmedium aus diesem abgelassen wird, befinden sich alle Mündungsstellen einer weiteren Anschlussformation oder einer Kopplungsformation in den Strömungsraum 72 bevorzugt zwischen der zweiten Anschlussformation 78 und der dritten Anschlussformation 80, so dass sie längs des oben beschriebenen Spülpfads gelegen sind und von einem von der zweiten Anschlussformation 78 zur dritten Anschlussformation 80 strömenden Reinigungsfluid erreicht werden.

Um die Ventilkörper 56 der Ventilanordnungen 86 und der Behälter-Ventilanordnungen 54 sowie der Trenn-Ventilanordnung 74 möglichst effizient reinigen zu können, ragen diese entweder in den Strömungsraum 72 ein oder befinden sich vollständig in diesem. Bevorzugt ragen die Ventilkörper 56 wenigstens mit mehr als der Hälfte ihres Körpervolumens in den Strömungsraum 72 ein.

In Fig. 3 ist die in Fig. 2 gezeigte Anordnung in der durch die Pfeile III-III bezeichneten, zur Zeichenebene von Fig. 2 orthogonalen Schnittebene dargestellt. Dabei ist in Fig. 3 eine vorzugsweise unter der Baugruppe aus Fluid-Versorgungsschnittstelle 62 und daran angekoppeltem Zellkulturbehälter 10 angeordnete Steueranordnung 94 dargestellt. Die Stellung der zweiten Ventilanordnung 86 in der zweiten Anschlussformation 78 und die Ventilstellung der Trenn-Ventilanordnung 74 unterscheiden sich zu Erklärungszwecken in Fig. 3 von jenen der Fig. 2.

Die Steueranordnung 94 kann eine um eine Walzenachse W drehbare Walze 96 aufweisen, die von einem Antrieb 98, etwa einem elektromotorischen Antrieb zur Drehung um die Walzenachse W antreibbar sein kann.

Über den Umfang der Mantelfläche 96a der Walze 96 kann eine Mehrzahl von Signalmitteln 100 angeordnet sein, welche im vorliegenden Beispiel durch Permanentmagnete gebildet sind. Diese Permanentmagnete sind vorzugsweise derart orientiert, dass ihre N-S-Polarisierungsrichtung mit einer von der Walzenachse W ausgehenden radialen Richtung übereinstimmt.

Dabei sind jeweils zu einer konkreten Ventilstellungskonfiguration der Ventilanordnungen 54, 74 und 86 der mit einem Zellkulturbehälter 10 in Kopplungseingriff stehenden Fluid-Versorgungsschnittstelle 62 gehörende Signalmittel 100 zu einem Satz 102 von Signalmitteln zusammengefasst. In Fig. 3 bildet daher die in der Schnittebene gelegene oberhalb der Walzenachse W gelegene Reihe von sechs Signalmitteln 100 einen derartigen Satz.

Diesem diametral gegenüber ist ein weiterer Satz von Signalmitteln vorgesehen, der in Fig. 3 allerdings nicht gezeichnet ist. Stattdessen sind lediglich die für die Signalmittel vorgesehenen Aufnahmeräume in der Walze 96 dargestellt. Zwischen diesen können längs des Umfangs der Walze 96 weitere Sätze von Signalmitteln vorgesehen sein.

Zwischen der Fluid-Ventilschnittstelle 62 und dem damit in Kopplungseingriff stehenden Zellkulturbehälter 10 einerseits und der Walze 96 andererseits kann eine Vermittlungsanordnung 104 vorgesehen sein, um ein zeitlich noch präziseres Schalten der Ventilanordnungen 54, 74 und 86 zu ermöglichen.

Wie in einem Satz 102 von Signalmitteln 100 der Walze 96 ist bevorzugt auch in der Vermittlungsanordnung für jede schaltbare Ventilanordnung 54, 74 und 86 einer Ventilstellungskonfiguration jeweils genau ein Permanentmagnet 106 vorgesehen. Jeder Permanentmagnet 106 ist dabei in einem Kanal 108 längs dieses Kanals 108 verschieblich zwischen einer walzennäheren Stellung und einer ventilanordnungsnäheren Stellung verschieblich gelagert.

Die Permanentmagneten 106 sind dabei derart ausgewählt, dass das von ihnen ausgehende und auf den Ventilkörper 56 wirkende Magnetfeld zumindest in der ventilanordnungsnäheren Stellung stärker ist als das von den magnetischen Spannbauteilen 60 ausgehende und auf den jeweiligen Ventilkörper 56 wirkende Magnetfeld. Weiter sind die Magnete 106 vorzugsweise längs ihrer Verlagerungsachse polarisiert angeordnet, etwa dergestalt, dass ein Pol, etwa der Nordpol, zu der jeweils zugeordneten Ventilanordnung hinweist und der jeweils andere Pol, etwa der Südpol, zur Walze 96 hinweist.

Die Vermittlungsanordnung 104 ist bevorzugt derart angeordnet, dass die Permanentmagnete 106 in den jeweiligen Kanälen 108 durch die durch den Pfeil g angedeutete Schwerkraft in ihre walzennähere Stellung vorgespannt sind, in der sich beispielhaft die Permanentmagnete Nr. 2, 3, 5 und 6 (bei Zählung von links nach rechts) in Fig. 3 befinden.

Durch entsprechende Orientierung der Signalmittel 100 können bei Annäherung dieser Signalmittel 100 an die Permanentmagnete 106 der Vermittlungsanordnung 104 durch die von den Signalmitteln 100 ausgehenden Magnetfelder die Permanentmagnete 106 von ihrer walzennäheren Stellung zu ihrer ventilanordnungsnäheren Stellung verlagert werden, etwa durch Gegenüberstellung gleichnamiger, also sich abstoßender Pole von Permanentmagneten 106 und Signalmittel 100. Durch Gegenüberstellung ungleichnamiger, also sich anziehender Pole der Permanentmagnete 106 und der Signalmittel 100 werden die Permanentmagnete 106 dagegen zusätzlich zur stets wirkenden Schwerkraft magnetisch in ihrer walzennäheren Stellung gehalten.

Bei Annäherung eines Magneten 106 der Vermittlungsanordnung 104 an die ihm zugeordnete Ventilanordnung wird der Ventilkörper 56 derselben durch den in seiner ventilanordnungsnäheren Stellung befindlichen Permanentmagneten 106 stärker angezogen als durch das Spannbauteil 60 der jeweiligen Ventilanordnung. Der Ventilkörper 56 bewegt sich daher aus seiner Sperrstellung, in welche er an dem Anlagebauteil 59 zum Verschluss einer Durchgangsöffnung durch die Ventilanordnung anliegt, in eine Stellung, in der ein Durchgang durch die jeweilige Ventilanordnung möglich ist und somit eine Strömung längs der Ventilanordnung zugeordneten Ventilströmungspfads gestattet ist.

Bei dem in Fig. 3 gezeigten Beispiel ist der Ventilkörper 56 der Ventilanordnung 86 der zweiten Anschlussformation durch die Steueranordnung 94 von seinem Ventilsitz 58, insbesondere von dem Anlagebauteil 59 entfernt, so dass der zweite Fluidströmungspfad 90 zum Durchfluss eines Fluids, hier: Reinigungsfluid, freigegeben ist.

Ebenso ist durch die Steuervorrichtung 94 in der in Fig. 3 gezeigten Stellung die Trenn-Ventilanordnung 74 in ihre Durchlassstellung verstellt, da auch deren Ventilkörper 56 vom Anlagebauteil 59 weg zum zugeordneten vierten Permanentmagneten der Vermittlungsanordnung 104 hin verlagert ist. Somit ist zusätzlich zum zweiten Fluidströmungspfad auch der zwischen dem ersten Kopplungsströmungspfad 44a und dem zweiten Kopplungsströmungspfad 47a verlaufende Verbindungsströmungspfad 75 für einen Fluiddurchfluss freigegeben.

Im vorliegenden Beispiel besteht eine 1:1-Zuordnung zwischen den Signalmitteln 100 und den Ventilanordnungen 54, 74 und 86, welche an einer mit einem Zellkulturbehälter 10 gekoppelten Fluid-Versorgungsschnittstelle 62 vorhanden sind. Ebenso besteht eine derartige 1:1-Zuordnung zwischen den Magneten 106 der Vermittlungsanordnung 104 und den vorhandenen Ventilanordnungen. Die in Fig. 3 ganz links angeordneten Signalmittel 100 und Permanentmagnete 106 sind der zweiten Ventilanordnung 86 in der zweiten Anschlussformation 78 zugeordnet. Deren rechte Nachbarn als Signalmittel 100 bzw. Permanentmagnet 106 sind der ersten Behälter-Ventilanordnung 54 an der ersten Gegenkopplungsformation 38 zugeordnet. Deren in Fig. 3 jeweilige rechte Nachbarn sind der ersten Ventilanordnung 86 der ersten Anschlussformation 76 zugeordnet. Deren rechte Nachbarn sind der Trenn-Ventilanordnung 74 zugeordnet. Deren rechte Nachbarn sind der zweiten Behälter-Ventilanordnung 54 der zweiten Gegenkopplungsformation 40 zugeordnet und die in Fig. 3 ganz rechte Kombination aus Signalmittel 100 und Permanentmagnet 106 ist der dritten Ventilanordnung 86 der dritten Anschlussformation 80 zugeordnet.

Die um den Umfang der Mantelfläche 96a der Walze 96 verteilten Sätze 102 von Signalmitteln 100 weisen jeweils unterschiedlich in ihrer Polarisation angeordnete Singalmittel 100 auf, um die dem jeweiligen Signalmittelsatz 102 zugeordnete Ventilstellungskonfiguration aller beteiligten sechs Ventilanordnungen 54, 74 und 86 durch Annäherung des jeweiligen Satzes 102 von Signalmitteln 100 an die Vermittlungsanordnung 104 einzustellen.

Die in Fig. 3 gezeigte Stellung der Ventilanordnungen 54, 74 und 86 ist lediglich beispielhafter Natur und keiner konkreten Funktion zugeordnet.

In den Fig. 4 und 5 ist beispielhaft gezeigt, wie das Anlagebauteil 59 der in der vorliegenden Ausführungsform dargestellten Ventilanordnungen 54 zur Abdichtung der Kopplungsstelle zwischen der versorgungsschnittstellenseitigen ersten Kopplungsformation 64 und der zellkulturbehälterseitigen Gegenkopplungsformation 38 dient. Das dabei geschilderte Prinzip gilt aber auch für die Kopplung aus zweiter Kopplungsformation 66 und zweiter Gegenkopplungsformation 40 und würde auch an der Kopplungsstelle einer möglicherweise vorhandenen dritten Kopplungsformation und dritten Gegenkopplungsformation gelten.

Ebenso sind die montagebedingt vorteilhaft an einem spannbauteilfernen Längsende verlaufenden Trennflächen zwischen einzelnen Bauteilen des Gehäuses 68 der Fluid-Versorgungsschnittstelle 62 nach dem in den Fig. 4 und 5 gezeigten Prinzip durch die verformende Anlage des Anlagebauteils 59 an eine Gegendichtfläche eines anderen Gehäusebauteils abgedichtet.

In Fig. 4 ist die erste Gegenkopplungsformation 38 außer Eingriff mit der zugeordneten ersten Kopplungsformation 66 dargestellt, so dass zwar der Ventilkörper 56 an der negativ-konischen Anlagefläche 59a des Anlagebauteils 59 dichtend anliegt - und zwar aufgrund der magnetischen Spannkraft, die vom Spannbauteil 60 ausgeht - jedoch ist das Anlagebauteil 59 ansonsten im Wesentlichen unverformt. Das Anlagebauteil 59 weist an seinem Spannbauteil fernen Längsende eine umlaufende Stirnringfläche 59b auf. In dem dargestellten Beispiel ist diese Ringfläche 59b orthogonal zur Zeichenebene der Fig. 4 orientiert. In diesem Zustand kann die Gegenkopplungsformation durch eine strichliniert halbseitig dargestellte Aufsteckkappe 61 bedeckt und vor Verschmutzung geschützt sein.

Die Kopplungsformation 36 weist eine Gegendichtfläche 64a auf, welche dazu ausgebildet ist, bei hergestelltem fluidübertragendem Kopplungseingriff zwischen der ersten Kopplungsformation 64 und der ersten Gegenkopplungsformation 38 in Anlageeingriff mit der ringförmigen Endfläche (Strinringfläche) 59b des Anlagebauteils 59 zu gelangen.

In Fig. 5 sind die erste Kopplungsformation 64 und die erste Gegenkopplungsformation 38 bei hergestelltem Kopplungseingriff dargestellt, wie er auch in Fig. 2 zu sehen ist.

Dort ist zu erkennen, wie das spannbauteilfernere Längsende des Anlagebauteils 59 mit seiner Stirnringfläche 59b unter Verformung in Anlageeingriff mit der Gegendichtfläche 64a des die erste Kopplungsformation 64 aufweisenden Gehäusebauteils des Gehäuses 68 der Fluid-Versorgungsschnittstelle 62 gelangt ist. Durch diese verformende Anlage ist die Kopplungsstelle und sind insbesondere der Kopplungsströmungspfad 44a und der Liefer-Fluidströmungspfad 44 nach radial außen gegen unerwünschten Fluidaustritt abgedichtet. Gleiches gilt für einen unerwünschten Fluideintritt in diese Fluidströmungspfade.

Diese oben gegebene Beschreibung der Fig. 4 und 5 trifft für die radiale Abdichtung aller in der vorliegend diskutierten Ausführungsform gezeigten Ventilanordnungen zu.

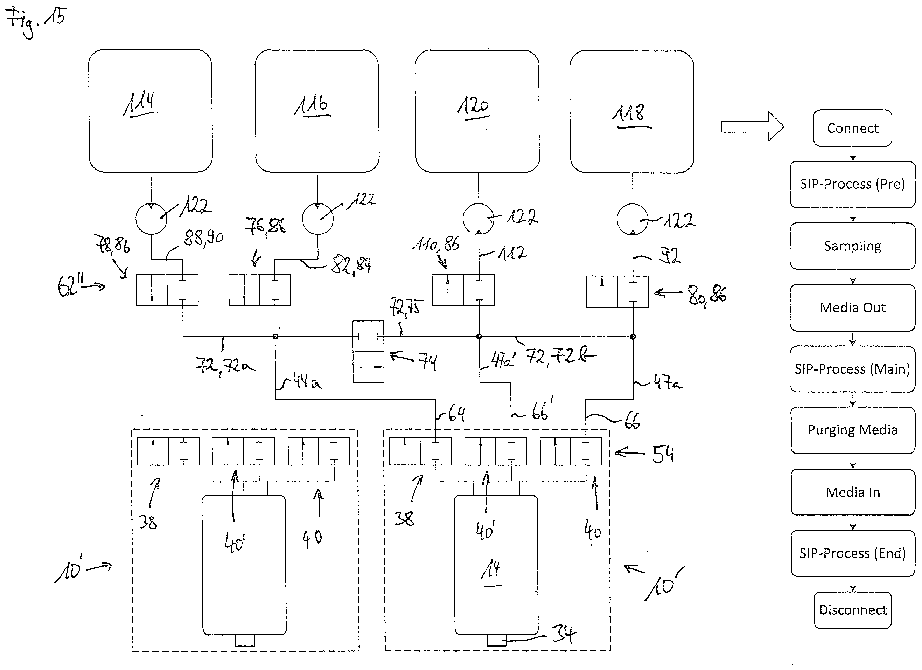

In den Fig. 6 bis 14 ist schematisch ein beispielhafter Betriebsvorgang einer Zellkulturanlage gezeigt mit Zellkulturbehältern 10 gemäß Fig. 1 und mit einer modifizierten Fluid-Versorgungsschnittstelle 62', welche im Gegensatz zu der in den Fig. 2 und 3 gezeigten Fluid-Versorgungsschnittstelle 62 eine vierte Anschlussformation aufweist. Die vierte Anschlussformation ist in den Fig. 6 bis 14 (sowie in den Fig. 15 bis 21) mit 110 bezeichnet. Diese weist ebenfalls eine Ventilanordnung 86 auf. Der vierte Fluidströmungspfad, welcher von dem Strömungsraum 72 durch die vierte Ventilanordnung 86 der vierten Anschlussformation 10 hindurch verläuft, trägt das Bezugszeichen 112.

Der Reinigungsfluidvorrat ist mit 114 bezeichnet. Der Nährmediumvorrat trägt das Bezugszeichen 116. Ein an die dritte Anschlussformation 80 angeschlossener Entsorgungsbehälter ist mit 118 bezeichnet. An die vierte Anschlussformation 110 ist ein Probeentnahmebehälter 120 angeschlossen, in welchem entnommene Proben aus dem jeweils angeschlossenen Zellkulturbehälter 110 afugefangen werden.

Zwischen den jeweiligen ersten bis vierten Anschlussformationen 76, 78, 80 und 110 und den mit ihnen über Fluidleitungen verbundenen Vorräten bzw. Behältern 114, 116, 118 und 120 sind nicht weiter interessierende Förderpumpen 122 vorgesehen, welche eine Förderung von Fluiden in den an sie angeschlossenen Fluidleitungen in die jeweils gewünschte Förderrichtung gewährleisten.

In Fig. 6 ist eine Basisventilstellungskonfiguration gezeigt, wie sie beim Herstellen eines fluidübertragenden Kopplungseingriffs zwischen der ersten und der zweiten Kopplungsformation 64 und 66 und der ersten und der zweiten Gegenkopplungsformation 38 bzw. 40 vorliegt. Alle vorhandenen Ventilanordnungen 54, 74 und 86 sind dabei in ihrer Sperrstellung um jeglichen unerwünschten Fluidmittelstrom zu vermeiden.

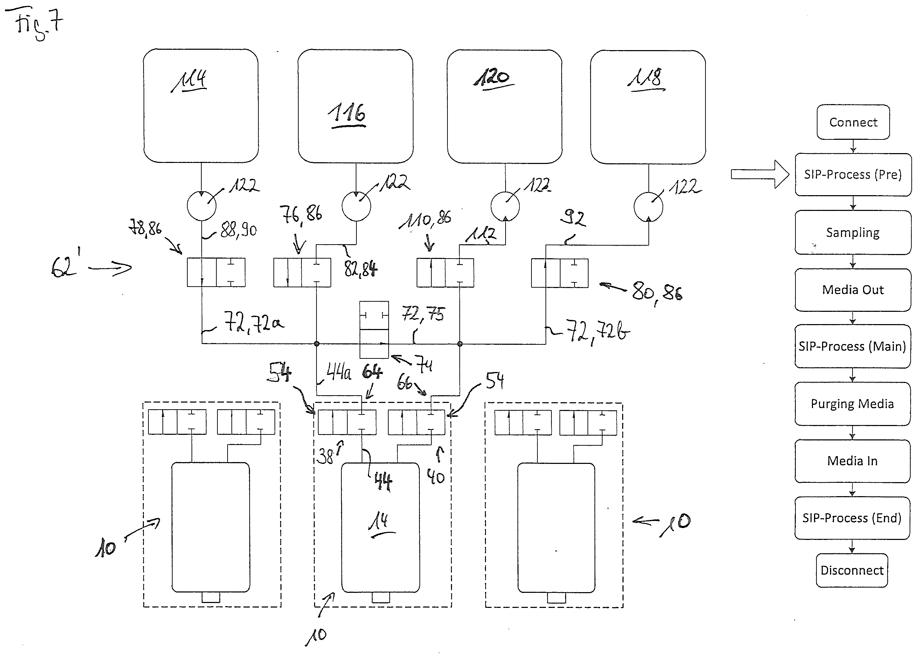

Fig. 7 zeigt einen vorzugsweise auf die Herstellung des Kopplungseingriffs folgenden SIP-Prozess, mit welchem ein vorbestimmter Reinigungszustand an den Behälter-Ventilanordnungen 54 und in der Fluid-Versorgungsschnittstelle 62' hergestellt wird. Hierzu ist die Trenn-Ventilanordnung 74 und sind die Ventilanordnungen 86 der zweiten und der dritten Anschlussformation 78 bzw. 80 in ihre Durchlassstellung geschaltet. Über die an den Reinigungsfluidvorrat 114 gekoppelte Pumpe 122 wird Reinigungsfluid aus dem Vorrat 114 durch die zweite Anschlussformation 78 in den Strömungsraum 72 eingeleitet, durch diesen hindurchgeleitet und durch die dritte Anschlussformation 80, ggf. unter Unterstützung der an den Fluidströmungspfad 92 angeschlossenen Förderpumpe 122 in den Entsorgungsbehälter 118 gefördert. Aufgrund des Einragens der Ventilkörper 56 in den so mit Reinigungsfluid durchspülten Strömungsraum 72 findet eine Reinigung auch der beteiligten Ventilanordnungen 54, 74 und 86 statt, die einen Sauberkeitszustand herstellt, der eine Kreuzkontamination zwischen zeitlich aufeinanderfolgend angekoppelten unterschiedlichen Zellkulturbehältern 10 vermeidet.

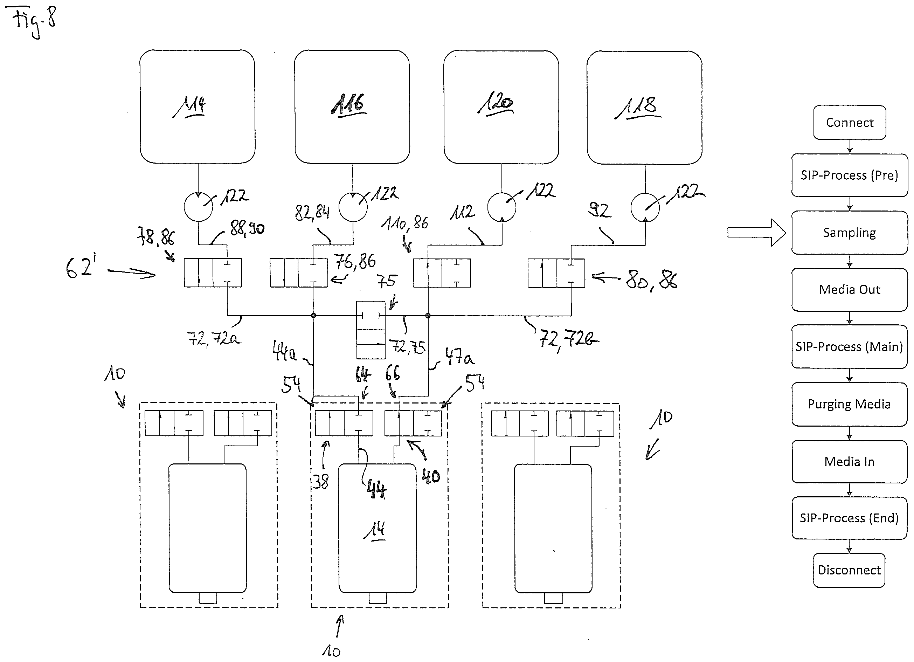

In Fig. 8 ist an den zuvor geschilderten SIP-Reinigungsprozess anschließend eine Stichprobenentnahme gezeigt. Die Wahl der Reihenfolge von Medienentnahme aus einem Zellkulturbehälter 10 bevor in diesen Medien eingeleitet werden, hat den Vorteil, dass ein Einleiten von Nährmedium in den Zellkulturbehälter 10 erst nach einem weiteren Reinigungsvorgang erfolgt, was das Risiko einer Kreuzkontamination aufgrund einer Verschmutzung eines zuvor in Kopplungseingriff genommenen Zellkulturbehälters 10 weiter reduziert.

Zur stichprobenhaften Medienentnahme ist die Behälter-Ventilanordnung 38 der ersten Gegenkopplungsformation in ihre Durchlassstellung geschaltet. Außerdem ist die Ventilanordnung 86 der vierten Anschlussformation 110 in ihre Durchlassstellung geschaltet. Alle übrigen Ventilanordnungen befinden sich in ihrer Sperrstellung. Mit der zwischen der vierten Anschlussformation 110 und dem Probeentnahmebehälter 120 vorgesehenen Förderpumpe 122 kann somit eine vorbestimmte Menge an Medium aus dem Kulturvolumen 14 des angekoppelten Zellkulturbehälters 10 in den Probeentnahmebehälter 120 abgeführt werden. Die Trenn-Ventilanordnung 74 sorgt dabei dafür, dass der Teilströmungsraum 72a von dem aus dem Zellkulturbehälter 10 entnommenen Medium nicht erreicht wird.

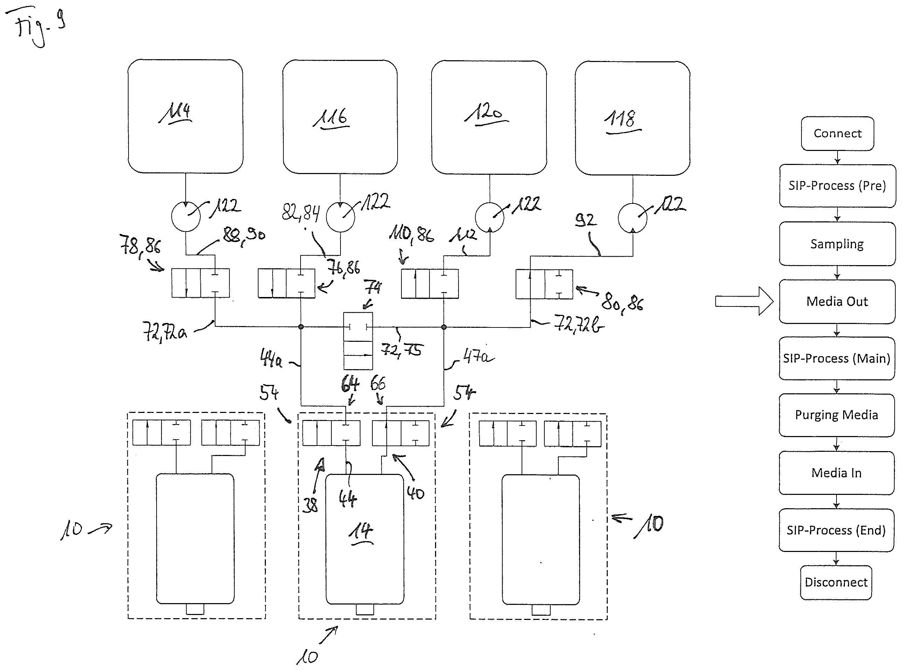

In Fig. 9 ist ein vorteilhafterweise auf die Probeentnahme folgendes Auslassen von gebrauchtem oder verbrauchtem Nährmedium aus dem Kulturvolumen 14 des angekoppelten Zellkulturbehälters 10 gezeigt. Der Teilströmungsraum 72b, der im vorhergehenden Sampling-Verfahrensschritt bereits mit dem aus dem Kulturvolumen 14 entnommenen Medium gefüllt wurde, wird nun erneut oder bleibt mit dem selben Medium gefüllt. Allerdings wird im Gegensatz zum vorhergehenden Schritt das durch die zweite Gegenkopplungsformation 40 ausgeleitete gebrauchte Nährmedium nun durch die Ventilanordnung 86 der dritten Anschlussformation 80 vermittels der daran unmittelbar angeschlossenen Förderpumpe 122 in den Entsorgungsbehälter 118 gefördert. Die übrigen Ventilanordnungen 86 der ersten, zweiten und vierten Anschlussformation sowie die zweite Behälter-Ventilanordnung 54 der zweiten Gegenkopplungsformation 40 und die Trenn-Ventilanordnung 74 befinden sich in ihrer Sperrstellung, um eine unnötige Benetzung von Strömungspfaden durch gebrauchtes Nährmedium zu vermeiden.

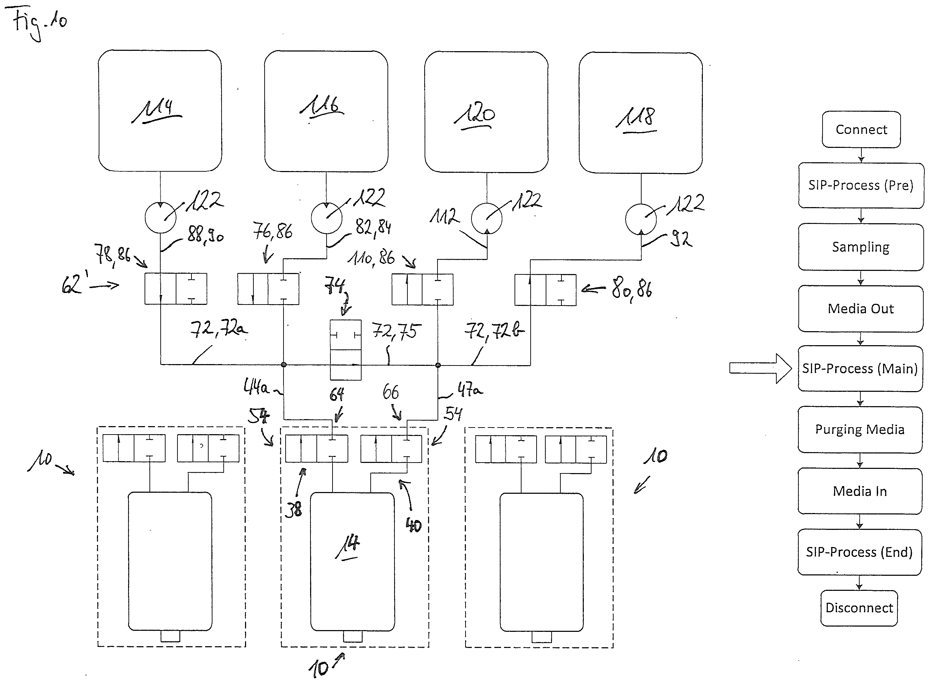

Bevor eine Befüllung des Zellkulturbehälters 10 mit frischem Nährmedium erfolgt, findet eine erneute SIP-Reinigung statt, welche in Fig. 10 gezeigt ist. Die dort gezeigte Ventilstellungskonfigurationentspricht exakt jener von Fig. 7, welche demselben Ziel diente, und auf deren Beschreibung ausdrücklich verwiesen wird.

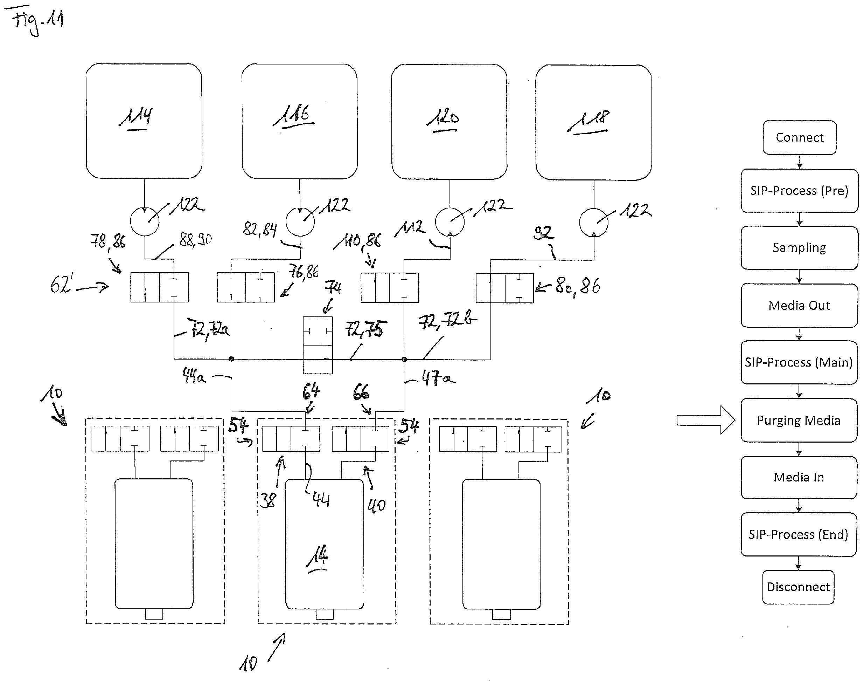

In Fig. 11 ist dargestellt, wie vorteilhaft vor dem Befüllen des Zellkulturbehälters 10 mit frischem Nährmedium die daran beteiligten Fluidströmungspfade mit frischem Nährmedium gespült werden, um etwaig noch vorhandene Reste an Reinigungsflüssigkeit aus der Fluid-Versorgungsschnittstelle 62' zu entfernen. Hierzu sind beide Behälter-Ventilanordnungen 54 - wie schon zuvor bei dem SIP-Reinigungsvorgang - in der Sperrstellung.

Von den Ventilanordnungen der Fluid-Versorgungsschnittstelle 62' befindet sich die Ventilanordnung 86 der ersten Anschlussformation 76, die Trenn-Ventilanordnung 74 und die Ventilanordnung 86 der dritten Anschlussformation in ihrer Durchlassstellung. Alle übrigen Ventilanordnungen befinden sich in der Sperrstellung. Die an die betreffenden Anschlussformationen mit geöffneten Ventilanordnungen angeschlossenen Förderpumpen 122 sorgen für einen Durchgang von frischem Nährmedium ausgehend vom Nährmediumvorrat 116 in den Entsorgungsbehälter 118.

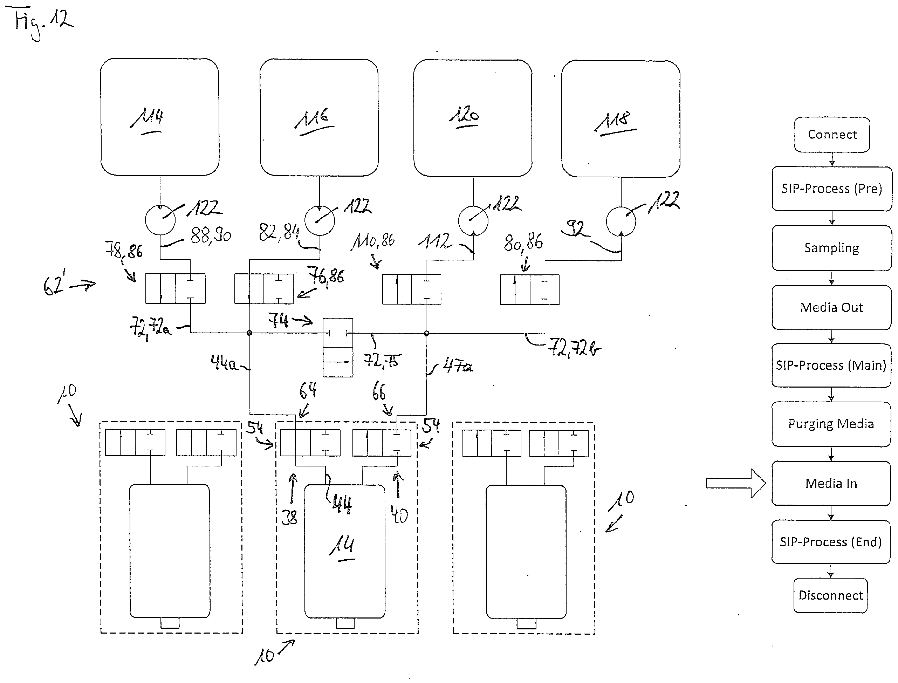

In Fig. 12 ist schließlich der auf den zuvor beschriebenen Spülvorgang folgende Vorgang eines Befüllens des Zellkulturbehälters 10 mit frischem Nährmedium dargestellt. Hierzu ist zum einen die erste Behälter-Ventilanordnung 54 der ersten Gegenkopplungsformation 38 in ihre Durchlassstellung verstellt, während sich die zweite Behälter-Ventilanordnung 54 der zweiten Gegenkopplungsformation 40 in ihrer Sperrstellung befindet. Auf Seiten der Fluid-Versorgungsschnittstelle 62' befinden sich alle Ventilanordnungen in der Sperrstellung mit Ausnahme der Ventilanordnung 86 der ersten Anschlussformation 76. Somit kann durch die an diese erste Anschlussformation 76 angeschlossene Förderpumpe 122 aus dem Nährmediumvorrat 116 frisches Nährmedium durch die erste Anschlussformation 76, den TeilStrömungsraum 72a, den ersten Kopplungsströmungspfad 44a und die erste Gegenkopplungsformation 38 in das Kulturvolumen 14 des Zellkulturbehälters 10 eingeleitet werden.

Somit wird der Teilströmungsraum 72a stets nur von frischem Nährmedium oder von Reinigungsfluid durchströmt. Der jeweils andere Teilströmungsraum 72b wird dagegen lediglich von gebrauchtem Nährmedium oder von Reinigungsfluid durchströmt. Durch die Trennung des Strömungsraums 72 in die beiden Teilströmungsräume kann also verhindert werden, dass gebrauchtes Nährmedium eines vorhergehend angekoppelten Zellkulturbehälters 10 sich noch in Resten in einem von frischem Nährmedium durchströmten Teilströmungsraum befindet und von dort in einen nachfolgend angekoppelten Zellkulturbehälter 10 gelangen kann. Dies zeigt die weitere Reduzierung des Risikos einer Kreuzkontamination.

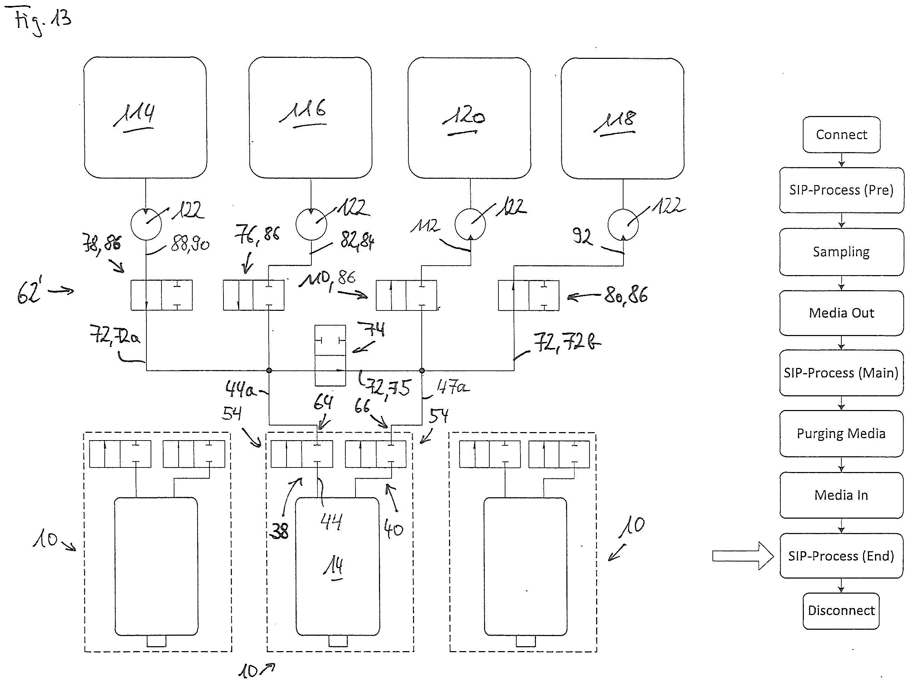

Auf das Befüllen des angekoppelten Zellkulturbehälters 10 mit frischem Nährmedium erfolgt vor dem Lösen des Kopplungseingriffs mit dem noch angekoppelten Zellkulturbehälter 10 ein weiterer SIP-Reinigungsvorgang, welcher in Fig. 13 dargestellt ist. Die Ventilstellungskonfiguration entspricht dabei aus nachvollziehbaren Gründen jener der Fig. 10 und 7, da es sich bei jenen um im Wesentlichen denselben Reinigungsvorgang handelt.

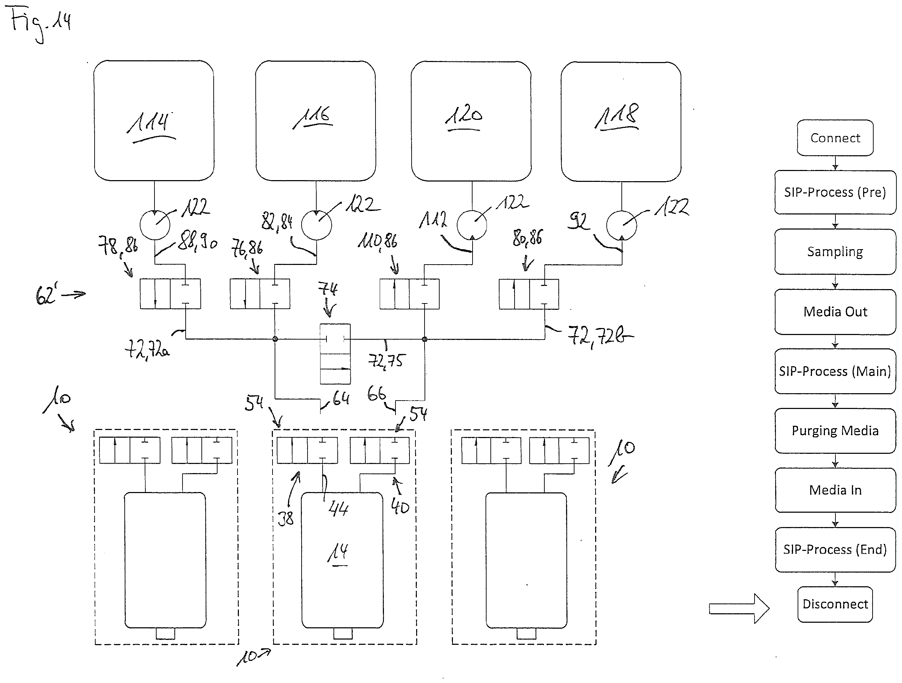

Nach einem Reinigen der Fluid-Versorgungsschnittstelle 62' sowie der beteiligten Behälter-Ventilanordnungen und der benetzbaren Stellen der Gegenkopplungsformationen 38 und 40 erfolgt ein Abkoppeln der Fluid-Versorgungsschnittstelle 62' vom bis dahin angekoppelten Zellkulturbehälter 10. Dieser Abkoppelvorgang ist in Fig. 14 dargestellt.

In den Fig. 15 bis 18 sind jene Vorgänge des bereits in den Fig. 6 bis 14 dargestellten Verfahrens dargestellt, welche nicht ausschließlich einen Fluidfluss von einem Reservoir durch die Fluid-Verbindungsschnittstelle in einen Behälter 118 oder 120 betreffen, ohne dass Fluid in einen Zellkulturbehälter gelangt oder aus diesem entnommen wird. Solche Verfahrensschritte ohne Änderung des Kopplungs- oder Strömungszustands am Kopplungseingriff zwischen Zellkulturbehälter und Fluid-Versorgungsschnittstelle sind alle SIP-Reinigungsschritte ("SIP-Process") sowie das Spülen der Fluid-Versorgungsschnittstelle mit Nährmedium ("Purging Media").

In Fig. 15 ist der Zustand der Zellkulturanlage mit alternativen Zellkulturbehältern 10' und alternativer Fluid-Versorgungsschnittstelle 62" unmittelbar nach Herstellen eines fluidübertragenden Kopplungseingriffs zwischen den Kopplungsformationen der Fluid-Versorgungsschnittstelle 62" und dem Zellkulturbehälter 10' dargestellt.

Der alternative Zellkulturbehälter 10' der Zellkulturanlage gemäß Fig. 15 bis 19 unterscheidet sich von dem bisher diskutierten Zellkulturbehälter 10 lediglich dadurch, dass er zusätzlich zu der ersten Gegenkopplungsformation 38 und der zweiten Gegenkopplungsformation 40 eine weitere dritte Gegenkopplungsformation 40' aufweist.

Entsprechend weist die Fluid-Versorgungsschnittstelle 62" zusätzlich zur ersten Kopplungsformation 64 und zur zweiten Kopplungsformation 66 eine dritte Kopplungsformation 66' auf. Die dritte Kopplungsformation 66' und die dritte Gegenkopplungsformation 40' können vorteilhafterweise wie die erste und die zweite Kopplungsformation bzw. Gegenkopplungsformation ausgebildet sein und sich von diesen außer durch den Anbringungsort am Zellkulturbehälter 10' und an der Fluid-Versorgungsschnittstelle 62" nicht unterscheiden.

Bei dem in Fig. 15 gezeigten Ankopplungsvorgang ("Connect") sind aus nachvollziehbaren Gründen alle Behälter-Ventilanordnungen 54 der ersten bis dritten Gegenkopplungsformation in der Sperrstellung, um ein unkontrolliertes Austreten von Fluid aus dem Kulturvolumen 14 zu vermeiden.

Alle Ventilanordnungen 74 und 86 der Fluid-Versorgungsschnittstelle 62" sind beim Kopplungsvorgang der Fig. 15 ebenfalls in ihrer Sperrstellung, um ein unerwünschtes Austreten von Fluid aus einem der Vorräte 114 und 116 oder den Behältern 118 und 120 zu vermeiden. Außerdem sind bevorzugt die Pumpen 122 ausgeschaltet.

Der an das Ankoppeln folgende SIP-Reinigungsvorgang entspricht jenem von Fig. 7 (siehe obige Beschreibung) mit der Maßgabe, dass alle drei Behälter-Ventilanordnungen 54 des alternativen Zellkulturbehälters 10' in der Schließstellung sind.

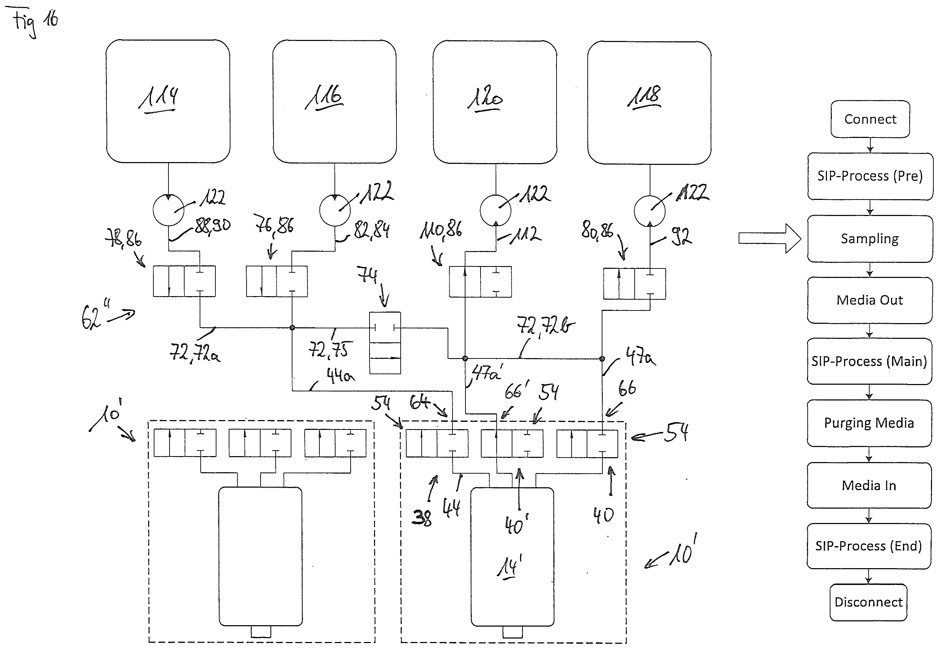

In Fig. 16 ist die Stichprobenentnahme aus dem angekoppelten alternativen Zellkulturbehälter 10' dargestellt. Die Stellung der fluid-versorgungsschnittstellenseitigen Ventilanordnungen 74 und 86 entspricht exakt jener von Fig. 8. Dies bedeutet, die Ventilanordnung 86 der vierten Anschlussformation befindet sich in Durchlassstellung, alle übrigen Ventilanordnungen der Ventil-Versorgungsschnittstellte 62" befinden sich in ihrer Sperrstellung.

Da der alternative Zellkulturbehälter 10' drei Gegenkopplungsformationen 38, 40 und 40' aufweist und bevorzugt die erste Gegenkopplungsformation 38 wie im vorhergehenden Beispiel zur Zufuhr von frischem Nährmedium in das Kulturvolumen 14 verwendet werden soll, ist weiterhin bevorzugt die zweite Gegenkopplungsformation 40 ausschließlich zum Auslassen von gebrauchtem Fluid aus dem Kulturvolumen 14 für die Entsorgung vorgesehen und ist die dritte Gegenkopplungsformation 40' mit der dieser zugeordneten Behälter-Ventilanordnung 54 und dem zugeordneten Liefer-Fluidströmungspfad ausschließlich einer Stichprobenentnahmefunktion zugeordnet. Aus diesem Grunde ist bei dem in Fig. 16 dargestellten Verfahren nur die dritte Behälter-Ventilanordnung 54 der dritten Gegenkopplungsformation 40' in ihre Durchlassstellung geschaltet, während die übrigen Behälter-Ventilanordnungen 54 des Zellkulturbehälters 10' in ihrer Sperrstellung sind.

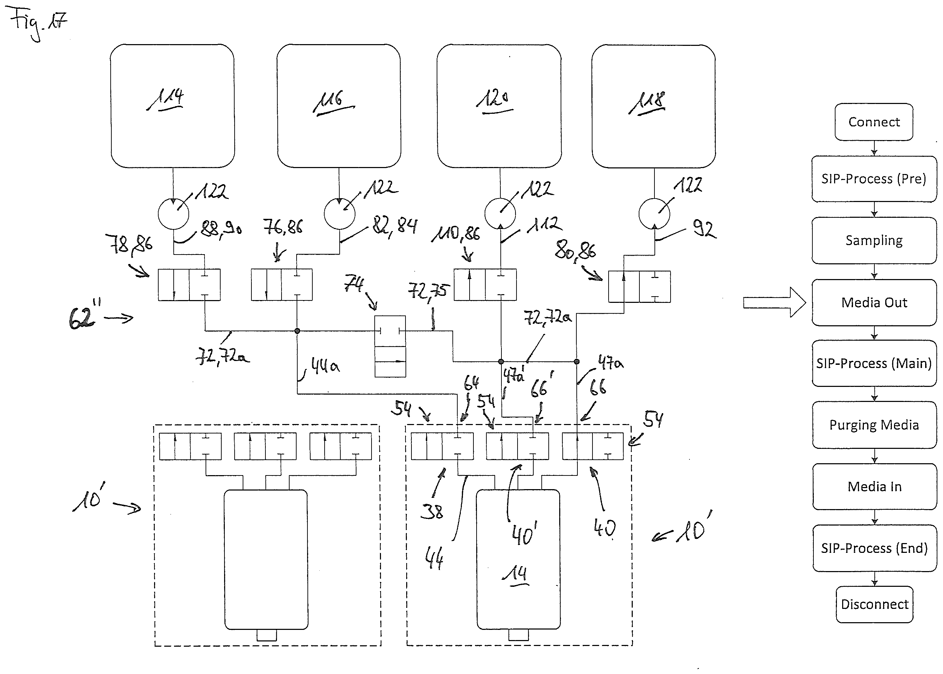

Entsprechend dem oben Gesagten ist bei der in Fig. 17 dargestellten Entsorgung von gebrauchtem Fluid aus dem Kulturvolumen 14 in den Entsorgungsbehälter 118 nur die zweite Behälter-Ventilanordnung 54 der zweiten Behälter-Gegenkopplungsformation 40 in ihre Durchlassstellung geschaltet, während die erste und die dritte Behälter-Ventilanordnung der ersten und der dritten Gegenkopplungsformation 38 bzw. 40' in ihrer Sperrstellung sind. Die Ventilstellungskonfiguration der Ventilanordnungen 74 und 86 der Fluid-Versorgungsschnittstelle 86 entsprechen jener von Fig. 9, auf deren Beschreibung ausdrücklich verwiesen wird.

Auf die Entsorgung von gebrauchtem Fluid aus dem Kulturvolumen 14, insbesondere gebrauchtem Nährmedium, folgt, wie bei dem vorhergehenden Beispiel auch, ein nun nicht eigens dargestellter SIP-Reinigungsvorgang. Da bei diesem alle Behälter-Ventilanordnungen 54 sich in ihrer Sperrstellung befinden und die Ventilstellungskonfiguration der Fluid-Versorgungsschnittstelle 62" exakt jener von Fig. 10 entspricht, wird für den nachfolgenden SIP-Reinigungsvorgang ausdrücklich auf die Beschreibung der Fig. 10 verwiesen.

Gleiches gilt für den Spülvorgang, bei welchem etwaig in der Fluid-Versorgungsschnittstelle noch vorhandenes Reinigungsfluid durch frisches Nährmedium ausgespült wird. Für diesen im vorliegenden Beispiel ebenfalls nicht eigens dargestellten Vorgang wird ausdrücklich auf die Beschreibung der vorhergehenden Figur 11 verwiesen.

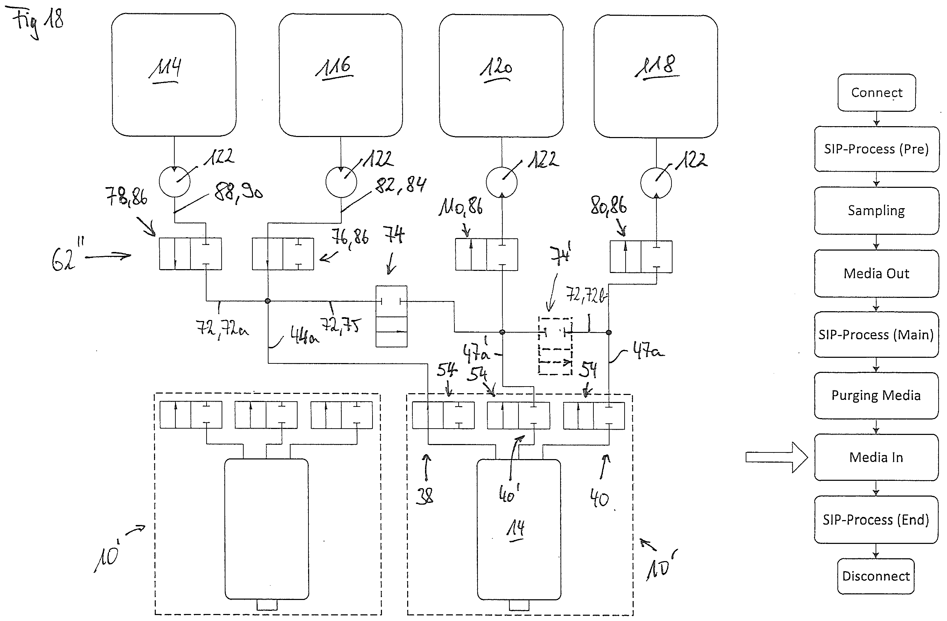

Fig. 18 zeigt den Vorgang des Einleitens eines frischen Nährmediums in das Zellkulturvolumen 14 des alternativen Zellkulturbehälters 10'. Die fluid-versorgungsschnittstellenseitigen Ventilanordnungen 74 und 86 entsprechen in ihrer Ventilstellungskonfiguration jener von Fig. 12, auf deren Beschreibung im Zusammenhang mit der Ventilstellung der fluid-versorgungsschnittstellenseitigen Ventilanordnungen und den zugehörigen Pumpenbetrieb ausdrücklich verwiesen wird.

Seitens des Zellkulturbehälters 10' ist nur die erste Behälter-Ventilanordnung 54 der ersten Gegenkopplungsformation 38 in ihrer Durchlassstellung, die übrigen beiden Behälter-Ventilanordnungen befinden sich in der Sperrstellung.

Bezüglich des nachfolgenden SIP-Verfahrensschritts und der Abkopplung der Ventil-Versorgungsschnittstelle 62" von dem Zellkulturbehälter 10' wird auf die Beschreibung der Fig. 13 und 14 verwiesen mit der Maßgabe, dass sich während dieser Verfahrensschritte alle drei Behälter-Ventilanordnungen 54 des Zellkulturbehälters 10' in der Sperrstellung befinden. Die Ventilstellungskonfiguration der fluid-versorgungsschnittstellenseitigen Ventilanordnungen 74 und 86 entspricht jenen der Fig. 13 und 14.

Ergänzend wird darauf hingewiesen, dass an der alternativen Fluid-Versorgungsschnittstelle 62", wie sie in den Fig. 15 bis 18 gezeigt ist, nicht nur die tatsächlich dargestellte Trenn-Ventilanordnung 74 vorgesehen sein kann, sondern es kann über diese hinaus eine zweite Trenn-Versorgungsschnittstelle 74' vorgesehen sein, welche sich vorzugsweise an der in Fig. 18 dargestellten strichlinierten Stelle befindet. Diese zweite Trenn-Ventilanordnung 74' ist immer dann in ihrer Durchlassstellung, wenn ein SIP-Reinigungsschritt oder ein Spülschritt mit frischem Nährmedium ausgeführt wird, also dann, wenn Fluid von einem Vorrat zum Entsorgungsbehälter gefördert werden soll. In allen übrigen Verfahrensschritten ist auch eine zweite Trenn-Ventilanordnung 74' bevorzugt in ihrer Sperrstellung.

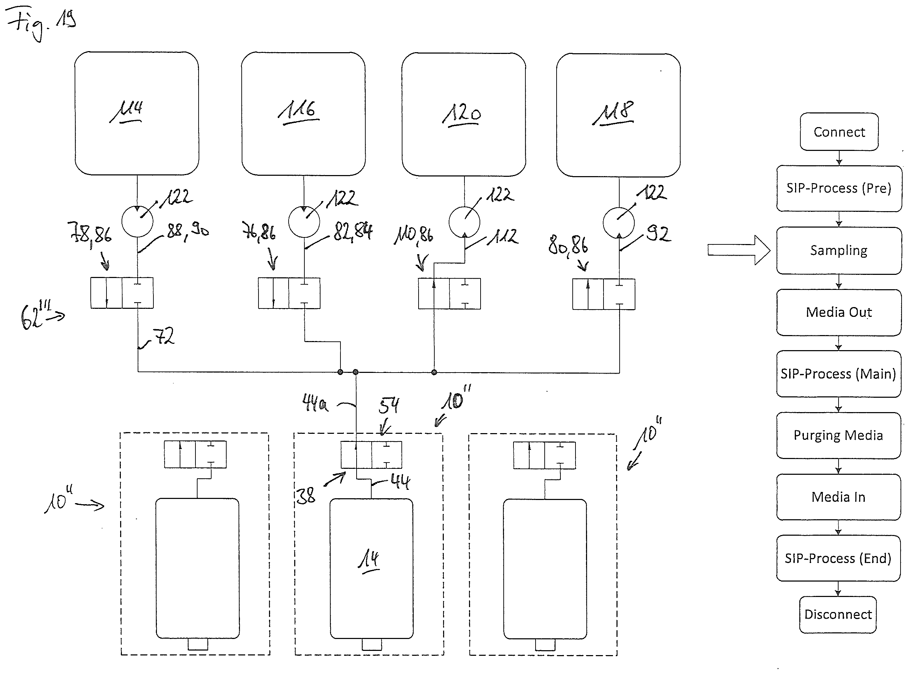

In den Fig. 19 bis 21 ist die oben beschriebene Zellkulturanlage mit einem weiter modifizierten Zellkulturbehälter 10" dargestellt. Dieser Zellkulturbehälter 10' weist nur eine einzige Gegenkopplungsformation 38 auf. Dementsprechend weist auch die weiter modifizierte Fluid-Versorgungsschnittstelle 62"' nur eine einzige Kopplungsformation 64 auf. Da somit jede Strömung zum Zellkulturbehälter 10" und von diesem durch ein- und denselben Kopplungsströmungspfad 44a verläuft, kann - muss aber nicht - eine Trenn-Ventilanordnung entfallen. In dem in den Fig. 19 bis 21 dargestellten Beispiel ist die Trenn-Ventilanordnung weggelassen. Ansonsten entspricht die Fluid-Versorgungsschnittstelle 62'" hinsichtlich der Anschlussformationen und der dort vorgesehenen Ventilanordnungen 86 sowie der daran angeschlossenen Förderpumpen 122, Vorräte 114 und 116 sowie Behälter 118 und 120 den zuvor beschriebenen Fluid-Versorgungsschnittstellen 62' und 62", auf deren Beschreibung zur Erläuterung der Ausführungsform der Fig. 19 bis 21 ausdrücklich verwiesen wird.

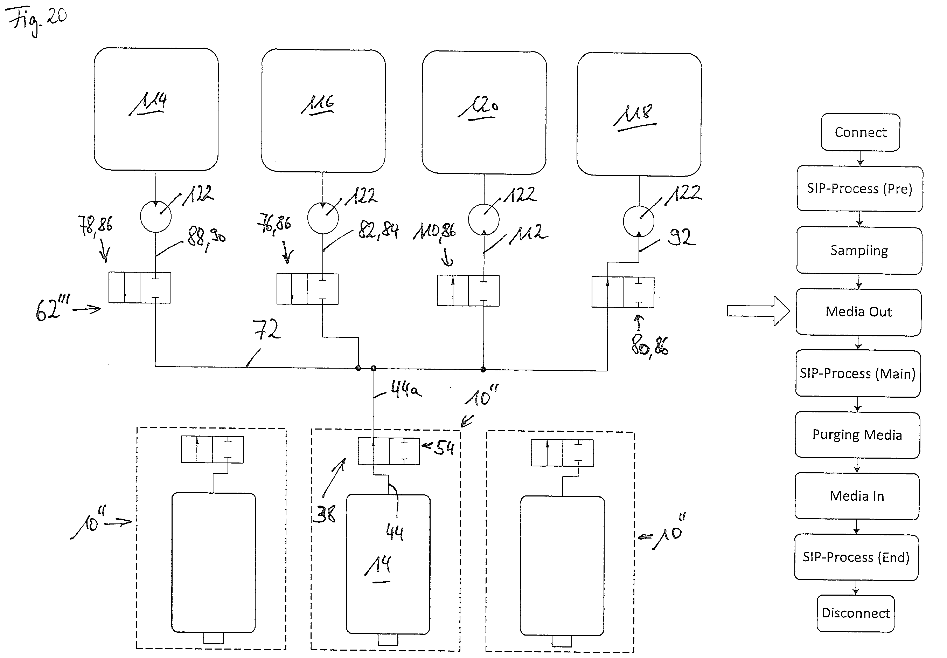

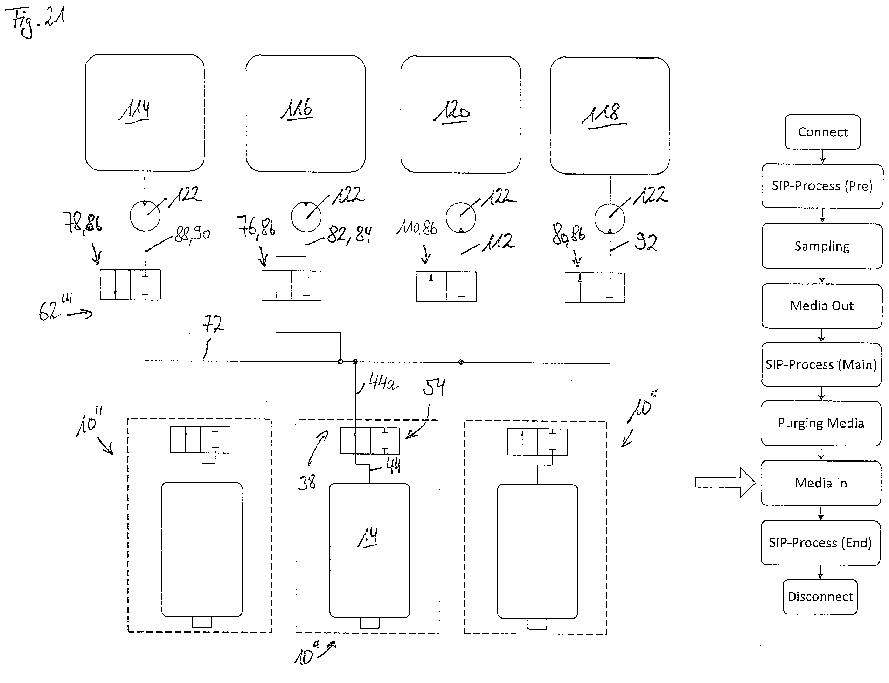

In den Fig. 19, 20 und 21 sind nur jene Verfahrensschritte der Entnahme von Fluid aus dem Kulturvolumen 14 und des Befüllens des Kulturvolumens 14 dargestellt, bei welchen tatsächlich Fluid durch den Liefer-Fluidströmungspfad 44 der einzigen Gegenkopplungsformation 38 strömt. Dies sind die Verfahrensschritte der Stichprobenentnahme ("Sampling" - Fig. 19), der Medienentsorgung ("Media Out" - Fig. 20) und der Einleitung von frischem Nährmedium in das Kulturvolumen 14 ("Media In" - Fig. 21). In allen übrigen Verfahrensschritten ist die einzige Behälter-Ventilanordnung 54 der einzigen Gegenkopplungsformation 38 in ihrer Sperrstellung. Die Ventilanordnungen 86 der Fluid-Versorgungsschnittstelle 62'" befinden sich in gleichen Verfahrensschritten (siehe Verfahrensfolge am rechten Rand der Fig. 6 bis 21) jeweils in der gleichen Stellung wie die zuvor beschriebenen Modifikationen 62' und 62". Dies gilt auch für die eigens gezeigten Verfahrensschritte der Stichprobenentnahme (Fig. 19), der Medienausleitung aus dem Zellkulturbehälter 10" (Fig. 20) und der Einleitung von frischem Nährmedium in den Zellkulturbehälter 10" (Fig. 21). Insofern entspricht die Ventilstellungskonfiguration der Ventilanordnungen 86 der Fluid-Versorgungsschnittstelle 62'" von Fig. 19 jener von Fig. 8 und Fig. 16, der Ventilanordnungen 86 von Fig. 20 entspricht jener der der Fig. 9 und 17, und die Ventilstellungskonfiguration der Ventilanordnungen 86 der Fig. 21 entspricht jener der der Fig. 12 und 18. Zur Beschreibung der Fig. 19 bis 21 wird ausdrücklich auf die Beschreibung der oben genannten ausführlicher beschriebenen Figuren verwiesen.

Im Unterschied zu den zuvor beschriebenen Ausführungsformen strömt nun Fluid stets entlang ein- und desselben Liefer-Fluidströmungspfads und Kopplungsströmungspfads, unabhängig davon, ob frisches Nährmedium in den Zellkulturbehälter 10" eingeleitet, aus diesem entsorgt oder zur Stichprobenüberprüfung aus diesem entnommen wird.

## Patentansprüche

1. Fluid-Versorgungsschnittstelle (62; 62'; 62"; 62"') für eine Zellkulturanlage zur Versorgung von in unterschiedlichen Zellkulturbehältern (10; 10'; 10") vorhandenen Zellkulturen mit einem Nährmedium, wobei die Fluid-Versorgungsschnittstelle (62; 62'; 62"; 62"') umfasst:
ein einen Strömungsraum (72) definierendes Gehäuse (68),
eine erste Anschlussformation (76) zur fluidübertragenden Verbindung einer ersten Fluidleitung (84) mit dem Gehäuse (68),
eine von der ersten gesondert ausgebildete zweite Anschlussformation (78) zur fluidübertragenden Verbindung einer zweiten Fluidleitung (88) mit dem Gehäuse (68),
eine von den ersten beiden gesondert ausgebildete dritte Anschlussformation (80) zur fluidübertragenden Verbindung des Gehäuses (68) mit einer dritten Fluidleitung,
eine von den Anschlussformationen (76, 78, 80) gesondert ausgebildete Kopplungsformation (64, 66), welche zum betriebsmäßig herstellbaren und lösbaren fluidübertragenden Kopplungseingriff mit einer entsprechenden Gegenkopplungsformation (38, 40) eines Zellkulturbehälters (10; 10'; 10") ausgebildet ist,
einen ersten Fluidströmungspfad (87), welcher zwischen dem Strömungsraum (72) und der ersten Anschlussformation (76) zur Einleitung eines ersten Fluids von außen in den Strömungsraum (72) verläuft,
einen zweiten Fluidströmungspfad (90), welcher zwischen dem Strömungsraum (72) und der zweiten Anschlussformation (78) zur Einleitung eines vom ersten verschiedenen zweiten Fluids von außen in den Strömungsraum (72) verläuft,
einen dritten Fluidströmungspfad (92), welcher zur Ausleitung eines Fluids aus dem Strömungsraum (72) zwischen dem Strömungsraum (72) und der dritten Anschlussformation (80) verläuft, und
einen Kopplungsströmungspfad (44a, 47a), welcher zwischen dem Strömungsraum (72) und der Kopplungsformation (64, 66) verläuft, um über die Kopplungsformation (64, 66) ein Fluid aus dem Strömungsraum (72) auszuleiten oder/und in diesen einzuleiten,
wobei der erste (82), der zweite (90) und der dritte Fluidströmungspfad (92) jeweils eine Ventilanordnung (86) aufweisen, welche ohne eine von der Ventilanordnung (86) bis zur Außenseite des Gehäuses (68) kontinuierlich durchgehende signal- oder/und energieübertragende körperliche Verbindung vollständig - mit Ausnahme des jeweiligen Fluidströmungspfads - von dem Gehäuse (68) umgeben in diesem aufgenommen ist,
wobei jeder Ventilanordnung (86) eine Steueranordnung (94) mit einem ein elektrisches oder/und magnetisches oder/und elektromagnetisches Feld erzeugenden Signalmittel (100) zugeordnet ist, dessen Feld auf ein entsprechend feldsensitives Gegensignalmittel (56) der Ventilanordnung (86) berührungslos einwirkt, wobei jede Ventilanordnung (86) mittels des auf ihr Gegensignalmittel (56) einwirkenden Feldes zwischen einer Sperrstellung, in der die Ventilanordnung (86) eine Fluidströmung in dem Fluidströmungspfad (82, 90, 92), in dem sie angeordnet ist, unterbricht, und einer Durchlassstellung, in der die Ventilanordnung (86) eine Fluidströmung gestattet, schaltbar ist.

2. Fluid-Versorgungsschnittstelle nach Anspruch 1,
**dadurch gekennzeichnet, dass** die Fluid-Versorgungsschnittstelle (62'; 62"; 62'") weiter umfasst:
eine von den übrigen dreien gesondert ausgebildete vierte Anschlussformation (110) zur fluidübertragenden Verbindung des Gehäuses (68) mit einer vierten Fluidleitung und
einen vierten Fluidströmungspfad (112), welcher zur Ausleitung eines Fluids aus dem Strömungsraum (72) zwischen dem Strömungsraum (72) und der vierten Anschlussformation (110) verläuft.

3. Fluid-Versorgungsschnittstelle nach Anspruch 1 oder 2,
**dadurch gekennzeichnet, dass** die Kopplungsformation (64) eine erste Kopplungsformation (64) ist, dass die Fluid-Versorgungsschnittstelle (62; 62'; 62") eine von der ersten (64) gesondert ausgebildete zweite Kopplungsformation (66) aufweist, welche zum betriebsmäßig herstellbaren und lösbaren fluidübertragenden Kopplungseingriff mit einer entsprechenden zweiten Gegenkopplungsformation (40) des Zellkulturbehälters (10; 10') ausgebildet ist, und dass die Fluid-Versorgungsschnittstelle (62; 62'; 62") einen zweiten Kopplungsströmungspfad (47a) aufweist, welcher zwischen dem Strömungsraum (72) und der zweiten Kopplungsformation (66) verläuft, um über die zweite Kopplungsformation (66) Fluid aus dem Strömungsraum (72) auszuleiten oder/und in diesen einzuleiten.

4. Fluid-Versorgungsschnittstelle nach Anspruch 3,
**dadurch gekennzeichnet, dass** die Fluid-Versorgungsschnittstelle (62; 62'; 62") einen zwischen der ersten (64) und der zweiten Kopplungsformation (66) verlaufenden Verbindungsströmungspfad (75) aufweist und die Fluid-Versorgungsschnittstelle (62; 62'; 62") in diesem eine Trenn-Ventilanordnung (74) aufweist, welche ohne eine von der Trenn-Ventilanordnung (74) bis zur Außenseite des Gehäuses (68) kontinuierlich durchgehende signal- oder/und energieübertragende körperliche Verbindung vollständig - mit Ausnahme des Verbindungsströmungspfads (75) - von dem Gehäuse (68) umgeben in diesem aufgenommen ist,
wobei der Trenn-Ventilanordnung (74) eine Steueranordnung (94) mit einem ein elektrisches oder/und magnetisches oder/und elektromagnetisches Feld erzeugenden Signalmittel (100) zugeordnet ist, dessen Feld auf ein entsprechend feldsensitives Gegensignalmittel (56) der Trenn-Ventilanordnung (74) berührungslos einwirkt, wobei die Trenn-Ventilanordnung (74) mittels des auf ihr Gegensignalmittel (56) einwirkenden Feldes zwischen einer Sperrstellung, in der die Trenn-Ventilanordnung (74) eine Fluidströmung in dem Verbindungsströmungspfad (75) unterbricht, und einer Durchlassstellung, in der die Trenn-Ventilanordnung (74) eine Fluidströmung gestattet, schaltbar ist.

5. Fluid-Versorgungsschnittstelle nach Anspruch 4,
**dadurch gekennzeichnet, dass** der Verbindungsströmungspfad (75) der einzige zwischen der ersten (64) und der zweiten Kopplungsformation (66) verlaufende Fluidströmungspfad (75) ist.

6. Fluid-Versorgungsschnittstelle nach einem der Ansprüche 4 oder 5,
**dadurch gekennzeichnet, dass** die Trenn-Ventilanordnung (74) derart angeordnet ist, dass durch sie der erste (82) und der zweite Fluidströmungspfad (90) vom zweiten Kopplungsströmungspfad (47a), aber nicht vom ersten Kopplungsströmungspfad (44a) trennbar ist, und dass durch sie der dritte Fluidströmungspfad (92) vom ersten Kopplungsströmungspfad (44a), aber nicht vom zweiten Kopplungsströmungspfad (47a) trennbar ist.

7. Fluid-Versorgungsschnittstelle nach Anspruch 6, unter Einbeziehung des Anspruchs 2,
**dadurch gekennzeichnet, dass** die Trenn-Ventilanordnung (74) derart angeordnet ist, dass durch sie der vierte Fluidströmungspfad (110) vom ersten Kopplungsströmungspfad (44a), aber nicht vom zweiten Kopplungsströmungspfad (47a) trennbar ist.

8. Fluid-Versorgungsschnittstelle nach einem der vorhergehenden Ansprüche,
**dadurch gekennzeichnet, dass** die Fluid-Versorgungsschnittstelle (62") eine von der ersten (64) und der zweiten (66) gesondert ausgebildete dritte Kopplungsformation (66') und einen dritten Kopplungsströmungspfad (47a') aufweist, welcher zwischen dem Strömungsraum (72) und der dritten Kopplungsformation (66') verläuft, um über die dritte Kopplungsformation (66') Fluid aus dem Strömungsraum (72) auszuleiten oder/und in diesen einzuleiten, wobei die dritte Kopplungsformation (66') zum betriebsmäßig herstellbaren und lösbaren fluidübertragenden Kopplungeingriff mit einer entsprechenden dritten Gegenkopplungsformation (40') des Zellkulturbehälters (10') ausgebildet ist.

9. Fluid-Versorgungsschnittstelle nach Anspruch 8,
**dadurch gekennzeichnet, dass** der Verbindungsströmungspfad (75) ein erster Verbindungsströmungspfad (75) und die Trenn-Ventilanordnung (74) eine erste Trenn-Ventilanordnung (74) ist und dass die Fluid-Versorgungsschnittstelle (62") einen zwischen der zweiten (66) und der dritten Kopplungsformation (66') verlaufenden zweiten Verbindungsströmungspfad aufweist und die Fluid-Versorgungsschnittstelle (62") in diesem eine von der ersten gesonderte zweite Trenn-Ventilanordnung (74') aufweist, welche ohne eine von der zweiten Trenn-Ventilanordnung (74') bis zur Außenseite des Gehäuses (68) kontinuierlich durchgehende signal- oder/und energieübertragende körperliche Verbindung vollständig - mit Ausnahme des zweiten Verbindungsströmungspfads - von dem Gehäuse (68) umgeben in diesem aufgenommen ist, wobei der zweiten Trenn-Ventilanordnung (74') eine Steueranordnung (94) mit einem ein elektrisches oder/und magnetisches oder/und elektromagnetisches Feld erzeugenden Signalmittel (100) zugeordnet ist, dessen Feld auf ein entsprechend feldsensitives Gegensignalmittel (56) der zweiten Trenn-Ventilanordnung (74') berührungslos einwirkt, wobei die zweite Trenn-Ventilanordnung (74') mittels des auf ihr Gegensignalmittel (56) einwirkenden Feldes zwischen einer Sperrstellung, in der die zweite Trenn-Ventilanordnung (74') eine Fluidströmung in dem zweiten Verbindungsströmungspfad unterbricht, und einer Durchlassstellung, in der die zweite Trenn-Ventilanordnung (74') eine Fluidströmung gestattet, schaltbar ist.

10. Fluid-Versorgungsschnittstelle nach Anspruch 9,
**dadurch gekennzeichnet, dass** der zweite Verbindungsströmungspfad der einzige zwischen der zweiten (66) und der dritten Kopplungsformation (66') verlaufende Fluidströmungspfad ist.

11. Fluid-Versorgungsschnittstelle nach Anspruch 9 oder 10, unter Einbeziehung des Anspruchs 2,
**dadurch gekennzeichnet, dass** die zweite Trenn-Ventilanordnung (74') derart angeordnet ist, dass entweder
durch sie der dritte Fluidströmungspfad (92) vom zweiten Kopplungsströmungspfad (47a), aber nicht vom dritten Kopplungsströmungspfad (47a') trennbar ist, und dass durch sie der vierte Fluidströmungspfad (112) vom dritten Kopplungsströmungspfad (47a'), aber nicht vom zweiten Kopplungsströmungspfad (47a) trennbar ist,
oder dass
durch sie der vierte Fluidströmungspfad (112) vom zweiten Kopplungsströmungspfad, aber nicht vom dritten Kopplungsströmungspfad (47a') trennbar ist, und dass durch sie der dritte Fluidströmungspfad (92) vom dritten Kopplungsströmungspfad (47a'), aber nicht vom zweiten Kopplungsströmungspfad (47a) trennbar ist.

12. Fluid-Versorgungsschnittstelle nach einem der vorhergehenden Ansprüche,
**dadurch gekennzeichnet, dass** für wenigstens eine Ventilanordnung (54, 74, 86), vorzugsweise eine Mehrzahl von Ventilanordnungen (54, 74, 86), besonders bevorzugt für alle Ventilanordnungen (54, 74, 82) gilt:
- das Signalmittel (100) und das Gegensignalmittel (56) umfassen jeweils eine Elektrode zum Aufbau eines elektrischen Feldes zwischen diesen, wobei das Gegensignalmittel (56) mit einem piezoelektrischen Aktuator der Ventilanordnung (54, 74, 86) derart zusammenwirkt, dass das elektrische Feld eine Gestaltänderung des piezoelektrischen Aktuators bewirkt, die wiederum eine Verstellung der Ventilanordnung (54, 74, 86) zwischen Sperrstellung und Durchlassstellung bewirkt, oder/und
- das Signalmittel (100) und das Gegensignalmittel (56) umfassen einen Magneten (100) einerseits und ein auf dessen Magnetfeld ansprechendes ferromagnetisches oder/und magnetisiertes Bauteil (56) andererseits, wobei das zwischen Signalmittel (100) und Gegensignalmittel (56) wirkende Magnetfeld eine Verlagerung des Gegensignalmittels (56) bewirkt, die wiederum eine Verstellung der Ventilanordnung (54, 74, 86) zwischen Sperrstellung und Durchlassstellung bewirkt, oder/und
- das Signalmittel (100) umfasst einen Magneten und das Gegensignalmittel (56) umfasst ein auf dessen Magnetfeld induktiv ansprechendes elektrisch leitendes Bauteil, wobei das zwischen Signalmittel (100) und Gegensignalmittel (56) wirkende Magnetfeld eine Induktion im Gegensignalmittel (56) bewirkt, die wiederum eine Verstellung der Ventilanordnung (54, 74, 86) zwischen Sperrstellung und Durchlassstellung bewirkt, oder/und
- das Signalmittel (100) umfasst einen Sender elektromagnetischer Wellen, wie etwa optischer Signale oder Funksignale, und das Gegensignalmittel (56) umfasst einen entsprechenden Empfänger, wobei die Ventilanordnung (54, 74, 86) einen Energiespeicher und einen Aktuator aufweist, welche derart untereinander und mit dem Gegensignalmittel (56) gekoppelt sind, dass das Gegensignalmittel (56) abhängig von den empfangenen elektromagnetischen Wellen den aus dem Energiespeicher gespeisten Aktuator zur Schaltung der Ventilanordnung (54, 74, 86) zwischen der Sperrstellung und der Durchlassstellung steuert.

13. Fluid-Versorgungsschnittstelle nach Anspruch 12,
**dadurch gekennzeichnet, dass** das Signalmittel (100) und das Gegensignalmittel (56) einen Magneten (100) einerseits und ein auf dessen Magnetfeld ansprechendes ferromagnetisches oder/und magnetisiertes Bauteil (56) andererseits umfassen, wobei das Gegensignalmittel (56) ein Ventilkörper (56) der Ventilanordnung (54, 74, 86) ist, der unter Einwirkung des zwischen Signalmittel (100) und Gegensignalmittel (56) wirkenden Magnetfelds von seinem Ventilsitz (58) weg oder/und zur dichtenden Anlage an diesen verlagerbar ist.

14. Fluid-Versorgungsschnittstelle nach Anspruch 13,
**dadurch gekennzeichnet, dass** die Ventilanordnung (54, 74, 86) magnetisch in die Sperrstellung vorgespannt ist und durch das vom Signalmittel (100) ausgehende Magnetfeld in die Durchlassstellung verstellbar ist.

15. Fluid-Versorgungsschnittstelle nach Anspruch 14,
**dadurch gekennzeichnet, dass** ein Ventilsitz (58) der Ventilanordnung (54, 74, 86) einen permanentmagnetisches oder ein ferromagnetisches Spannbauteil (60) aufweist, so dass zwischen dem Spannbauteil (60) und dem Ventilkörper (56) eine magnetische Spannkraft, insbesondere Anziehungskraft, wirkt, welche den Ventilkörper (56) zur dichtenden Anlage an den Ventilsitz (58) spannt.

16. Fluid-Versorgungsschnittstelle nach Anspruch 15,
**dadurch gekennzeichnet, dass** der Ventilsitz (58) ein elastomeres Anlagebauteil (59) aufweist, an welchem der Ventilkörper (56) in der Sperrstellung der Ventilanordnung (54, 74, 86) unmittelbar anliegt, wobei bevorzugt das Anlagebauteil (59) zwischen dem Ventilkörper (56) und dem Spannbauteil (60) angeordnet ist, wobei dann die zwischen Ventilkörper (56) und Spannbauteil (60) wirkende magnetische Spannkraft eine Anziehungskraft ist.

17. Fluid-Versorgungsschnittstelle nach einem der vorhergehenden Ansprüche,
**dadurch gekennzeichnet, dass** das Signalmittel (100) einen örtlich verlagerbaren Permanentmagneten (100) oder einen Elektromagneten umfasst.

18. Fluid-Versorgungsschnittstelle nach Anspruch 17,
**dadurch gekennzeichnet, dass** die Steueranordnung (94) eine Mehrzahl von Sätzen (102) von Signalmitteln (100) aufweist, wobei die Signalmittel (100) eines Satzes (102) jeweils eine Ventilstellungskonfiguration von Ventilanordnungen (54, 74, 86) der Fluid-Versorgungsschnittstelle (62; 62'; 62"; 62'") definieren.

19. Fluid-Versorgungsschnittstelle nach Anspruch 18,
**dadurch gekennzeichnet, dass** die Steueranordnung (94) eine um eine Walzenachse (W) drehbare Walze (96) aufweist, wobei die Mehrzahl von Signalmittelsätzen (102) in Umfangsrichtung um die Walzenachse (W) derart verteilt angeordnet sind, dass durch Verdrehung der Walze (96) unterschiedliche Ventilstellungskonfigurationen der Fluid-Versorgungsschnittstelle (62; 62'; 62"; 62'") einstellbar sind.

20. Fluid-Versorgungsschnittstelle nach einem der Ansprüche 17 bis 19,
**dadurch gekennzeichnet, dass** die Steueranordnung (94) eine Vermittlungsanordnung (104) aufweist, welche zwischen einem Signalmittel (100) und einer durch das Signalmittel (100) verstellbaren Ventilanordnung (54, 74, 86) angeordnet ist, wobei die Vermittlungsanordnung (104) wenigstens einen zwischen einer der Ventilanordnung (54, 74, 86) näher gelegenen Aktivstellung und einer dem Signalmittel (100) näher gelegenen Inaktivstellung verlagerbaren Magneten (106), insbesondere Permanentmagneten (106) aufweist.

21. Fluid-Versorgungsschnittstelle nach Anspruch 20, unter Einbeziehung des Anspruchs 18 oder 19,
**dadurch gekennzeichnet, dass** die Vermittlungsanordnung (104) für jedes Signalmittel (100) eines Satzes (102) von Signalmitteln (100) einen verlagerbaren Magneten (106) aufweist.

22. Fluid-Versorgungsschnittstelle nach Anspruch 20 oder 21,
**dadurch gekennzeichnet, dass** der wenigstens eine verlagerbare Magnet (106) in eine seiner Stellungen, vorzugsweise in seine Inaktivstellung, vorgespannt ist, vorzugsweise durch Schwerkraft.

23. Fluid-Versorgungsschnittstelle nach einem der vorhergehenden Ansprüche,
**dadurch gekennzeichnet, dass** wenigstens ein Teil der Ventilanordnungen (54, 74, 86), vorzugsweise alle Ventilanordnungen (54, 74, 86), in einer Durchlassströmungsrichtung längs des Fluidströmungspfads (82, 90, 92, 75), in dem sie angeordnet sind, durch einen vorbestimmten Fluiddruckunterschied von der Sperrstellung in die Durchlassstellung verstellbar sind, in der entgegegesetzten Strömungsrichtung dagegen nicht, wobei bevorzugt die Durchlassströmungsrichtung des ersten und des zweiten Fluidströmungspfads (82, 90) in den Strömungsraum (72) hinein gerichtet ist und die Durchlassströmungsrichtung des dritten Fluidströmungspfads (92) aus dem Strömungsraum (72) hinaus gerichtet ist.

24. Fluid-Versorgungsschnittstelle nach einem der vorhergehenden Ansprüche,
**dadurch gekennzeichnet, dass** der Kopplungsströmungspfad (44a, 47a, 47a') oder die Kopplungsströmungspfade (44a, 47a, 47a') bei gelöstem Kopplungseingriff frei von sie unterbrechenden oder zum Durchlass freigebenden Ventilanordnungen sind.

25. Zellkulturanlage, umfassend wenigstens einen Zellkulturbehälter (10; 10'; 10") zur Aufnahme und Versorgung adhärenter Zellen darin, ein Nährmediumreservoir (116), ein Reinigungsfluidreservoir (114) sowie eine Fluid-Versorgungsschnittstelle (62; 62'; 62"; 62'") nach einem der vorhergehenden Ansprüche mit der Maßgabe, dass:
- die erste Anschlussformation (76) das Gehäuse (68) mit dem Nährmediumreservoir (116) fluidübertragend verbindet und somit der erste Fluidströmungspfad (82) zwischen dem Strömungsraum (72) und dem dem Nährmediumreservoir (116) verläuft,
- die zweite Anschlussformation (78) das Gehäuse (68) mit dem Reinigungsfluidreservoir (114) fluidübertragend verbindet und somit der zweite Fluidströmungspfad (90) zwischen dem Strömungsraum (72) und dem Reinigungsfluidreservoir (114) verläuft,
- die dritte Anschlussformation (80) das Gehäuse (68) mit einem Abfluss (118) fluidübertragend verbindet und somit der dritte Fluidströmungspfad (92) zwischen dem Strömungsraum (72) und dem Abfluss (118) verläuft,
- die Kopplungsformation (64, 66, 66') zum betriebsmäßig herstellbaren und lösbaren fluidübertragenden Kopplungseingriff mit einer Gegenkopplungsformation (38, 40, 40') des Zellkulturbehälters (10; 10'; 10") ausgebildet ist,
- das erste Fluid das Nährmedium ist,
- das zweite Fluid das Reinigungsfluid ist,
- der Kopplungsströmungspfad (44a, 47a, 47a') dazu ausgebildet ist, über die Kopplungsformation (64, 66, 66') in einem mit der Gegenkopplungsformation (38, 40, 40') gekoppelten Zustand Nährmedium aus dem Strömungsraum (72) in den Zellkulturbehälter (10; 10'; 10") einzuleiten oder/und aus diesem in den Strömungsraum (72) auszuleiten.

26. Zellkulturanlage nach Anspruch 25,
**dadurch gekennzeichnet, dass** sie eine Fluid-Versorgungsschnittstelle (62', 62"; 62'") gemäß Anspruch 2 oder gemäß einem der Ansprüche 12 bis 24 unter Einbeziehung des Anspruchs 2 mit der Maßgabe umfasst, dass:
- die vierte Anschlussformation (110) das Gehäuse (68) mit einer Sample-Aufnahmevorrichtung (120) fluidübertragend verbindet und somit der vierte Fluidströmungspfad (112) zwischen dem Strömungsraum (72) und der Sample-Aufnahmevorrichtung (120) verläuft.

27. Zellkulturanlage nach Anspruch 25 oder 26,
**dadurch gekennzeichnet, dass** im Strömungsraum (72) ein Strömungsweg von der Ventilanordnung (86) der zweiten Anschlussformation (78) zur Ventilanordnung (86) der dritten Anschlussformation (80) der längste Strömungsweg zwischen zwei Ventilanordnungen (74, 86) oder zwischen einer Ventilanordnung (74, 86) und einer Kopplungsformation (64, 66, 66') ist.

28. Zellkulturanlage nach einem der Ansprüche 25 bis 27,
**dadurch gekennzeichnet, dass** im Strömungsraum (72) ein Strömungsweg von der Ventilanordnung (86) der zweiten Anschlussformation (78) zur Ventilanordnung (86) der dritten Anschlussformation (80) an der Ventilanordnung (82) der ersten Anschlussformation (76) und gegebenenfalls an der Ventilanordnung (86) der vierten Anschlussformation (110) vorbei verläuft.

29. Zellkulturanlage nach Anspruch 28,
**dadurch gekennzeichnet, dass** der Ventilkörper (56) der Ventilanordnung (86) der ersten Anschlussformation (76) und gegebenenfalls der Ventilkörper (56) der Ventilanordnung (86) der vierten Anschlussformation (110) wenigstens zum Teil, vorzugsweise wenigstens mehr als zur Hälfte in den Strömungsweg von der Ventilanordnung (82) der zweiten Anschlussformation (78) zur Ventilanordnung (86) der dritten Anschlussformation (80) hineinragen.

30. Zellkulturanlage nach einem der Ansprüche 25 bis 29,
**dadurch gekennzeichnet, dass** die Gegenkopplungsformation (38) des Zellkulturbehälters (10; 10') eine erste Gegenkopplungsformation (38) ist, dass der Zellkulturbehälter (10; 10') eine von der ersten Gegenkopplungsformation (38) gesondert ausgebildete zweite Gegenkopplungsformation (40) aufweist, und dass die Zellkulturanlage weiter eine Fluid-Versorgungsschnittstelle (62; 62'; 62") gemäß einem der Ansprüche 3 bis 7 oder 12 bis 24 unter Einbeziehung des Anspruchs 3 aufweist, mit der Maßgabe, dass die erste Kopplungsformation (64) zum betriebsmäßig herstellbaren und lösbaren fluidübertragenden Kopplungseingriff mit der ersten Gegenkopplungsformation (38) und die zweite Kopplungsformation (86) zum betriebsmäßig herstellbaren und lösbaren fluidübertragenden Kopplungseingriff mit der zweiten Gegenkopplungsformation (40) des Zellkulturbehälters (10; 10') ausgebildet ist sowie dass der zweite Kopplungsströmungspfad (47a) zwischen dem Strömungsraum (72) und der zweiten Kopplungsformation (66) verläuft, um über die zweite Kopplungsformation (66) bei mit der zweiten Gegenkopplungsformation (40) hergestelltem Kopplungseingriff Nährmedium aus dem Strömungsraum (72) in den Zellkulturbehälter (10; 10') einzuleiten oder/und aus diesem in den Strömungsraum (72) auszuleiten.

31. Zellkulturanlage nach Anspruch 30,
**dadurch gekennzeichnet, dass** der Zellkulturbehälter (10') eine von der ersten (36) und der zweiten (40) gesondert ausgebildete dritte Gegenkopplungsformation (40') aufweist, und dass die Zellkulturanlage eine Fluid-Versorgungsschnittstelle (62") gemäß einem der Ansprüche 8 bis 24 unter Einbeziehung des Anspruchs 8 aufweist, mit der Maßgabe, dass die dritte Kopplungsformation (66') zum betriebsmäßig herstellbaren und lösbaren fluidübertragenden Kopplungseingriff mit der dritten Gegenkopplungsformation (40') des Zellkulturbehälters (10') ausgebildet ist sowie dass der dritte Kopplungsströmungspfad (47a') zwischen dem Strömungsraum (72) und der dritten Kopplungsformation (66') verläuft, um über die dritte Kopplungsformation (66') bei mit der dritten Gegenkopplungsformation (40') hergestelltem Kopplungseingriff Nährmedium aus dem Strömungsraum (72) in den Zellkulturbehälter (10') einzuleiten oder/und aus diesem in den Strömungsraum (72) auszuleiten.

32. Zellkulturanlage nach einem der Ansprüche 25 bis 31,
**dadurch gekennzeichnet, dass** wenigstens eine Gegenkopplungsformation (38, 40, 40'), vorzugsweise jede Gegenkopplungsformation (38, 40, 40'), des Zellkulturbehälters (10; 10'; 10") je eine Behälter-Ventilanordnung (54) aufweist.

33. Zellkulturanlage nach Anspruch 32,
**dadurch gekennzeichnet, dass** die Behälter-Ventilanordnung (54) bei hergestelltem Kopplungseingriff zwischen Kopplungsformation und Gegenkopplungsformation ohne eine von der Behälter-Ventilanordnung (54) bis zur Außenseite der Gegenkopplungsformation (38, 40, 40') und des Gehäuses (68) der Fluid-Versorgungsschnittstelle (62; 62'; 62"; 62'") kontinuierlich durchgehende signal- oder/und energieübertragende körperliche Verbindung vollständig - mit Ausnahme des die Kopplungsformation (64, 66, 66') und die Gegenkopplungsformation (38, 40, 40') durchsetzenden Fluidströmungspfads - von der Gegenkopplungsformation und des Gehäuses der Fluid-Versorgungsschnittstelle umgeben ist.

34. Zellkulturanlage nach Anspruch 32 oder 33,
**dadurch gekennzeichnet, dass** die wenigstens eine Behälter-Ventilanordnung (54) durch die Steueranordnung (94) zwischen einer Sperrstellung und einer Durchlassstellung schaltbar ist.

35. Zellkulturanlage nach einem der Ansprüche 32 bis 34,
**dadurch gekennzeichnet, dass** im Strömungsraum (72) ein Strömungsweg von der Ventilanordnung (86) der zweiten Anschlussformation (78) zur Ventilanordnung (86) der dritten Anschlussformation (80) - bei hergestelltem Kopplungseingriff zwischen der wenigstens einen Kopplungsformation (64, 66, 66') der Fluid-Versorgungsschnittstelle (62; 62'; 62"; 62'") und der wenigstens einen Gegenkopplungsformation (38, 40, 40') eines Zellkulturbehälters (10; 10'; 10") - an der wenigstens einen Behälter-Ventilanordnung (54), vorzugsweise an allen Behälter-Ventilanordnungen (54), vorbei verläuft.

36. Zellkulturanlage nach Anspruch 35,
**dadurch gekennzeichnet, dass** - bei hergestelltem Kopplungseingriff zwischen der wenigstens einen Kopplungsformation (64, 66, 66') der Fluid-Versorgungsschnittstelle (62; 62'; 62"; 62'") und der wenigstens einen Gegenkopplungsformation (38, 40; 40') eines Zellkulturbehälters (10; 10'; 10") - der Ventilkörper (56) der wenigstens einen Behälter-Ventilanordnung (54), bevorzugt jeder Ventilkörper (56) aller Behälter-Ventilanordnungen (54), wenigstens zum Teil, vorzugsweise wenigstens mehr als zur Hälfte, in den Strömungsweg von der Ventilanordnung (86) der zweiten Anschlussformation (78) zur Ventilanordnung (86) der dritten Anschlussformation (80) hineinragt.

37. Zellkulturanlage nach einem der Ansprüche 25 bis 36,
**dadurch gekennzeichnet, dass** alle in der Fluid-Versorgungsschnittstelle (62; 62'; 62"; 62'") vorgesehenen Ventilanordnungen (74, 86), vorzugsweise auch alle in dem wenigstens einen Zellkulturbehälter (10; 10'; 10") vorgesehenen Ventilanordnungen (54), im Wesentlichen identisch aufgebaut sind.

38. Zellkulturanlage nach einem der Ansprüche 25 bis 37,
**dadurch gekennzeichnet, dass** alle in dem wenigstens einen Zellkulturbehälter (10; 10'; 10") und in der Fluid-Versorgungsschnittstelle (62; 62'; 62"; 62'") vorgesehenen Ventilanordnungen (54, 74, 86) bei hergestelltem Kopplungseingriff durch eine gemeinsame Steueranordnung (94) mit einer Mehrzahl von Signalmitteln (100) schaltbar sind.

39. Zellkulturanlage nach einem der Ansprüche 25 bis 38, unter Einbeziehung des Anspruchs 16,
**dadurch gekennzeichnet, dass** in wenigstens einer Anschlussformation (76, 78, 80), vorzugsweise in einer Mehrzahl von Anschlussformationen (76, 78, 80), eine ringförmige axiale Endfläche (59b) des elastomeren Anlagebauteils (59) als Dichtfläche den der Anschlussformation (76, 78, 80) zugeordneten Fluidströmungspfad (82, 90, 92) radial außen umgebend verformt an einer Gegendichtfläche (64a) anliegt.

40. Zellkulturanlage nach einem der Ansprüche 25 bis 39, unter Einbeziehung des Anspruchs 16,
**dadurch gekennzeichnet, dass** die Behälter-Ventilanordnung (54) gemäß Anspruch 16 ausgebildet ist, wobei - bei hergestelltem Kopplungseingriff zwischen der wenigstens einen Kopplungsformation (64, 66, 66') der Fluid-Versorgungsschnittstelle (62; 62'; 62"; 62'") und der wenigstens einen Gegenkopplungsformation (38, 40, 40') eines Zellkulturbehälters (10; 10'; 10") - eine ringförmige axiale Endfläche (59b) des elastomeren Anlagebauteils (59) der Behälter-Ventilanordnung (54) als Dichtfläche den der Kopplungsformation (64, 66, 66') zugeordneten Fluidströmungspfad (44a, 47a, 47a') radial außen umgebend verformt an einer Gegendichtfläche (64a) der Fluid-Versorgungsschnittstelle (62; 62'; 62"; 62'") anliegt.

41. Zellkulturanlage nach einem der Ansprüche 25 bis 40,
**dadurch gekennzeichnet, dass** sie mehr Zellkulturbehälter (10; 10'; 10") als Fluid-Versorgungsschnittstellen (62; 62'; 62"; 62'") aufweist, wobei insbesondere die Zellkulturanlage nicht mehr als 5, bevorzugt nicht mehr als 3, besonders bevorzugt genau eine Fluid-Versorgungsschnittstelle (62; 62'; 62"; 62"') aufweist.

42. Zellkulturanlage nach Anspruch 41,
**dadurch gekennzeichnet, dass** die Zellkulturanlage eine Bewegungsvorrichtung, etwa einen Mehrachsenroboter oder einen in einer Bewegungsebene beweglichen Kreuztisch mit daran zur orthogonal Bewegungsebene beweglich vorgesehenem Bewegungsschlitten, aufweist, mit welcher die Fluid-Versorgungsschnittstelle (62; 62'; 62"; 62'") nacheinander in fluidübertragenden Kopplungseingriff mit unterschiedlichen Zellkulturbehältern (10; 10'; 10") bringbar ist.

43. Zellkulturbehälter für eine Zellkulturanlage nach einem der Ansprüche 25 bis 42 sowie zum herstellbaren und lösbaren Kopplungseingriff mit einer Fluid-Versorgungsschnittstelle (62; 62'; 62"; 62'") nach einem der Ansprüche 1 bis 24, wobei der Zellkulturbehälter (10; 10'; 10") einen ein Kulturvolumen (14) einschließenden Behälterkörper (12) mit einer Einfüll- oder/und Belüftungsöffnung (30) aufweist, durch welche hindurch Gas, Flüssigkeit, Paste oder/und Festkörper in den Behälterkörper (12) einfüllbar und aus diesem entnehmbar sind,
**dadurch gekennzeichnet, dass** der Zellkulturbehälter (10; 10'; 10") zusätzlich wenigstens eine von der Einfüll- oder/und Belüftungsöffnung (30) gesondert ausgebildete Gegenkopplungsformation (38, 40, 40') aufweist, welche zur Herstellung und Lösung eines Kopplungseingriffs mit einer entsprechenden Kopplungsformation (64, 66, 66') der Fluid-Versorgungsschnittstelle (62; 62'; 62"; 62'") ausgebildet ist, wobei ein Liefer-Fluidströmungspfad (44, 47) zwischen der wenigstens einen Gegenkopplungsformation (38, 40, 40') und dem Kulturvolumen (14) verläuft, um über den Liefer-Fluidströmungspfad (44, 47) ein Fluid in das Kulturvolumen (14) einzuleiten oder/und aus diesem auszuleiten, wobei die wenigstens eine Gegenkopplungsformation (38, 40, 40') eine Behälter-Ventilanordnung (54) aufweist,
wobei vorzugsweise die Behälter-Ventilanordnung (54) durch eine Steueranordnung (94) mit einem ein elektrisches oder/und magnetisches oder/und elektromagnetisches Feld erzeugenden Signalmittel (100), dessen Feld auf ein entsprechend feldsensitives Gegensignalmittel (56) der Behälter-Ventilanordnung (54) berührungslos einwirkt, zwischen einer Sperrstellung, in der die Behälter-Ventilanordnung (54) eine Fluidströmung in dem Liefer-Fluidströmungspfad (44, 47) unterbricht, und einer Durchlassstellung, in der die Behälter-Ventilanordnung (54) eine Fluidströmung gestattet, schaltbar ist.

44. Zellkulturbehälter nach Anspruch 43,
**dadurch gekennzeichnet, dass** die Einfüll- oder/und Belüftungsöffnung (30) und die wenigstens eine gesondert ausgebildete Gegenkopplungsformation (38, 40, 40') an entgegengesetzten Enden des Zellkulturbehälters (10; 10'; 10") vorgesehen sind.

45. Zellkulturbehälter nach Anspruch 43 oder 44,
**dadurch gekennzeichnet, dass** der Zellkulturbehälter (10; 10'; 10") abgesehen von der wenigstens einen Behälter-Ventilanordnung (54) der Gegenkopplungsformation (38, 40, 40') ein passiver Behälter ohne im Behälter eingebaute, durch Energiezufuhr betreibbare Funktionseinheiten, wie etwa Heizeinrichtung, Rühreinrichtung und dergleichen, ist, besonders bevorzugt ein passiver Einweg-Wegwerf-Zellkulturbehälter (10; 10'; 10") ist.

46. Zellkulturbehälter nach einem der Ansprüche 43 bis 45,
**dadurch gekennzeichnet, dass** der Zellkulturbehälter (10; 10'; 10") zwei im Wesentlichen parallele Stirnwände (16) und diese verbindende, um einen Stirnwandrand umlaufenden Mantelwandabschnitte (18, 20, 22, 24, 26, 28) aufweist, welche gemeinsam das Kulturvolumen (14) begrenzen, wobei bevorzugt die wenigstens eine Gegenkopplungsformation (38, 40, 40'), besonders bevorzugt auch die Einfüll- oder/und Belüftungsöffnung (30) in einem Mantelwandabschnitt (22, 24) vorgesehen sind.

## Claims

1. A liquid supply interface (62; 62'; 62"; 62"') for a cell culturing system for supplying cell cultures found in different cell culture containers (10; 10'; 10") with a nutrient medium, wherein the liquid supply interface (62; 62'; 62"; 62'") comprises:
a housing (68) defining a flow area (72),
a first connection formation (76) for the liquid-transferring connection of a first fluid line (84) to the housing (68),
a second connection formation (78) formed separately from the first for the liquid-transferring connection of a second fluid line (88) to the housing,
a third connection formation (80) formed separately from the first two for the liquid-transferring connection of the housing (68) to a third fluid line,
a coupling formation (64, 66) formed separately from the connection formations (76, 78, 80), which is formed for the producible and detachable liquid-transferring coupling contact according to the operation, with a corresponding counter-coupling formation (38, 40) of a cell culture container (10, 10', 10"),
a first liquid flow path (87), which extends between the flow area (72) and the first connection formation (76) for introducing a first liquid from the outside into the flow area (72),
a second liquid flow path (90), which extends between the flow area (72) and the second connection formation (78) for introducing a second liquid different from the first from the outside into the flow area (72),
a third liquid flow path (92), which extends between the flow area (72) and the third connection formation (80) for removing a liquid from the flow area (72), and
a coupling flow path (44a, 47a), which extends between the flow area (72) and the coupling formation (64, 66) in order to remove a liquid from the flow area (72) and/or to introduce it to said flow area via the coupling formation (64, 66),
wherein the first (82), the second (90) and the third liquid flow path (92) each have a valve configuration (86), which is completely surrounded - with the exception of the respective liquid flow path - by the housing (68) and incorporated in it, without a continuous signal and/or power-transferring physical connection from the valve configuration (86) up to the outside of the housing (68),
wherein a control configuration (94) with a signaling means (100) generating an electric and/or magnetic and/or electromagnetic field is assigned to each valve configuration (86), the field of which acts upon a correspondingly field sensitive counter-signaling means (56) of the valve configuration (86) without contact, wherein each valve configuration (86) can be switched via the field, acting upon its counter-signaling means (56), between a blocked position, in which the valve configuration (86) interrupts a liquid flow in the liquid flow path (82, 90, 92) in which it is arranged, and an outlet position in which the valve configuration (86) enables a liquid flow.

2. The liquid supply interface according to claim 1,
**characterized in that** the liquid supply interface (62; 62'; 62"; 62"') further comprises:
a fourth connection formation (110) formed separately from the other three for the liquid-transferring connection of the housing (68) to a fourth fluid line and
a fourth liquid flow path (112), which extends between the flow area (72) and the fourth connection formation (110) for removing a liquid from the flow area (72).

3. The liquid supply interface according to claim 1 or 2,
**characterized in that** the coupling formation (64) is a first coupling formation (64), that the liquid supply interface (62; 62'; 62"; 62'") comprises a second coupling formation (66) formed separately from the first (64), which is formed for the producible and detachable liquid-transferring coupling contact according to the operation, with a corresponding second counter-coupling formation (40) of the cell culture container (10; 10'), and that the liquid supply interface (62; 62'; 62") comprises a second coupling flow path (47a), which extends between the flow area (72) and the second coupling formation (66) in order to remove a liquid from the flow area (72) and/or to introduce it to said flow area via the second coupling formation (66).

4. The liquid supply interface according to claim 3,
**characterized in that** the liquid supply interface (62; 62'; 62") has a connection flow path (75) extending between the first (64) and the second coupling formation (66) and the liquid supply interface (62; 62'; 62") has a separating valve configuration (74) in it which is completely surrounded - with the exception of the connection flow path (75) - by the housing (68) and incorporated in it, without a continuous signal and/or power-transferring physical connection from the separating valve configuration (74) up to the outside of the housing (68), wherein a control configuration (94) with a signaling means (100) generating an electric and/or magnetic and/or electromagnetic field is assigned to the separating valve configuration (74), the field of which acts upon a correspondingly field sensitive counter-signaling means (56) of the separating valve configuration (74) without contact, wherein the separating valve configuration (74) can be switched via the field, acting upon its counter-signaling means (56), between a blocked position, in which the separating valve configuration (74) interrupts a liquid flow in the connection flow path (75), and an outlet position in which the separating valve configuration (74) enables a liquid flow.

5. The liquid supply interface according to claim 4,
**characterized in that** the connection flow path (75) is the only liquid flow path (75) extending between the first (64) and the second coupling formation (66).

6. The liquid supply interface according to any one of claims 4 or 5,
**characterized in that** the separating valve configuration (74) is arranged such that by it the first (82) and the second liquid flow path (90) can be separated from the second coupling flow path (47a) but not from the first coupling flow path (44a), and that by it the third liquid flow path (92) can be separated from the first coupling flow path (44a) but not from the second coupling flow path (47a).

7. The liquid supply interface according to claim 6 together with claim 2,
**characterized in that** the separating valve configuration (74) is arranged such that by it the fourth liquid flow path (110) can be separated from the first coupling flow path (44a) but not from the second coupling flow path (47a).

8. The liquid supply interface according to any one of the preceding claims,
**characterized in that** the liquid supply interface (62") has a third coupling formation (66') formed separately from the first (64) and the second coupling formation (66) and a third coupling flow path (47a'), which extends between the flow area (72) and the third coupling formation (66') in order to remove liquid from the flow area (72) and/or to introduce it to said flow area via the third coupling formation (66'), wherein the third coupling formation (66') is formed for the producible and detachable liquid-transferring coupling contact according to the operation, with a corresponding third counter-coupling formation (40') of the cell culture container (10').

9. The liquid supply interface according to claim 8,
**characterized in that** the connection flow path (75) is a first connection flow path (75) and the separating valve configuration (74) is a first separating valve configuration (74), and that the liquid supply interface (62") has a second connection flow path extending between the second (66) and the third coupling formation (66'), and the liquid supply interface (62") in it has a second separating valve configuration (74') separate from the first one which is completely surrounded - with the exception of the second connection flow path - by the housing (68) and incorporated in it, without a continuous signal and/or power-transferring physical connection from the second separating valve configuration (74') up to the outside of the housing (68),
wherein a control configuration (94) with a signaling means (100) generating an electric and/or magnetic and/or electromagnetic field is assigned to the second separating valve configuration (74'), the field of which acts upon a correspondingly field sensitive counter-signaling means (56) of the second separating valve configuration (74') without contact, wherein the second separating valve configuration (74') can be switched via the field, acting upon its counter-signaling means (56), between a blocked position, in which the second separating valve configuration (74') interrupts a liquid flow in the second connection flow path, and an outlet position in which the second separating valve configuration (74') enables a liquid flow.

10. The liquid supply interface according to claim 9,
**characterized in that** the second connection flow path is the only liquid flow path extending between the second (66) and the third coupling formation (66').

11. The liquid supply interface according to claim 9 or 10 together with claim 2,
**characterized in that** the second separating valve configuration (74') is arranged such that either
by it the third liquid flow path (92) can be separated from the second coupling flow path (47a) but not from the third coupling flow path (47a'), and that by it the fourth liquid flow path (112) can be separated from the third coupling flow path (47a') but not from the second coupling flow path (47a),
or that
by it the fourth liquid flow path (112) can be separated from the second coupling flow path but not from the third coupling flow path (47a'), and that by it the third liquid flow path (92) can be separated from the third coupling flow path (47a') but not from the second coupling flow path (47a).

12. The liquid supply interface according to anyone of the preceding claims,
**characterized in that** to at least one valve configuration (54, 74, 86) preferably a plurality of valve configurations (54, 74, 86), more preferably to all valve configurations (54, 74, 82) the following applies:
- the signaling means (100) and the counter-signaling means (56) each comprise an electrode for establishing an electric field between each other, wherein the counter-signaling means (56) interacts with a piezoelectric actuator of the valve configuration (54, 74, 86) such that the electric field brings about a structural change to the piezoelectric actuator, which in turn brings about an adjustment in the valve configuration (54, 74, 86) between the blocked position and the outlet position; and/or
- the signaling means (100) and the counter-signaling means (56) comprise a magnet on the one hand and a ferromagnetic and/or magnetized component attracting its magnetic field on the other hand, wherein the magnetic field in effect between the signaling means and the counter-signaling means (56) causes a shift in the counter-signaling means (56), which in turn causes an adjustment in the valve configuration (54, 74, 86) between the blocked position and the outlet position; and/or
- the signaling means (100) comprises a magnet and the counter-signaling means (56) comprises an electrically conducting component inductively attracting its magnetic field, wherein the magnetic field effective between the signaling means (100) and the counter-signaling means (56) effects an induction in the counter-signaling means (56), which in turn causes an adjustment in the valve configuration (54, 74, 86) between the blocked position and the outlet position; and/or
- the signaling means (100) comprises a transmitter of electromagnetic waves, such as optical signals or radio signals, and the counter-signaling means (56) comprises a corresponding receiver, wherein the valve configuration (54, 74, 86) has a power storage unit and an actuator, which are coupled to one another and to the counter-signaling means (56) such that the counter-signaling means (56) controls the actuator fed from the power storage unit for switching the valve configuration (54, 74, 86) between the blocked position and the outlet position, depending on the electromagnetic waves received.

13. The liquid supply interface according to claim 12,
**characterized in that** the signaling means (100) and the counter-signaling means (56) comprise a magnet (100) on the one hand and a ferromagnetic and/or magnetized component (56) attracting its magnetic field, on the other hand, wherein the counter-signaling means (56) is a valve body (56) of the valve configuration (54, 74, 86), which can be shifted away from its valve seat (58) and/or towards said seat for forming a sealing contact to it under the effect of the magnetic field in effect between the signaling means (100) and the counter-signaling means (56).

14. The liquid supply interface according to claim 13,
**characterized in that** the valve configuration (54, 74, 86) is pretensioned magnetically in the blocked position and is adjustable into the outlet position by the magnetic field starting from the signaling means (100).

15. The liquid supply interface according to claim 14,
**characterized in that** a valve seat (58) of the valve configuration (54, 74, 86) has a permanent magnetic or ferromagnetic tension component such that a magnetic tension force, in particular an attracting force, is in effect between the tension component (60) and the valve body (56), which tensions the valve body (56) at the valve seat (58) for sealing contact.

16. The liquid supply interface according to claim 15,
**characterized in that** the valve seat (58) has an elastomer contact component (59), to which the valve body (56) abuts in the blocked position of the valve configuration (54, 74, 86), wherein preferably the contact component (59) is arranged between the valve body (56) and the tension component (60), wherein the magnetic tensioning force in effect between the valve body (56) and the tension component (60) is an attracting force.

17. The liquid supply interface according to anyone of the preceding claims,
**characterized in that** the signaling means (100) comprises a locally shiftable permanent magnet (100) or an electric magnet.

18. The liquid supply interface according to claim 17,
**characterized in that** the control configuration (94) comprises a plurality of sets (102) of signaling means (100), wherein the signaling means (100) of a set (102) each define a valve position configuration of valve configurations (54, 74, 86) of the liquid supply interface (62; 62'; 62"; 62'").

19. The liquid supply interface according to claim 18,
**characterized in that** the control configuration (94) comprises a roller (96) rotatable around a roller axis (W), wherein the plurality of sets of signaling means (102) are arranged distributed in circumferential direction around the roller axis (W) in such a way that by rotation of the roller (96) different valve position configurations of the liquid supply interface (62; 62'; 62"; 62'") can be adjusted.

20. The liquid supply interface according to anyone of claims 17 to 19,
**characterized in that** the control configuration (94) comprises a switching arrangement (104), which is arranged between a signaling means (100) and a valve configuration (54, 74, 86) adjustable by the signaling means (100), wherein the switching arrangement (104) comprises at least one shiftable magnet (106), in particular a permanent magnet (106), between an active position closer to the valve configuration (54, 74, 86) and an inactive position closer to the signaling means (100).

21. The liquid supply interface according to claim 20 together with claim 18 or 19,
**characterized in that** the switching arrangement (104) comprises for each signaling means (100) of a set (102) of signaling means (100) a shiftable magnet (106).

22. The liquid supply interface according to claim 20 or 21,
**characterized in that** the at least one shiftable magnet (106) is pretensioned in one of its positions, preferably in its inactive position, preferably by gravity.

23. The liquid supply interface according to any one of the preceding claims,
**characterized in that** at least part of the valve configurations (54, 74, 86), preferably all valve configurations (54, 74, 86), are adjustable in an outlet flow direction along the liquid flow path (82, 90, 92, 75) in which they are arranged, from the blocked position into the outlet position, via a predetermined liquid pressure difference, but not in the opposite flow direction, wherein preferably the outlet flow direction of the first and of the second liquid flow path (82, 90) is directed into the flow area (72), and the outlet flow direction of the third liquid flow path (92) is directed away from the flow area (72).

24. The liquid supply interface according to any one of the preceding claims,
**characterized in that** the coupling flow path (44a, 47a, 47a') or the coupling flow paths (44a, 47a, 47a') at detached coupling contact are free from valve configurations interrupting them or enabling their outlet.

25. A cell culturing system comprising at least a cell culture container (10; 10'; 10") for collecting and supplying adherent cells therein, a nutrient medium reservoir (116), a cleaning fluid reservoir (114) as well as a liquid supply interface (62; 62'; 62"; 62'") according to any one of the preceding claims, provided that:
- the first connection formation (76) connects the housing (68) with the nutrient medium reservoir (116) in a liquid-transferring manner and thus the first liquid flow path (82) extends between the flow area (72) and the nutrient medium reservoir (116),
- the second connection formation (78) connects the housing (68) with the cleaning fluid reservoir (114) in a liquid-transferring manner and thus the second liquid flow path (90) extends between the flow area (72) and the cleaning fluid reservoir (114),
- the third connection formation (80) connects the housing (68) with a discharge (118) in a liquid-transferring manner and thus the third liquid flow path (92) extends between the flow area (72) and the discharge (118),
- the coupling formation (64, 66, 66') for coupling contact, which is producible and detachable in a liquid-transferring manner according to the operation, is formed with a counter-coupling formation (38, 40, 40') of the cell culture container (10; 10';10"),
- the first liquid is the nutrient medium,
- the second liquid is the cleaning fluid,
- the coupling flow path (44a, 47a, 47a') is formed to remove nutrient medium from the flow area (72) and supply it to the cell culture container (10; 10'; 10") and/or to remove it from said container and introduce it to the flow area (72) via the coupling formation (64, 66, 66'), in a state coupled with the counter-coupling formation (38, 40, 40').

26. The cell culturing system according to claim 25,
**characterized in that** it comprises a liquid supply interface (62'; 62"; 62'") according to claim 2 or according to any one of claims 12 to 24 together with claim 2, provided that:
- the fourth connection formation (110) connects the housing (68) with a sample collecting device (120) in a liquid-transferring manner and thus the fourth liquid flow path (112) extends between the flow area (72) and the sample collecting device (120).

27. The cell culturing system according to claim 25 or 26,
**characterized in that** in the flow area (72) a flow path from the valve configuration (86) of the second connection formation (78) to the valve configuration (86) of the third connection formation (80) is the longest flow path between two valve configurations (74, 86) or between a valve configuration (74, 86) and a coupling formation (64, 66, 66').

28. The cell culturing system according to any one of claims 25 to 27,
**characterized in that** in the flow area (72) a flow path from the valve configuration (86) of the second connection formation (78) to the valve configuration (86) of the third connection formation (80) passes by the valve configuration (82) of the first connection formation (76) and, if applicable, by the valve configuration (86) of the fourth connection formation (110).

29. The cell culturing system according to claim 28,
**characterized in that** the valve body (56) of the valve configuration (86) of the first connection formation (76) and, if applicable, the valve body (56) of the valve configuration (86) of the fourth connection formation (110) protrudes at least partially, preferably at least more than half, into the flow path from the valve configuration (82) of the second connection formation (78) to the valve configuration (86) of the third connection formation (80).

30. The cell culturing system according to any one of claims 25 to 29,
**characterized in that** the counter-coupling formation (38) of the cell culture container (10; 10') is a first counter-coupling formation (38), that the cell culture container (10; 10') comprises a second counter-coupling formation (40) formed separately from the first counter-coupling formation (38), and that the cell culturing system further comprises a liquid supply interface (62; 62'; 62") according to any one of claims 3 to 7 or 12 to 24 together with claim 3, provided that the first coupling formation (64) is formed with the first counter-coupling formation (38) for the producible and detachable liquid-transferring coupling contact according to the operation, and the second coupling formation (86) is formed with the second counter-coupling formation (40) of the cell culture container (10; 10') for the producible and detachable liquid-transferring coupling contact according to the operation, and that the second coupling flow path (47a) extends between the flow area (72) and the second coupling formation (66), in order to remove nutrient medium from the flow area (72) and supply it to the cell culture container (10; 10') and/or to remove it from said container and introduce it to the flow area (72) via the second coupling formation (66) at coupling contact made with the second counter-coupling formation (40).

31. The cell culturing system according to claim 30,
**characterized in that** the cell culture container (10') comprises a third counter-coupling formation (40') formed separately from the first (36) and the second (40), and that the cell culturing system comprises a liquid supply interface (62") according to any one of claims 8 to 24 together with claim 8, provided that the third coupling formation (66') is formed for the producible and detachable liquid-transferring coupling contact according to the operation with the third counter-coupling formation (40') of the cell culture container (10'), and that the third coupling flow path (47a') extends between the flow area (72) and the third counter-coupling formation (66'), in order to remove nutrient medium from the flow area (72) and supply it to the cell culture container (10') and/or to remove it from said container and introduce it to the flow area (72) via the third coupling formation (66') at coupling contact made with the third counter-coupling formation (40').

32. The cell culturing system according to any one of claims 25 to 31,
**characterized in that** at least one counter-coupling formation (38, 40, 40'), preferably every counter-coupling formation (38, 40, 40') of the cell culture container (10; 10'; 10") comprises one container-valve configuration (54) each.

33. The cell culturing system according to claim 32,
**characterized in that** the container-valve configuration (54) at coupling contact made between coupling formation and counter-coupling formation without a continuous signal and/or power-transferring physical connection from the container-valve configuration (54) up to the outside of the counter-coupling formation (38, 40, 40') and the housing (68) of the liquid supply interface (62; 62'; 62"; 62"') - with the exception of the liquid flow path passing through the coupling formation (64, 66, 66') and the counter-coupling formation (38, 40, 40') - is completely surrounded by the counter-coupling formation and the housing of the liquid supply interface.

34. The cell culturing system according to claim 32 or 33,
**characterized in that** the at least one container-valve configuration (54) can be switched between a blocked position and an outlet position by the control configuration (94).

35. The cell culturing system according to according to any one of claims 32 to 34,
**characterized in that** a flow path in the flow area (72) passes by the at least one container-valve configuration (54), preferably all container-valve configurations (54), from the valve configuration (86) of the second connection formation (78) to the valve configuration (86) of the third connection formation (80) when coupling contact is established between the at least one coupling formation (64, 66, 66') of the liquid supply interface (62; 62'; 62"; 62'") and the at least one counter-coupling formation (38, 40, 40') of a cell culture container (10; 10'; 10").

36. The cell culturing system according to claim 35,
**characterized in that** - when coupling contact is established between the at least one coupling formation (64, 66, 66') of the liquid supply interface (62; 62'; 62"; 62'") and the at least one counter-coupling formation (38; 40; 40') of a cell culture container (10; 10'; 10") - the valve body (56) of the at least one container-valve configuration (54), preferably every valve body (56) of all container-valve configurations (54), protrudes at least partially, preferably more than half, into the flow path from the valve configuration (86) of the second connection formation (78) to the valve configuration (86) of the third connection formation (80).

37. The cell culturing system according to any one of claims 25 to 36,
**characterized in that** all valve configurations (74, 86) provided in the liquid supply interface (62; 62'; 62"; 62'"), preferably also all valve configurations (54) provided in the at least one cell culture container (10; 10'; 10"), are substantially of identical structure.

38. The cell culturing system according to any one of claims 25 to 37,
**characterized in that** all valve configurations (54, 74, 86) provided in the at least one cell culture container (10; 10'; 10") and in the liquid supply interface (62; 62'; 62"; 62'") at established coupling contact can be switched by a common control configuration (94) with a plurality of signaling means (100).

39. The cell culturing system according to any one of claims 25 to 38 together with claim 16,
**characterized in that** in at least one connection formation (76, 78, 80), preferably in a plurality of connection formations (76, 78, 80), an annular axial end face (59b) of the elastomer contact component (59) as a sealing surface abuts in a deformed manner a counter-sealing surface (64a) surrounding radially and externally the liquid flow path (82, 90, 92) allocated to the connection formation (76, 78, 80).

40. The cell culturing system according to any one of claims 25 to 39 together with claim 16,
**characterized in that** the container-valve configuration (54) is formed according to claim 16, wherein - at established coupling contact between the at least one coupling formation (64, 66, 66') of the liquid supply interface (62; 62'; 62"; 62'") and the at least one counter-coupling formation (38, 40, 40') of a cell culture container (10; 10'; 10") - an annular axial end face (59b) of the elastomer contact component (59) of the container-valve configuration (54) as a sealing surface abuts in a deformed manner a counter-sealing surface (64a) of the liquid supply interface (62; 62'; 62"; 62'") surrounding radially and externally the liquid flow path (44a, 47a, 47a') allocated to the coupling formation (64, 66, 66').

41. The cell culturing system according to any one of claims 25 to 40,
**characterized in that** it comprises more cell culture containers (10; 10'; 10") than liquid supply interfaces (62; 62'; 62"; 62'"), wherein in particular the cell culturing system does not comprise more than 5, preferably not more than 3, more preferably comprises precisely one liquid supply interface (62; 62'; 62"; 62"').

42. The cell culturing system according to claim 41,
**characterized in that** the cell culturing system comprises a moving means, for example, a multi-axis robot or a cross table movable in a plane of movement with movement carriage provided on it movable to the orthogonal plane of movement, with which the liquid supply interface (62; 62'; 62"; 62'") can be brought one after the other in liquid-transferring coupling contact with different cell culture containers (10; 10'; 10").

43. A cell culture container for a cell culturing system according to any one of claims 25 to 42 as well as for producible and detachable coupling contact with a liquid supply interface (62; 62'; 62"; 62'") according to any one of claims 1 to 24, wherein the cell culture container (10; 10'; 10") comprises a container body (12) including a culture volume (14) with a filling and/or ventilation opening (30) through which gas, liquid, paste and/or solid bodies can be filled into the container body (12) and removed from it,
**characterized in that** the cell culture container (10; 10'; 10") in addition comprises at least one counter-coupling formation (38, 40, 40') separately formed from the filling and/or ventilation opening (30), which is formed for establishing and detaching a coupling contact with a corresponding coupling formation (64, 66, 66') of the liquid supply interface (62; 62'; 62"; 62'"), wherein a delivery liquid flow path (44, 47) extends between the at least one counter-coupling formation (38, 40, 40') and the culture volume (14), in order to introduce a liquid into the culture volume (14) and/or to remove liquid from said culture volume (14) via the delivery liquid flow path (44, 47), wherein the at least one counter-coupling formation (38, 40, 40') has a container-valve configuration (54),
wherein preferably the container-valve configuration (54) can be switched between a blocked position, in which the container-valve configuration (54) interrupts a liquid flow in the delivery flow path (44, 47) and an outlet position in which the container-valve configuration (54) enables a liquid flow, by a control configuration (94) with a signaling means (100) generating an electric and/or magnetic and/or electromagnetic field, the field of which acts upon a correspondingly field-sensitive counter-signaling means (56) of the container-valve configuration (54) without contact.

44. The cell culture container according to claim 43,
**characterized in that** the filling and/or ventilation opening (30) and the at least one separately formed counter-coupling formation (38, 40, 40') are provided at opposite ends of the cell culture container.

45. The cell culture container according to claim 43 or 44,
**characterized in that** the cell culture container (10; 10'; 10"), except for the at least one container-valve configuration (54) of the counter-coupling formation (38, 40, 40'), is a passive container without functional units built into the container and operable by energy supply, such as, for example, heating device, stirring device and the like, more preferably a passive one-way-disposable cell culture container (10; 10'; 10").

46. The cell culture container according to any one of claims 43 to 45,
**characterized in that** the cell culture container (10; 10'; 10") comprises two substantially parallel front walls (16) and shell wall sections (18, 22, 24, 26, 28) connecting them and which are circumferential around a front wall edge, which together limit the culture volume (14), wherein preferably the at least one counter-coupling formation (38, 40, 40'), more preferably also the filling and/or ventilation opening (30) are provided in a shell wall section (22, 24).

## Revendications

1. Interface d'alimentation en fluide (62; 62'; 62"; 62"') pour un système de culture de cellules pour l'alimentation des cultures de cellule existant dans des récipients différents de cultures de cellule (10; 10'; 10") avec un milieu nutritif, l'interface d'alimentation en fluide (62; 62'; 62"; 62"') comportant:
un carter (68) définissant une zone d'écoulement (72),
une première formation de raccordement (76) pour le raccordement transférant du fluide d'un premier conduit de fluide (84) avec le carter (68),
une deuxième formation de raccordement (78) formée séparément de la première formation pour le raccordement transférant du fluide d'un deuxième conduit de fluide (88) avec le carter (68),
une troisième formation de raccordement (80) formée séparément des deux premières formations pour le raccordement transférant du fluide du carter (68) avec un troisième conduit de fluide,
une formation de couplage (64, 66) formée séparément des formations de raccordement (76, 78, 80) qui est formée pour le contact de couplage transférant du fluide produisable et détachable selon le fonctionnement avec une formation de contre-couplage (38, 40) correspondante d'un récipient de cultures de cellule (10; 10'; 10"),
un premier chemin d'écoulement de fluide (87) s'étendant entre la zone d'écoulement (72) et la première formation de raccordement (76) pour introduire un premier fluide de l'extérieur dans la zone d'écoulement (72),
un deuxième chemin d'écoulement de fluide (90) s'étendant entre la zone d'écoulement (72) et la deuxième formation de raccordement (78) pour introduire un deuxième fluide séparément du premier fluide de l'extérieur dans la zone d'écoulement (72),
un troisième chemin d'écoulement de fluide (92) s'étendant entre la zone d'écoulement (72) et la troisième formation de raccordement (80) pour évacuer un fluide de la zone d'écoulement (72), et
un chemin d'écoulement de couplage (44a, 47a) s'étendant entre la zone d'écoulement (72) et la formation de couplage (64, 66) pour évacuer un fluide de la zone d'écoulement (72) et/ou introduire un fluide dans la zone d'écoulement (72) à travers la formation de couplage (64, 66),
le premier (82), le deuxième (90) et le troisième chemin d'écoulement de fluide (92) chacun comprenant un ensemble de vannes (86) qui, sans un raccordement physique continu transmettant un signal et/ou de l'énergie de l'ensemble de vannes (86) jusqu'à l'extérieur du carter (68), est entièrement entouré par le carter (68) et y logé, à l'exception du chemin d'écoulement de fluide respectif,
un système de commande (94) étant attribué à chaque ensemble de vannes (86) avec un moyen de signalisation (100) créant un champ électrique et/ou magnétique et/ou électromagnétique dont le champ agit sans contact sur un moyen de contre-signalisation (56) conformément sensible au champ de l'ensemble de vannes (86), chaque ensemble de vannes (86) étant commutable au moyen du champ agissant sur son moyen de contre-signalisation (56) entre une position de blocage dans laquelle l'ensemble de vannes (86) interrompt un écoulement de fluide dans le chemin d'écoulement de fluide (82, 90, 92) dans lequel il est agencé, et une position de passage dans laquelle l'ensemble de vannes (86) permet un écoulement de fluide.

2. Interface d'alimentation en fluide selon la revendication 1,
**caractérisé en ce que** l'interface d'alimentation en fluide (62; 62'; 62"; 62"') comporte en outre:
une quatrième formation de raccordement (110) formée séparément des trois autres formations pour le raccordement transférant du fluide du carter (68) avec un quatrième conduit de fluide et
un quatrième chemin d'écoulement de fluide (112) s'étendant entre la zone d'écoulement (72) et la quatrième formation de raccordement (110) pour évacuer un fluide de la zone d'écoulement (72).

3. Interface d'alimentation en fluide selon la revendication 1 ou 2,
**caractérisé en ce que** la formation de couplage (64) est une première formation de couplage (64), que l'interface d'alimentation en fluide (62; 62'; 62"; 62"') comporte une deuxième formation de couplage (66) formée séparément de la première formation (64) qui est formée pour le contact de couplage transférant du fluide produisable et détachable selon le fonctionnement avec une deuxième formation de contre-couplage (40) correspondante du récipient de cultures de cellule (10; 10'), et que l'interface d'alimentation en fluide (62; 62'; 62"; 62'") comporte un deuxième chemin d'écoulement de couplage (47a) s'étendant entre la zone d'écoulement (72) et la deuxième formation de couplage (66) pour évacuer du fluide de la zone d'écoulement (72) et/ou y introduire du fluide à travers la deuxième formation de couplage (66).

4. Interface d'alimentation en fluide selon la revendication 3,
**caractérisé en ce que** l'interface d'alimentation en fluide (62; 62'; 62"; 62'") comporte un chemin d'écoulement de raccordement (75) s'étendant entre la première (64) et la deuxième formation de couplage (66) et l'interface d'alimentation en fluide (62; 62'; 62"; 62'") dans ce chemin comporte un ensemble de vannes de séparation (74) qui, sans un raccordement physique continu transmettant un signal et/ou de l'énergie de l'ensemble de vannes de séparation (74) jusqu'à l'extérieur du carter (68), est entièrement entouré par le carter (68) et y logé, à l'exception du chemin d'écoulement de raccordement (75),
un système de commande (94) étant attribué à l'ensemble de vannes de séparation (74) avec un moyen de signalisation (100) créant un champ électrique et/ou magnétique et/ou électromagnétique dont le champ agit sans contact sur un moyen de contre-signalisation (56) conformément sensible au champ de l'ensemble de vannes de séparation (74), l'ensemble de vannes de séparation (74) étant commutable au moyen du champ agissant sur son moyen de contre-signalisation (56) entre une position de blocage dans laquelle l'ensemble de vannes de séparation (74) interrompt un écoulement de fluide dans le chemin d'écoulement de raccordement (75), et une position de passage dans laquelle l'ensemble de vannes de séparation (74) permet un écoulement de fluide.

5. Interface d'alimentation en fluide selon la revendication 4,
**caractérisé en ce que** le chemin d'écoulement de raccordement (75) est le seul chemin d'écoulement de fluide (75) s'étendant entre la première (64) et la deuxième formation de couplage (66).

6. Interface d'alimentation en fluide selon l'une quelconque des revendications 4 ou 5,
**caractérisé en ce que** l'ensemble de vannes de séparation (74) est agencé de manière à ce que par cet ensemble le premier (82) et le deuxième chemin d'écoulement de fluide (90) est séparable du deuxième chemin d'écoulement de couplage (47a) mais non pas du premier chemin d'écoulement de couplage (44a), et que par cet ensemble le troisième chemin d'écoulement de fluide (92) est séparable du premier chemin d'écoulement de couplage (44a) mais non pas du deuxième chemin d'écoulement de couplage (47a).

7. Interface d'alimentation en fluide selon la revendication 6 ensemble avec la revendication 2,
**caractérisé en ce que** l'ensemble de vannes de séparation (74) est agencé de manière à ce que par cet ensemble le quatrième chemin d'écoulement de fluide (110) est séparable du premier chemin d'écoulement de couplage (44a) mais non pas du deuxième chemin d'écoulement de couplage (47a).

8. Interface d'alimentation en fluide selon l'une quelconque des revendications précédentes,
**caractérisé en ce que** l'interface d'alimentation en fluide (62") comporte une troisième formation de couplage (66') formée séparément de la première formation (64) et de la deuxième formation (66) et un troisième chemin d'écoulement de couplage (47a') s'étendant entre la zone d'écoulement (72) et la troisième formation de couplage (66') pour évacuer du fluide de la zone d'écoulement (72) et/ou introduire du fluide dans la zone d'écoulement (72) à travers la troisième formation de couplage (66'), la troisième formation de couplage (66') étant formée pour le contact de couplage transférant du fluide produisable et détachable selon le fonctionnement avec une troisième formation de contre-couplage (40') correspondante du récipient de cultures de cellule (10').

9. Interface d'alimentation en fluide selon la revendication 8,
**caractérisé en ce que** le chemin d'écoulement de raccordement (75) est un premier chemin d'écoulement de raccordement (75) et l'ensemble de vannes de séparation (74) est un premier ensemble de vannes de séparation (74), et que l'interface d'alimentation en fluide (62") comprend un deuxième chemin d'écoulement de raccordement s'étendent entre la deuxième formation (66) et la troisième formation de couplage (66'), et l'interface d'alimentation en fluide (62") dans ce chemin comprend un deuxième ensemble de vannes de séparation (74') séparément du premier ensemble qui, sans un raccordement physique continu transmettant un signal et/ou de l'énergie du deuxième ensemble de vannes de séparation (74') jusqu'à l'extérieur du carter (68), est entièrement entouré par le carter (68) et y logé, à l'exception du deuxième chemin d'écoulement de raccordement,
un système de commande (94) étant attribué au deuxième ensemble de vannes de séparation (74') avec un moyen de signalisation (100) créant un champ électrique et/ou magnétique et/ou électromagnétique dont le champ agit sans contact sur un moyen de contre-signalisation (56) conformément sensible au champ du deuxième ensemble de vannes de séparation (74'), le deuxième ensemble de vannes de séparation (74') étant commutable au moyen du champ agissant sur son moyen de contre-signalisation (56) entre une position de blocage dans laquelle le deuxième ensemble de vannes de séparation (74') interrompt un écoulement de fluide dans le deuxième chemin d'écoulement de raccordement, et une position de passage dans laquelle le deuxième ensemble de vannes de séparation (74') permet un écoulement de fluide.

10. Interface d'alimentation en fluide selon la revendication 9,
**caractérisé en ce que** le deuxième chemin d'écoulement de raccordement est le seul chemin d'écoulement de raccordement s'étendant entre la deuxième formation (66) et la troisième formation de couplage (66').

11. Interface d'alimentation en fluide selon la revendication 9 ou 10 ensemble avec la revendication 2,
**caractérisé en ce que** le deuxième ensemble de vannes de séparation (74') est agencé de manière à ce que soit
par cet ensemble le troisième chemin d'écoulement de fluide (92) est séparable du deuxième chemin d'écoulement de couplage (47a) mais non pas du troisième chemin d'écoulement de couplage (47a'), et que par cet ensemble le quatrième chemin d'écoulement de fluide (112) est séparable du troisième chemin d'écoulement de couplage (47a') mais non pas du deuxième chemin d'écoulement de couplage (47a),
ou soit que
par cet ensemble le quatrième chemin d'écoulement de fluide (112) est séparable du deuxième chemin d'écoulement de couplage mais non pas du troisième chemin d'écoulement de couplage (47a'), et que par cet ensemble le troisième chemin d'écoulement de fluide (92) est séparable du troisième chemin d'écoulement de couplage (47a') mais non pas du deuxième chemin d'écoulement de couplage (47a).

12. Interface d'alimentation en fluide selon l'une quelconque des revendications précédentes,
**caractérisé en ce que** pour au moins un ensemble de vannes (54, 74, 86), de préférence une pluralité des ensembles de vannes (54, 74, 86), particulièrement préféré pour tous les ensembles de vannes (54, 74, 82), le suivant est applicable:
- le moyen de signalisation (100) et le moyen de contre-signalisation (56) comprennent chacun une électrode pour créer un champ électrique entre ceux-ci, le moyen de contre-signalisation (56) coopérant avec un actionneur piézoélectrique de l'ensemble de vannes (54, 74, 86) de manière à ce que le champ électrique provoque un changement de structure de l'actionneur piézoélectrique qui à son tour provoque un ajustement de l'ensemble de vannes (54, 74, 86) entre la position de blocage et la position de passage, et/ou
- le moyen de signalisation (100) et le moyen de contre-signalisation (56) comprennent un aimant d'une part et un composant ferromagnétique et/ou magnétisé sensible à son champ magnétique d'autre part, le champ magnétique agissant entre le moyen de signalisation (100) et le moyen de contre-signalisation (56) provoquant un déplacement du moyen de contre-signalisation (56) qui provoque à son tour un ajustement de l'ensemble de vannes (54, 74, 86) entre la position de blocage et la position de passage, et/ou
- le moyen de signalisation (100) comprend un aimant et le moyen de contre-signalisation (56) comprend un composant électriquement conducteur sensible inductivement à son champ magnétique, le champ magnétique agissant entre le moyen de signalisation (100) et le moyen de contre-signalisation (56) provoquant une induction dans le moyen de contre-signalisation (56), qui à son tour provoque un ajustement de l'ensemble de vannes (54, 74, 86) entre la position de blocage et la position de passage, et/ou
- le moyen de signalisation (100) comprend un émetteur des ondes électromagnétiques comme, par exemple, des signaux optiques ou des signaux radio, et le moyen de contre-signalisation (56) comprend un récepteur correspondant, l'ensemble de vannes (54, 74, 86) comprenant un réservoir d'énergie et un actionneur qui sont couplés entre eux et avec le moyen de contre-signalisation (56) de manière à ce que le moyen de contre-signalisation (56), en fonction des ondes électromagnétiques reçues, commande l'actionneur alimenté du réservoir d'énergie pour la commutation de l'ensemble de vannes (54, 74, 86) entre la position de blocage et la position de passage.

13. Interface d'alimentation en fluide selon la revendication 12,
**caractérisé en ce que** le moyen de signalisation (100) et le moyen de contre-signalisation (56) comprennent un aimant (100) d'une part et un composant ferromagnétique et/ou magnétisé (56) sensible à son champ magnétique d'autre part, le moyen de contre-signalisation (56) étant un corps de vanne (56) de l'ensemble de vannes (54, 74, 86) qui - sous l'effet du champ magnétique agissant entre le moyen de signalisation (100) et le moyen de contre-signalisation (56) - est deplaçable en s'éloignant du siège de vanne (58) et/ou pour un contact étanche avec ce siège.

14. Interface d'alimentation en fluide selon la revendication 13,
**caractérisé en ce que** l'ensemble de vannes (54, 74, 86) est précontraint magnétiquement dans la position de blocage et est ajustable dans la position de passage par le champ magnétique émanant du moyen de signalisation (100).

15. Interface d'alimentation en fluide selon la revendication 14,
**caractérisé en ce qu'**un siège de vanne (58) de l'ensemble de vannes (54, 74, 86) comprend un composant tendeur (60) à magnétisation permanente ou ferromagnétique de manière qu'entre le composant tendeur (60) et le corps de vanne (56) une force de tension magnétique, surtout une force d'attraction, agit, tendant le corps de vanne (56) pour un contact étanche au siège de vanne (58).

16. Interface d'alimentation en fluide selon la revendication 15,
**caractérisé en ce que** le siège de vanne (58) comprend un composant de contact élastomérique (59) auquel le corps de vanne (56) directement vient en butée dans la position de blocage de l'ensemble de vannes (54, 74, 86), le composant de contact (59) de préférence étant agencé entre le corps de vanne (56) et le composant tendeur (60), la force de tension magnétique agissant entre le corps de vanne (56) et le composant tendeur (60) étant ensuite une force d'attraction.

17. Interface d'alimentation en fluide selon l'une quelconque des revendications précédentes,
**caractérisé en ce que** le moyen de signalisation (100) comprend un aimant permanent (100) deplaçable localement ou un électroaimant.

18. Interface d'alimentation en fluide selon la revendication 17,
**caractérisé en ce que** le système de commande (94) comprend une pluralité des jeux (102) de moyens de signalisation (100), les moyens de signalisation (100) d'un jeu (102) définissant chacun une configuration de position de vanne des ensembles de vannes (54, 74, 86) de l'interface d'alimentation en fluide (62; 62'; 62"; 62"').

19. Interface d'alimentation en fluide selon la revendication 18,
**caractérisé en ce que** le système de commande (94) comprend un rouleau (96) rotatif autour d'un axe du rouleau (W), la pluralité des jeux de moyens de signalisation (102) étant agencés distribués en circonférence autour de l'axe du rouleau (W) de manière à ce que par rotation du rouleau (96) des configurations différentes de position de vanne de l'interface d'alimentation en fluide (62; 62'; 62"; 62'") sont ajustables.

20. Interface d'alimentation en fluide selon l'une quelconque des revendications 17 à 19,
**caractérisé en ce que** le système de commande (94) comprend un système de commutation (104) agencé entre un moyen de signalisation (100) et un ensemble de vannes (54, 74, 86) ajustable par le moyen de signalisation (100), le système de commutation (104) comportant au moins un aimant (106), surtout un aimant permanent, deplaçable entre une position active plus proche de l'ensemble de vannes (54, 74, 86) et une position inactive plus proche du moyen de signalisation (100).

21. Interface d'alimentation en fluide selon la revendication 20 ensemble avec la revendication 18 ou 19,
**caractérisé en ce que** le système de commutation (104) comprend un aimant (106) deplaçable pour chaque moyen de signalisation (100) d'un jeu (102).

22. Interface d'alimentation en fluide selon la revendication 20 ou 21,
**caractérisé en ce que** l'au moins un aimant (106) deplaçable est précontraint, de préférence par gravité, dans une de ces positions, de préférence dans sa position inactive.

23. Interface d'alimentation en fluide selon l'une quelconque des revendications précédentes,
**caractérisé en ce qu'**au moins une partie des ensembles de vannes (54, 74, 86), de préférence tous les ensemble de vannes (54, 74, 86) sont ajustables par une différence de pression de fluide prédéterminée de la position de blocage dans la position de passage dans une direction d'écoulement de passage le long du chemin d'écoulement de fluide (82, 90, 92, 75) dans lequel ils sont agencés mais non pas dans la direction d'écoulement opposée, de préférence la direction d'écoulement de passage du premier et du deuxième chemin d'écoulement de fluide (82, 90) étant dirigée dans la zone d'écoulement (72), et la direction d'écoulement de passage du troisième chemin d'écoulement de fluide (92) étant dirigée vers l'extérieur de la zone d'écoulement (72).

24. Interface d'alimentation en fluide selon l'une quelconque des revendications précédentes,
**caractérisé en ce que** le chemin d'écoulement de couplage (44a, 47a 47a') ou les chemins d'écoulement de couplage (44a, 47a 47a') dans le cas de contact de couplage détaché sont libres des ensembles de vannes qui les interrompent ou les débloquent pour le passage.

25. Système de culture de cellules comportant au moins un récipient de cultures de cellule (10; 10'; 10") pour la réception et l'alimentation des cellules adhérentes dans ces récipients, un réservoir de milieu nutritif (116), un réservoir de fluide de nettoyage (114) et une interface d'alimentation en fluide (62; 62'; 62"; 62"') selon l'une quelconque des revendications précédentes à condition que:
- la première formation de raccordement (76) lie le carter (68) avec le réservoir de milieu nutritif (116) en transférant le fluide et donc le premier chemin d'écoulement (82) s'étende entre la zone d'écoulement (72) et le réservoir de milieu nutritif (116),
- la deuxième formation de raccordement (78) lie le carter (68) avec le réservoir de fluide de nettoyage (114) en transférant le fluide et donc le deuxième chemin d'écoulement de fluide (90) s'étende entre la zone d'écoulement (72) et le réservoir de fluide de nettoyage (114),
- la troisième formation de raccordement (80) lie le carter (68) avec un sortie (118) en transférant le fluide et donc le troisième chemin d'écoulement de fluide (92) s'étende entre la zone d'écoulement (72) et la sortie (118),
- la formation de couplage (64, 66, 66') soit formée pour le contact de couplage transférant du fluide produisable et détachable selon le fonctionnement avec une formation de contre-couplage (38, 40, 40') du récipient de cultures de cellule (10; 10'; 10"),
- le premier fluide soit le milieu nutritif,
- le deuxième fluide soit le fluide de nettoyage,
- le chemin d'écoulement de couplage (44a, 47a, 47a') soit formée pour introduire à travers la formation de couplage (64, 66, 66') dans un état couplé avec la formation de contre-couplage (38, 40, 40') du milieu nutritif de la zone d'écoulement (72) dans le récipient de cultures de cellule (10; 10'; 10") et/ou d'évacuer du milieu nutritif du récipient dans la zone d'écoulement (72).

26. Système de culture de cellules selon la revendication 25,
**caractérisé en ce qu'**il comprend une interface d'alimentation en fluide (62'; 62"; 62'") selon la revendication 2 ou selon l'une quelconque des revendications 12 à 24 ensemble avec la revendication 2 à condition que:
- la quatrième formation de raccordement (110) lie le carter (68) avec un dispositif collecteur d'échantillon (120) en transférant du fluide, et donc le quatrième chemin d'écoulement de fluide (112) s'étende entre la zone d'écoulement (72) et le dispositif collecteur d'échantillon (120).

27. Système de culture de cellules selon la revendication 25 ou 26,
**caractérisé en ce que** dans la zone d'écoulement (72) un chemin d'écoulement de l'ensemble de vannes (86) de la deuxième formation de raccordement (78) à l'ensemble de vannes (86) de la troisième formation de raccordement (80) est le chemin d'écoulement le plus long entre deux ensembles de vannes (74, 86) ou entre un ensemble de vannes (74, 86) et une formation de couplage (64, 66, 66').

28. Système de culture de cellules selon l'une quelconque des revendications 25 à 27,
**caractérisé en ce que** dans la zone d'écoulement (72) un chemin d'écoulement de l'ensemble de vannes (86) de la deuxième formation de raccordement (78) à l'ensemble de vannes (86) de la troisième formation de raccordement (80) passe à l'ensemble de vannes (82) de la première formation de raccordement (76) et, le cas échéant, à l'ensemble de vannes (86) de la quatrième formation de raccordement (110).

29. Système de culture de cellules selon la revendication 28,
**caractérisé en ce que** le corps de vanne (56) de l'ensemble de vannes (86) de la première formation de raccordement (76) et, le cas échéant, le corps de vanne (56) de l'ensemble de vannes (86) de la quatrième formation de raccordement (110) au moins partiellement, de préférence plus de la moitié, font saillie dans le chemin d'écoulement de l'ensemble de vannes (82) de la deuxième formation de raccordement (78) à l'ensemble de vannes (86) de la troisième formation de raccordement (80).

30. Système de culture de cellules selon l'une quelconque des revendications 25 à 29,
**caractérisé en ce que** la formation de contre-couplage (38) du récipient de cultures de cellule (10; 10') est un premier formation de contre-couplage (38), que le récipient de cultures de cellule (10; 10') comprend une deuxième formation de contre-couplage (40) formée séparément de la première formation de contre-couplage (38), et que le système de culture de cellules comprend en outre une interface d'alimentation en fluide (62; 62'; 62"; 62"') selon l'une quelconque des revendications 3 à 7 ou 12 à 24 ensemble avec la revendication 3, à condition que la première formation de couplage (64) soit formée pour le contact de couplage transférant du fluide produisable et détachable selon le fonctionnement avec la première formation de contre-couplage (38) et la deuxième formation de couplage (86) soit formée pour le contact de couplage transférant du fluide produisable et détachable selon le fonctionnement avec la deuxième formation de contre-couplage (40) du récipient de cultures de cellule (10; 10'), ainsi que le deuxième chemin d'écoulement de couplage (47a) s'étende entre la zone d'écoulement (72) et la deuxième formation de raccordement de couplage (66) pour introduire à travers la deuxième formation de couplage (66) - avec contact de couplage établi avec la deuxième formation de contre-couplage (40) - du milieu nutritif de la zone d'écoulement (72) dans le récipient de cultures de cellule (10; 10') et/ou évacuer du milieu nutritif du récipient dans la zone d'écoulement (72).

31. Système de culture de cellules selon la revendication 30, **caractérisé en ce que** le récipient de cultures de cellule (10') comprend une troisième formation de contre-couplage (40') formé séparément de la première (36) et de la deuxième (40) formation, et que le système de culture de cellules comprend une interface d'alimentation en fluide (62") selon l'une quelconque des revendications 8 à 24 ensemble avec la revendication 8, à condition que la troisième formation de couplage (66') soit formée pour le contact de couplage transférant du fluide produisable et détachable selon le fonctionnement avec la troisième formation de contre-couplage (40') du récipient de cultures de cellule (10'), ainsi que le troisième chemin d'écoulement de couplage (47a') s'étende entre la zone d'écoulement (72) et la troisième formation de raccordement de couplage (66') pour introduire à travers la troisième formation de couplage (66') - avec contact de couplage établi avec la troisième formation de contre-couplage (40') - du milieu nutritif de la zone d'écoulement (72) dans le récipient de cultures de cellule (10') et/ou évacuer du milieu nutritif du récipient dans la zone d'écoulement (72).

32. Système de culture de cellules selon l'une quelconque des revendications 25 à 31,
**caractérisé en ce qu'**au moins une formation de contre-couplage (38, 40, 40'), de préférence chaque formation de contre-couplage (38, 40, 40') du récipient de cultures de cellule (10; 10'; 10"), comprend chacun un ensemble de vannes à récipient (54).

33. Système de culture de cellules selon la revendication 32,
**caractérisé en ce que** l'ensemble de vannes à récipient (54) avec contact de couplage établi entre la formation de couplage et la formation de contre-couplage, sans un raccordement physique continu transmettant un signal et/ou de l'énergie de l'ensemble de vannes à récipient (54) jusqu'à l'extérieur de la formation de contre-couplage (38, 40, 40') et du carter (68) de l'interface d'alimentation en fluide (62; 62'; 62"; 62'"), est entièrement entouré par la formation de contre-couplage (38, 40, 40') et le carter de l'interface d'alimentation en fluide, à l'exception du chemin d'écoulement de raccordement traversant la formation de couplage (64, 66, 66') et la formation de contre-couplage (38, 40, 40').

34. Système de culture de cellules selon la revendication 32 ou 33,
**caractérisé en ce que** l'au moins un ensemble de vannes à récipient (54) est commutable par le système de commande (94) entre une position de blocage et une position de passage.

35. Système de culture de cellules selon l'une quelconque des revendications 32 à 34,
**caractérisé en ce que** dans la zone d'écoulement (72) un chemin d'écoulement de l'ensemble de vannes (86) de la deuxième formation de raccordement (78) à l'ensemble de vannes (86) de la troisième formation de raccordement (80) - avec contact de couplage établi entre l'au moins une formation de couplage (64, 66, 66') de l'interface d'alimentation en fluide (62; 62'; 62"; 62"') et l'au moins une formation de contre-couplage (38, 40, 40') d'un récipient de cultures de cellule (10; 10'; 10") - passe à l'au moins un ensemble de vannes à récipient (54), de préférence à tous les ensembles de vannes à récipient (54).

36. Système de culture de cellules selon la revendication 35,
**caractérisé en ce que** - avec contact de couplage établi entre l'au moins une formation de couplage (64, 66, 66') de l'interface d'alimentation en fluide (62; 62'; 62"; 62'") et l'au moins une formation de contre-couplage (38, 40, 40') d'un récipient de cultures de cellule (10; 10'; 10") - le corps de vanne (56) de l'au moins un ensemble de vannes à récipient (54), de préférence chaque corps de vanne (56) de tous les ensembles de vannes à récipient (54), au moins partiellement, de préférence au moins plus de la moitié, fait saillie dans le chemin d'écoulement de l'ensemble de vannes (86) de la deuxième formation de raccordement (78) à l'ensemble de vannes (86) de la troisième formation de raccordement (80).

37. Système de culture de cellules selon l'une quelconque des revendications 25 à 36,
**caractérisé en ce que** tous les ensembles de vannes (74, 86) prévus dans l'interface d'alimentation en fluide (62; 62'; 62"; 62"'), de préférence aussi tous les ensembles de vannes (54) prévus dans l'au moins un récipient de cultures de cellule (10; 10'; 10"), présentent une structure sensiblement identique.

38. Système de culture de cellules selon l'une quelconque des revendications 25 à 37,
**caractérisé en ce que** tous les ensembles de vannes (54, 74, 86) prévus dans l'au moins un récipient de cultures de cellule (10; 10'; 10") et dans l'interface d'alimentation en fluide (62; 62'; 62"; 62"') avec contact de couplage établi sont commutables par un système de commande (94) commun avec une pluralité de moyens de signalisation (100).

39. Système de culture de cellules selon l'une quelconque des revendications 25 à 38 ensemble avec la revendication 16,
**caractérisé en ce que** dans au moins une formation de raccordement (76, 78, 80), de préférence dans une pluralité de formations de raccordement (76, 78, 80), une face d'extrémité axiale annulaire (59b) du composant de contact élastomérique (59) en tant que surface d'étanchéité entourant radialement et extérieurement le chemin d'écoulement de fluide (82, 90, 92) attribué à la formation de raccordement (76, 78, 80) vient en butée de manière déformée sur une contre-surface d'étanchéité (64a).

40. Système de culture de cellules selon l'une quelconque des revendications 25 à 39 ensemble avec la revendication 16,
**caractérisé en ce que** l'ensemble de vannes à récipient (54) est formé selon la revendication 16, une face d'extrémité axiale annulaire (59b) du composant de contact élastomérique (59) de l'ensemble de vannes à récipient (54) en tant que surface d'étanchéité entourant radialement et extérieurement le chemin d'écoulement de fluide (44a, 47a, 47a') attribué à la formation de raccordement (64, 66, 66') venant en butée de manière déformée sur une contre-surface d'étanchéité (64a) de l'interface d'alimentation en fluide (62; 62'; 62"; 62'") - avec contact de couplage établi entre l'au moins une formation de couplage (64, 66, 66') de l'interface d'alimentation en fluide (62; 62'; 62"; 62'") et l'au moins une formation de contre-couplage (38, 40, 40') d'un récipient de cultures de cellule (10; 10'; 10").

41. Système de culture de cellules selon l'une quelconque des revendications 25 à 40,
**caractérisé en ce qu'**il comprend plus de récipients de cultures de cellule (10; 10'; 10") que des interfaces d'alimentation en fluide (62; 62'; 62"; 62"'), notamment le système de culture de cellules ne comprenant pas plus de 5, de préférence pas plus de 3, particulièrement préféré exactement une interface d'alimentation en fluide (62; 62'; 62"; 62"').

42. Système de culture de cellules selon la revendication 41,
**caractérisé en ce que** le système de culture de cellules comprend un dispositif de mouvement, par exemple, un robot multi-axe ou une table à mouvements croisés mobile dans un plan de mouvement avec chariot de mouvement prévu sur celle-ci et mobile par rapport au plan orthogonal de mouvement, avec lequel l'interface d'alimentation en fluide (62; 62'; 62"; 62'") peut être mis en contact de couplage transférant du fluide successivement avec des récipients de cultures de cellule (10; 10'; 10") différents.

43. Récipient de cultures de cellule pour un système de culture de cellules selon l'une quelconque des revendications 25 à 42 ainsi que pour un contact de couplage produisable et détachable avec une interface d'alimentation en fluide (62; 62'; 62"; 62"') selon l'une quelconque des revendications 1 à 24, le récipient de cultures de cellule (10; 10'; 10") comprenant un corps de récipient (12) comportant un volume de culture (14) avec une ouverture de remplissage et/ou de ventilation (30) à travers laquelle du gaz, du liquide, de la pâte et/ou des corps solides peuvent être remplis dans le corps de récipient (12) et enlevés de celui-ci,
**caractérisé en ce que** le récipient de cultures de cellule (10; 10'; 10") en outre comprend au moins une formation de contre-couplage (38, 40, 40') formée séparément de l'ouverture de remplissage et/ou de ventilation (30), qui est formée pour établir et détacher un contact de couplage avec une formation de couplage (64, 66, 66') correspondante de l'interface d'alimentation en fluide (62; 62'; 62"; 62"'), un chemin d'écoulement de fourniture de fluide (44, 47) s'étendant entre l'au moins une formation de contre-couplage (38, 40, 40') et le volume de culture (14) pour introduire à travers le chemin d'écoulement de fourniture de fluide (44, 47) un fluide dans le volume de culture (14) et/ou l'évacuer de celui-ci, l'au moins une formation de contre-couplage (38, 40, 40') comprenant un ensemble de vannes à récipient (54),
l'ensemble de vannes à récipient (54) étant commutable de préférence par un système de commande (94) avec un moyen de signalisation (100) créant un champ électrique et/ou magnétique et/ou électromagnétique, dont le champ agit sans contact sur un moyen de contre-signalisation (56) conformément sensible au champ de l'ensemble de vannes à récipient (54), entre une position de blocage dans laquelle l'ensemble de vannes à récipient (54) interrompt un écoulement de fluide dans le chemin d'écoulement de fourniture de fluide (44, 47), et une position de passage dans laquelle l'ensemble de vannes à récipient (54) permet un écoulement de fluide.

44. Récipient de cultures de cellule selon la revendication 43,
**caractérisé en ce que** l'ouverture de remplissage et/ou de ventilation (30) et l'au moins une formation de contre-couplage (38, 40, 40') formée séparément sont prévues à des extrémités opposées du récipient de cultures de cellule (10; 10'; 10").

45. Récipient de cultures de cellule selon la revendication 43 ou 44,
**caractérisé en ce que** le récipient de cultures de cellule (10; 10'; 10"), à l'exception de l'au moins un ensemble de vannes à récipient (54) de la formation de contre-couplage (38, 40, 40'), est un récipient passif sans des unités fonctionnelles intégrées dans le récipient utilisables par apport d'énergie telles que dispositif de chauffage, agitateur et similaires, particulièrement préféré un récipient passif de cultures de cellule (10; 10'; 10") à usage unique ou jetable.

46. Récipient de cultures de cellule selon l'une quelconque des revendications 43 à 45,
**caractérisé en ce que** le récipient de cultures de cellule (10; 10'; 10") comprend deux parois frontales (16) sensiblement parallèles et des parois d'enveloppe (18, 20, 22, 24, 26, 28) les raccordant qui sont circonférentielles autour d'un bord de paroi frontale qui limitent ensemble le volume de culture (14), l'au moins une formation de contre-couplage (38, 40, 40'), particulièrement préféré, aussi l'ouverture de remplissage et/ou de ventilation (30), étant prévue dans une paroi d'enveloppe (22, 24).
